# EUROPEAN PATENT SPECIFICATION

(11) **EP 3 118 613 B1**
(45) Date of publication and mention of the grant of the patent: **22.07.2020**
(21) Application number: 16178248.7
(22) Date of filing: 06.07.2016
(51) Int. Cl.: H01L 51/05

(54) **ELECTRIC COMPONENT WITH ORGANIC PART**
ELEKTRISCHE KOMPONENTE MIT ORGANISCHEM TEIL
COMPOSANT ÉLECTRIQUE À PARTIE ORGANIQUE

(30) Priority: 17.07.2015 EP 15177300; 21.10.2015 EP 15190887
(43) Date of publication of application: 18.01.2017
(73) Proprietor: ACREO SWEDISH ICT AB, 164 25 Kista (SE)
(72) Inventor: Berggren, Magnus, 602 18 Norrköping (SE); Simon, Daniel, 582 25 Linköping (SE); Crispin, Xavier, 610 20 Kimstad (SE); Gabrielsson, Roger, 601 74 Norrköping (SE); Stavrinidou, Eleni, 601 74 Norrköping (SE); Gomez, Eliot F., 602 16 Norrköping (SE)
(74) Representative: AWA Sweden AB

(56) References cited:
- F. N. DALTON: "In-situ root extent measurements by electrical capacitance methods", PLANT AND SOIL, vol. 173, no. 1, 1 June 1995 (1995-06-01), pages 157-165, XP055260619, NL ISSN: 0032-079X, DOI: 10.1007/BF00155527
- ELODIE PARDIEU ET AL: "Synthesis and characterization of [beta]-substituted 3,4-ethylenedioxy terthiophene monomers for conducting polymer applications", SYNTHETIC METALS, vol. 171, 10 April 2013 (2013-04-10), pages 23-31, XP055260666, CH ISSN: 0379-6779, DOI: 10.1016/j.synthmet.2013.03.012
- MICHAEL F. PEPITONE ET AL: "Synthesis of 2,5-Bis[(3,4-ethylenedioxy)thien-2-yl]- 3-Substituted Thiophenes", ORGANIC LETTERS, vol. 5, no. 18, 15 August 2003 (2003-08-15), pages 3229-3232, XP055260684, US ISSN: 1523-7060, DOI: 10.1021/ol035071s
- JEAN RONCALI ET AL: "3,4-Ethylenedioxythiophene (EDOT) as a versatile building block for advanced functional [pi]-conjugated systems", JOURNAL OF MATERIALS CHEMISTRY, vol. 15, no. 16, 1 January 2005 (2005-01-01), pages 1589-1610, XP055260689, GB ISSN: 0959-9428, DOI: 10.1039/B415481A
- LIANGQI OUYANG ET AL: "polymerization of poly(3,4-ethylenedioxythiophene) in the living rat hippocampus does not cause a significant loss of performance in a delayed alternation task", JOURNAL OF NEURAL ENGINEERING, INSTITUTE OF PHYSICS PUBLISHING, BRISTOL, GB, vol. 11, no. 2, 6 February 2014 (2014-02-06), page 26005, XP020260639, ISSN: 1741-2552, DOI: 10.1088/1741-2560/11/2/026005 [retrieved on 2014-02-06]
- E. Stavrinidou ET AL: "Electronic plants", Science Advances, vol. 1, no. 10, 20 November 2015 (2015-11-20), pages e1501136-e1501136, XP055457517, DOI: 10.1126/sciadv.1501136
- ELENI STAVRINIDOU ET AL: "In vivo polymerization and manufacturing of wires and supercapacitors in plants", PROCEEDINGS OF THE NATIONAL ACADEMY OF SCIENCES OF THE UNITED STATES OF AMERICA, vol. 114, no. 11, 27 February 2017 (2017-02-27), pages 2807-2812, XP055586557, US ISSN: 0027-8424, DOI: 10.1073/pnas.1616456114

## Description

### Technical field

The present invention relates to electronic components comprising an organic part such as a plant, a method of operating an electronic component, and a method of providing a continuous electronic conductor inside an organic part, and a compound that e.g. may be arranged in such an organic part.

### Background

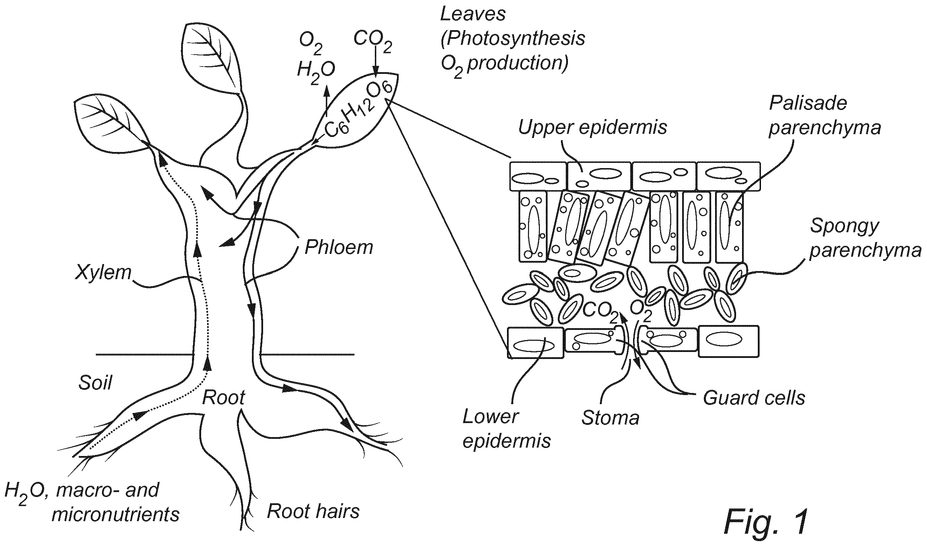

The growth and function of plants are powered by photosynthesis, and are orchestrated by hormones, nutrients, that are further affected by environmental, physical, and chemical stimuli. These signals are transported over long distances through the xylem and phloem vascular circuits to selectively trigger, modulate and power processes throughout the organism [101], see Fig. 1. Artificial regulation of plant processes is today achieved by exposing the plant to exogenously added chemicals or through molecular genetic tools that are used to endogenously change metabolism and signal transduction pathways in more or less refined ways [102]. F.N. DALTON: "In-situ root extent measurements by electrical capacitance methods", PLANT AND SOIL, vol 173, no. 1, 1 June 1995, (1995-06-01), pages 157-165, XP055260619, NL, ISSN: 0032-079X, DOI 10.1007/BF00155527. In this article a conceptual model is presented that provides a rational basis for using plant root capacitance as an in-situ measurement for assessing plant root development. This method is based on measuring the electrical capacitance of an equivalent parallel resistance-capacitance circuit formed by the interface between soil-water and the plant root surface. Nutrient solution studies using tomato (Lycopersicon esculentum Mill.) showed a good correlation between plant root capacitance and root mass.

However, many long-standing questions in plant biology are left unanswered due to a lack of technology that can precisely regulate or measure plant functions locally and in vivo. There is thus a need to record, address and locally regulate isolated - or connected - plant functions (even at the single cell level) in a highly complex and spatiotemporally resolved manner. Further, it is foreseen that many new opportunities may arise from a technology that may regulate or measure plant functions. Such a technology may also be able to harvest, regulate or measure chemicals and energy within plant. However, none of the currently available or tested methods have yielded promising results.

Hence, there is a desire to provide devices, methods and materials which may at least in part enable technical implementation(s) and interaction(s) with plants which do not rely on external stimuli e.g. via chemicals.

### Summary of the invention

It is an object of the present invention to improve the current state of the art, to solve or alleviate at least some of the above problems.

According to a first aspect of the present invention, an electronic component is provided. The electronic component comprises an organic part e.g. a petal, a petiole, a stem portion, a petal portion, a petiole portion or a leaf portion, an electronic conducting material is provided within the organic plant part, and the electronic conducting material forms a circuitry or a continuous electronic conductor, wherein said electronic conducting material is an organic polymer semiconducting material.

According to an embodiment of the present invention, an electronic component is provided. The electronic component comprises:
- an organic plant part e.g. a petal, a petiole, a stem portion, a petal portion, a petiole portion or a leaf portion;
- electronic conducting material provided in one or more of the transport channel(s) of the organic plant part; and
   wherein the electronic conducting material forms a continuous electronic conductor and/or a circuitry.

According one embodiment of the present invention, an electronic component is provided. The electronic component comprises:
- an organic plant part being a leaf;
- electronic conducting material provided to surround the cells within the spongy mesophyll of the leaf; and
   wherein the electronic conducting material forms an electronic conductor or a an continuous electronic conductor, wherein said electronic conducting material is an organic polymer semiconducting material.

In general, and in all embodiments of the invention, it should be understood that organic part is an organic plant part.

In this application the terms electronic component and electric component is used interchangeably.

In at least one exemplary embodiment, the organic part is a living organic part, e.g. a living plant or a part thereof. Optionally, the organic part may is arranged to be supplied with water and/or nutrition for keeping it alive.

The present invention is based on the realization that an electronic technology utilizing the transport channel(s), of an organic part enables new pathways to harvest e.g. a plant's photosynthesis and other complex biochemical processes, as well as the possibility to create electronic components and circuits being arranged at least partly within the organic part, e.g. a plant. Further, the electronic component may also be used with further components in order to control, measure or otherwise influence or detect processes within an organic part. For example, the electronic components may be used, possibly in conjunction with other component or devices, for regulation and control of plant physiology and functions, from the event of seeding to the state of a fully matured plant, and to electroactively convert plant chemicals into desired products, e.g. electrical energy and chemical end products. Further, the growth i.e. production of fruits, berries, or other types of vegetables may be controlled or at least partly influenced. This means that the production of the plant may be controlled depending on the oncoming weather, season, or other types of factors which can be calculated or statistically pre-determined by humans. Moreover, the invention may also be understood to allow manufacturing of electronic components by using the transport channel(s) of an organic part to form the structure or scaffolding for the electronic component.

The term "electronic conductor" and the term "electronic conducting material" should henceforth in the context of the following text be interpreted as an organic polymer semiconducting material. The semiconducting material is selected from a group comprising: semiconducting organic polymers, such as poly(3,4-ethylenedioxythiophene) (PEDOT), polyaniline, polythiophene, polypyrrole, and polyphenylenevinylene (PPN)

A continuous electronic conductor should be interpreted as the uninterrupted extension of an electronic conductor. This means that small parts of electronic conductors which are spatially separated, and not in direct electronic contact, from each other do not form a continuous electronic conductor.

The term "electronic circuitry" should be interpreted as comprising at least an electronic conductor attached to an electric component, such as a resistor, inductor, transformer, capacitor or transistor. The electronic circuitry or can include devices and conductors that transport and/or adapt or transform electronic and/or ionic signals. Hence, they may be a part of electronic, electrochemical and/or iontronic organic device. Electronic devices are e.g. based on metals or semiconductors, e.g. solid-state organic semiconductors, and electrochemical devices are typically based on the combination of organic semiconductors and ion conductors (electrolytes), and the iontronic devices may e.g. comprise a combination of ion selective (bipolar) membranes.

In at least one exemplary embodiment, the electronic conducting material may form a conductor or it can form a circuity optionally including the conductor. For example, the electronic conducting material may form a circuitry without any conductor, i.e. the electronic conducting material may be e.g. a electrochromic element in a display. In such a display the color can be switched due to a voltage is applied across the electronic element. Alternatively, a display may comprise of electrochromic element and conductor or conductors. The conductor or conductors provide a voltage across the electrochromic elements, i.e. the circuitry is formed by the conductors and the electrochromic elements. In other words, the cicuitry may be formed by a combination of different conducting materials.

In at least one exemplary embodiment, the electrical conductivity of the continuous electronic conductor may be at least 10⁻¹⁰ S/m, or at least 10⁻⁵ S/m, or at least 10⁻³ S/m, or at least 10⁻¹ S/m, or at least 10 S/m, provided that it is semiconducting. The continuous electronic conductor preferably provides a sufficiently large electrical conductivity in order to provide an efficient electric path for sending or receiving electric signals, or influencing or measuring biological activities within a plant. The choice of electronic conductor is based on what should be achieved. In exemplary application a low conductivity is enough. PEDOT:PSS and PEDOT:S-H are two examples of materials which at least in one oxidation state has a low electronic conductivity.

In relation to this invention a transport channel is a naturally occurring channel in the organic part for transport of substances, e.g. nutrients, water, electrolytes, sugars and/or hormones in the plant. The transport channel(s) may be known as e.g. Xylem or Phloem. The organic part may be understood as at least a part or portion of a plant. Alternatively, an entire plant, or substantially an entire plant, may be comprised in the term "organic part". Plant should in the context of this description be interpreted broadly as an organic entity preferably utilizing a photosynthesis process to produce energy in the form of chemical energy. Plant may refer to both gymnosperms and angiosperms. Even if numerous examples herein and henceforth refer to flowers, trees or berries, it is understood that this is only for the sake of brevity and that the present invention may be used to advantage with, in principle, any kind of plant.

In at least one exemplary embodiment, the length of the continuous electronic conductor is at least 0.1 mm or at least 0.5 mm or at least 1 mm or at least 2 mm or at least 5 mm or at least 1 cm or at least 5 cm or at least 10 cm, or wherein the length of the circuitry is at least 100 µm or at least 1 mm or at least 2mm or at least 5mm or at least 1 cm or at least 5 cm or at least 10 cm. The length of the continuous electronic conductor or the length of the circuitry should be interpreted as the length measured in a transport channel of the organic part. Stated differently, the length of a continuous electronic conductor in the context of this description is the length of the continuous electronic conductor as measured in the direction in which a transport channel of the organic part extends. The length may be measured along the longitudinal direction of a transport channel. The continuous electronic conductor may be construed as substantially being formed or arranged within the transport channel(s). The length of the circuitry should be interpreted as the length of the circuitry and thereto connecting electronic conductors.

Electronic and/or electrochemical contacts to such an electronic component may be formed using dendrite electrodes manufactured in soil where e.g. a plant is planted, and/or by grafting inoculated electrodes into the stem of plants. Distributed wires and interconnect networks may thus be achieved along the vascular systems of the plants by dispensing electronic and ionic conductors along the transport channel(s). By the use of certain material, compounds and methods self-organization of conductors along the inner periphery of extended transport channels may be acheived. Hence, devices may be formed and distributed along the various components and junctions of the plant, i.e. leaves, petioles, axils, stem etc.

For example, circuits and components, wires, interconnects and contacts, may comprise of substantially linear (1D), areal (2D) and bulky (3D) iontronic- and electronic conducting and electro-active structures that are in vivo-processed and - manufactured inside the various constructs of an organic part such as a plant. Interconnects should herein and henceforth be construed as nodes in an electrical network or circuit, i.e. connection points between wires. This may e.g. be done by first dispensing materials into compartments and vascular pathways via grafted inlets, the roots and hairs and also through the stoma gateways. Then, immediately after entrance, the electro-active materials, e.g. in the form as dissolved oligomers and polymers, may be distributed and organizes promoted by transpiration, cohesion-tension transport and the plant tissues as the templates, respectively. Finally, the stress responsive mechanism of the plant may trigger gelling and also polymerization of the electro-active materials into desired architectures and device structures.

In at least one exemplary embodiment of the invention, the continuous electronic conductor extends along the inner wall of the transport channel in the circumferential direction; and wherein the continuous electronic conductor preferably extends along at least 10 %, or at least 20 %, or at least 40 %, or at least 60 %, or at least 80 %, or at least 90 % of the circumference of the inner wall. Stated differently, the continuous electronic conductor may extend and cover also a circumferential portion of the transport channel. The continuous electronic conductor may thus be formed on or at the inner wall(s) of the transport channel(s). Stated differently, the electronic conductor may extend along the inner periphery of a transport channel. In at least one embodiment, the continuous electronic conductor, seen in cross-section of the transport channel, may fill or occupy less than 90% of the cross-sectional area of the transport channel, or less than 70%, or less than 50%, or less than 30%, or less than 10%. Hence, even if the continuous electronic conductor is formed on or at the inner wall of the transport channel(s), the transport channel(s) need not be clogged by the continuous electronic conductor. Advantageously, the continuous electronic conductor may thus still enable transport of e.g. nutrients, water, electrolytes, sugars and/or hormones in the organic part such as a plant. Alternatively, the continuous electronic conductor, seen in cross-section of the transport channel, may fill substantially more than 90% of the cross-sectional area of the transport channel. However, the organic part may still be able to transport nutrients since an organic part typically comprises a large number of transports channels, and although some of these are clogged, the remaining unclogged transport channel(s) may still provide the ability to transport nutrients. The mean thickness of the continuous electronic conductor may be less than 1µm or less than 3µm or less than 5µm or less than 10µm or less than 50µm.

In at least one exemplary embodiment, the continuous electronic conductor is fully or at least partly formed as a layer or is band-shaped. The layer or band-shaped continuous electronic conductor may thus be formed on the inner wall(s) of the transport channel(s). The mean thickness of the layer or band-shaped continuous electronic conductor may be less than 1µm or less than 3µm or less than 5µm or less than 10µm or less than 50µm.

Alternatively or additionally, the continuous electronic conductor is fully or at least partly formed as a wire. The wire may fill or occupy an area less than 90% of the cross-sectional area of the transport channel, or less than 70%, or less than 50%, or less than 30%, or less than 10% of the cross-sectional area of the transport channel; as seen in cross-section of the transport channel. Additionally or alternatively, the wire may fill or occupy an area larger than 90 % of the cross-sectional area of the transport channel, or larger than 70%, or larger than 50%, or larger than 30%, or larger than 10% of the cross-sectional area of the transport channel; as seen in cross-section of the transport channel. Hence, even if the continuous electronic conductor is formed on or at the inner wall of the transport channel(s), the transport channel(s) need not be clogged by the continuous electronic conductor

In at least one exemplary embodiment, the electronic conducting material forming the electronic conductor comprises conducting polymers.

The semiconducting material is an organic polymer semiconductor. The semiconducting material may be a p-type, n-type or ambipolar semiconductor. The organic polymer semiconductor material may be an organic semiconducting conjugated polymer. Examples of semiconducting conjugated polymers include, but are not limited to poly(alkylthiophene), poly(thienylene-vinylene), polyfluorene, polydiacetylene, poly(p-phenylene vinylene), or copolymers thereof. Typically, the conjugated polymer may be selected from the group consisting of poly-3-alkylthiophenes, for example, poly3-hexylthiophene (P3HT), poly(thienylene-vinylene), polyfluorene, polydiacetylene, poly(p-phenylene vinylene), or mixtures thereof.

Said polymeric conjugated material of said organic semiconducting layer may be selected from the group consisting of perylene, naphthalene, anthracene, tetracene, pentacene, hexacene, rubrene, phtalocyanines, porphyrines, and polymers of anilines, perylenediimides, naphthalenediimides, dialkylperylenedicarboximides, dialkylnaphthalenedicarboximides, triarylamines, pyrroles, dithienopyrroles, diketopyrrolopyrroles, carbazoles, thiophenes, dithiophenes, cyclopentadithiophenes, dithienothiophenes, benzodithiophenes, dialkoxybenzodithiophenes, benzothienobenzothiophenes, thienylenevinylenes, thiazoles, thiazolothiazoles, benzobisthiazoles, benzobisthiadiazoles, benzobisoxazoles, benzothiadiazoles, quinoxalines, thienoimides, fluorenes, indenofluorenes, indacenodithiophenes, siloles, dithienosiloles, dibenzosiloles, dibenzo(thieno)siloles, pyrazines, thienopyrazines, naphthodithiophenes, phthalimides, quinixalines, dithienylquinoxalines, benzoxadiazoles, acetylenes, sulfur nitrides, diacetylenes, phenylenes, thienylenevinylenes, phenylenevinylenes, cyanovinylenephenylene, phenylene sulfides, thiazines, phenothiazines, benzobisimidazobenzophenanthrolines, dithenodiimides, and derivatives thereof.

The transport of electrons and holes, the so-called ambipolar charge transport, in a semiconductor material may be advantageous. In at least one exemplary embodiment, the semiconductor is an ambipolar-type semiconductor.

In at least one exemplary embodiment, the electric component is at least one of a conductor, transistor, a capacitor, a super-capacitor and electrochromic device. The conductor in the organic part may be used to contact other types of device(s) and provide a signal or measurement path within the organic part or any other message. Optionally, the conducting material is not only provided inside the transport channel or transport channels but also extends outside the transport channel or transport channels and outside the plant so as to facilitate the application of e.g. an potential and/or to interact with other or similar types of devices outside the organic part. The transistor may be used as a switch, or in principle any other known purpose of transistors. An electrochromic device may e.g. be a display, an ornament and/or indicator. The electrochromic device may thus be used to indicate or display properties or current activities within for example a plant. The electrochromic device may alternatively be used as an ornament, e.g. to provide interesting effect on e.g. a flower used for indoor or outdoor decoration. For example the electronic conductor may be arranged of electrochromic material which is electrochemically switchable between two coloring states, thereby a plant has an adjustable color or pattern. Additionally and/or alternatively, the plant may function as an indicator of a signal from the plant or measured property thereof.

In at least one exemplary embodiment, a transistor is provided. The transistor comprises:
- a source and a drain which are electrically connected by a conductor
- a gate arranged at a distance from said conductor.
Moreover, the source is configured to be provided with a first potential, the drain is configured to be provided with a second potential and the gate is provided with the third potential, where the first and the second potentials are different from each other. The conductor is arranged of a semiconducting material and/or a material which is electrochemically switchable between two oxidation states, wherein the electronic conductivity of the oxidation states is different.

In at least one exemplary embodiment the electronic conducting material is an electrochromic material which forms electrochromic elements. Together with an electrolyte which is naturally occurring in the organic plant, an electrochromic device is formed. The electrochromic device will switch color when a voltage is induced across the elements.

In at least one exemplary embodiment, the electric component may further be a super-capacitor. The super capacitor comprises two conductors which are arranged at a distance from each other. Moreover, the space between the conductors comprises cellular domains and/or extracellular space where a natural electrolyte is occurring, which acts as a spacer. The electronic conducting material which forms the two conductors may e.g. be chosen from metals, conducting polymers, electrochemically active material, semiconducting organic polymers, metal oxides, metal oxide-filled polymer, carbon, any conductive polymers and their derivatives and/or combinations thereof. In at least one exemplary embodiment, the electronic conducting material is in direct or indirect electronic contact with external contacts, and/or connecting wires. In other words, the conducting material is not only provided inside the transport channel or transport channels but also extends outside the transport channel or transport channels and outside the plant so as to facilitate the application of e.g. an potential and/or to interact with other or similar types of devices outside the organic part. For example, the electronic conducting material may be in direct or indirect electronic contact with devices external, e.g. outside, the organic part in order to e.g. measure or control processes or transport within the organic part.

Direct electrical contact: Direct physical contact (common interface) between two phases that allows for the exchange of charges through the interface. Charge exchange through the interface can comprise transfer of electrons between electrically conducting phases, transfer of ions between ionically conducting phases, or conversion between electronic current and ionic current by means of electrochemistry at an interface between for example an electrochemically active material and an ion conductive material, or by occurrence of capacitive currents due to the charging of the Helmholtz layer at such an interface.

Ionic contact between two elements is provided by at least one material capable of transporting ions between the two elements. An electrolyte, in direct ionic contact (common interface) with a respective first and second element, is one example of a material which may provide ionic contact between the first and the second element. The electrolyte may hence be referred to as being in ionic contact with the two electrochemically active layers.

Two materials may be in electronic contact with each other, e.g. via a third material. Electronic contact between two elements is provided by at least one material capable of transporting electrons between the two elements. A first electronically conductive element, in direct electronic contact (common interface) with respective second and third electronically conductive element, is one example of a material which may provide electronic contact between the second and third electronically conductive elements.

According to a further aspect of the present invention, a system comprising an electric component according to the first aspect is provided. The electronic conducting material is electrically connected to a device for measuring and/or controlling properties related to the organic part.

Additionally or alternatively, the system may comprise an electronic circuit as described above and means for keeping the organic part alive, such as means for supplying the organic part with water and/or nutrition. These means may be provided continuously or at predetermined intervals. In other words, the organic part may continuously or at predetermined intervals be provided with e.g. water and/or nutrition for keeping it alive.

Effects and features of this further aspect of the present invention are largely analogous to those described above in connection with the previous aspect(s) of the invention. Embodiments mentioned in relation to this aspect of the present invention are largely compatible with the previous aspect(s) of the invention. Hence, the electronic component may provide fast and electronically controlled regulation of the balance between different plant hormones to regulate growth, prevent diseases and to promote the expression of specific structures of plants. A desire would be to record, address and regulate local and isolated, but also connected, plant functions (even at the single cell level) in a highly complex manner and at high spatiotemporal resolution.

As an example, a method for measuring and/or controlling properties related to the organic part of an electric component according to the first aspect is provided. The method comprises the steps of: providing a system comprising an electronic component according to the first aspect, and a device for measuring and/or controlling properties related to the organic part, connecting the device to the electronic component; and measuring and/or controlling at least one property related to the organic part.

According to a further aspect of the present invention, a method for operating an electric component in accordance with the first aspect is provided. The electronic conducting material comprises electrochemically active material. The method comprises the steps of: supplying first potential to a first portion of said electronic conducting material, a second potential to a second portion of said electronic conducting material which is different from said first potential, and electrochemically reacting the electronic conducting material being an electrochemically active material. In other words, the method for operating an electric component may comprise inducing an electric current through said electronic component and electrochemically reacting the electronic conducting material being an electrochemically active material.

An electrochemical reaction may alter the properties of the material such as the color and/or the electric conductivity. By this alteration the visual appearance or the behavior of the organic part may be adjusted.

Effects and features of this further aspect of the present invention are largely analogous to those described above in connection with the previous aspect(s) of the invention. Embodiments mentioned in relation to this aspect of the present invention are largely compatible with the previous aspect(s) of the invention.

In at least one exemplary embodiment, the potential difference is sustained for a predetermined period of time. By sustaining the potential difference an effect of the electrochemical activation may either be strengthened, weakened, or prolonged. For example, an electrochemical process may enable or disable the transport of ionic or non-ionic nutrients, electrolytes, sugars and/or hormones in the transport channel(s).

According to an exemplary embodiment, a method of providing a continuous electronic conductor inside an organic part is provided. The method comprises the steps of: providing a composition comprising at least one of: a metal, a semiconductor, a semiconducting polymer, a semiconducting oligomer, a semiconducting molecule, networks comprising metal parts, networks comprising organic parts; and drawing at least a portion of the composition into the organic part.

The composition may be a liquid, or be comprised in a liquid, or the composition may be a gel, or comprised in gel. In principle, the composition may be comprised in any form of material which enables the organic part to draw in at least a portion of the composition.

Effects and features of this examplary embodiment are largely analogous to those described above in connection with the previous aspect(s) of the invention. Embodiments mentioned in relation to this embodiment are largely compatible with the previous aspect(s) of the invention. According to one example, this aspect aspect may utilize compound 1 in order to form an electronic conductor within an organic part.

In at least one exemplary embodiment, drawing the portion of liquid into the organic part comprises using means of suction pressure, osmosis and/or capillary effects. Hence, the organic part may be alive and thereby it is possible to use the natural processes within the organic part which draws in nutrients, water, electrolytes, sugars and/or hormones in the organic part for drawing a liquid into the organic part.

In at least one exemplary embodiment, the organic part comprises roots, and the portion of liquid is drawn into the organic part via the roots. Advantageously, the natural process for an organic part, such as a plant, for drawing in nutrients, water, electrolytes, sugars and/or hormones e.g. the root(s) may be utilized to insert the liquid into the organic part. Alternatively, a non-natural or manual process may be used.

According to a further aspect of the present invention, a method of providing organic polymer electronic conducting material inside an organic part according to the first aspect is provided. The method comprises allowing the polymer electronic material is polymerised inside the organic part or in the transport channel or transport channels of the organic part.

Hereby, yet another advantage of the present invention is realized, as the present invention may utilize the natural occurring defense, or lignin polymerization processes of a plant to increase the speed at which polymerization occurs within the transport channel(s) of the organic part.

Effects and features of this further aspect of the present invention are largely analogous to those described above in connection with the previous aspect(s) of the invention. Embodiments mentioned in relation to this aspect of the present invention are largely compatible with the previous aspect(s) of the invention.

In at least one exemplary embodiment, the electronic conducting material in the organic part comprises a conductive polymer comprising the repeating unit of formula II 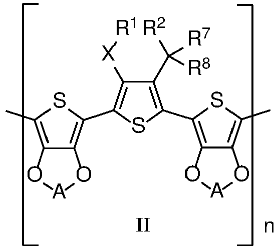 wherein
A represents a C₁-C₄-alkylene diradical optionally substituted with one to four R⁹;
X is hydrogen or CR⁵R⁶;
when X is hydrogen, R¹ is absent;
when X is CR⁵R⁶, R¹ is selected from the group C₃-C₆-cycloalkyl, aryl and C₁-C₈-alkyl in which one -CH₂- is optionally replaced by -O- or -S-, said C₃-C₆-cycloalkyl, C₁-C₈-alkyl or aryl optionally carrying one to three R³, said C₁-C₈-alkyl may be selected such that R¹ and R² form a 5-10 membered ring;
R² is selected from the group C₃-C₆-cycloalkyl, aryl and C₁-C₈-alkyl in which one -CH₂- is optionally replaced by -O- or -S-, said C₃-C₆-cycloalkyl, C₁-C₈-alkyl or aryl optionally carrying one to three R³, said C₁-C₈-alkyl may be selected such that R¹ and R² form a 5-10 membered ring;
R¹ and/or R² carries one or two R⁴;
each R³ and R⁹ is independently selected from hydrogen, hydroxy, halogen, C₁-C₃-alkyl, C₁-C₃-haloalkyl and C₁-C₃-alkoxy;
each R⁴ is independently selected from hydrogen, sulfonate, carboxylate, sulfinate, phosphonate and phosphinate or the corresponding acid or the corresponding sodium, potassium or lithium salt thereof; and R⁵, R⁶, R⁷ and R⁸ are each independently selected from hydrogen, halogen, C₁-C₃-alkyl, C₁-C₃-haloalkyl and C₁-C₃-alkoxy.

In at least one exemplary embodiment, the electronic conducting material in the organic part comprises conductive polymer comprising the repeating unit of formula II, wherein said repeating unit is

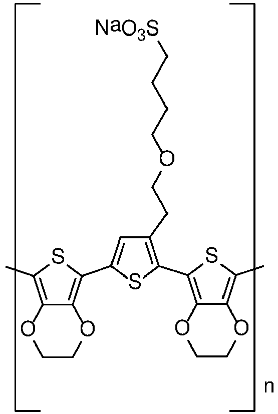

In at least on exemplary embodiment, relating to at least the aspects of the invention, at least a portion of an organic part is immersed, for at least three hours, in an aqueous solution comprising 0.1-10mg/ml of compound 1. Hence, the organic part may be allowed to draw in the aqueous solution comprising compound 1, and compound 1 may polymerize within the transport channel(s) of the organic part. For an organic part having a plurality of roots, only some roots may be immersed in the solution, such as a tenth, a fifth or half of the roots. In at least one exemplary embodiment, all the roots of an organic part are immersed in an aqueous solution comprising 0.1-10mg/ml of compound 1 .

Hence, the general inventive concept of the present invention, above and in the following description, may be construed, although not limited to, as relating to the administration, insertion, synthesis, and the generation of electronic and chemical systems to achieve various forms of electronic components such as conductors, circuitry and/or circuits in plants, in vivo, as well as to these electronic components per se.

Today, sophisticated technology, such as advanced materials, electronics and energy systems, typically requires rare and toxic compounds together with complex manufacturing. This puts demands on nature and society regarding resources and recycling. Plants are renewable, robust, large-volume and high-performing machineries and represent an untapped source for the production of advanced materials, electronics and energy systems. Further, genetic modification techniques may be used to improve the performance of plants, in terms of resistance to external parameters and increased production volume. However, deploying these techniques is limited by regulatory and societal conditions. Complementary or alternative techniques in accordance with the invention which can record and regulate plant performance, in vivo, based on where natural hormone chemistry and nutrients may thus provide large benefits.

Hence, organic electronics, based on natural and synthetic materials, manufactured inside living plants may be used to regulate, and to harvest from, the functions and processes of gymno- and angiosperms. It is therefore suggested to use the chemicals, tissues, vascular system and components of gymno- and angiosperms as an integral part and template, e.g. an organic part, to manufacture organic electronics, in vivo, and developing organic electronic circuits or circuitry to sense and deliver hormones, nutrients, sucrose etc. at high specificity, complexity and spatiotemporal resolution.

It is suggested to derive the four key-components of an integrated circuitry in plants, in vivo, and those are: electronic and/or electrochemical and/or iontronic contacts, wires and inter-connects and devices. Electronic and electrochemical contacts is achieved using for instance dendrite electrodes manufactured in the soil for an organic part such as a plant, and/or by grafting inoculated electrodes into the stem of plants. Distributed wires and interconnect networks is achieved along the vascular systems of plants by arranging electronic and ionic conductors along the xylem and/or phloem channels.

It is suggested to achieve self-organization of conductors along the inner wall, i.e. the periphery, or inner wall, of extended xylem channels and/or phloem channels. Electronic devices are thus formed and distributed along the various constructs of the plant, e.g. in the leaf along the stem and in the root. For instance, electrochemical or ion transistors, for signal routing, dispensing or sensing in the stem can be defined at specific positions along the stem, by combining two conducting xylem wires with a current modulating junction.

For the area of electronics, the above inventive concept, referred to as "E-plants", provides a radically novel platform, of materials, circuits, functions and applications, that is expected to boost creativity within its all associated fields, e.g. synthesis, manufacturing, devices and circuits and also applications. Specifically, the nature of functions and signals of plants will inspire the development of new devices and circuits based on novel materials and structures. Further, electronics require a continuous supply of input signals and energy; it is of generic interest to derive autarkic electronics that rely on signals and products from self-regenerating/converting systems such as plants. Electronics entirely integrated with plants, also defines a new path towards "green electronics" in its widest sense.

Plant biology is a rapidly growing field and new findings and applications rely on further understanding and new techniques. With e-Plant devices and circuit technology, many of the fundamental functions and properties can be in-situ and in-vivo regulated and recorded in a manner far beyond what electrodes, microscopy, syringes and microfluidics can provide today. The hope and beliefs is that e-Plant technology will complement existing genetic and molecular techniques to reveal many, still unanswered, secrets of plant biology.

E-Plants may define a new cross-disciplinary research field, with a technology that bridges the gap between signals and products of the plants and the addressing signals of electronics. When these two, still un-twinned, research areas meet each other, creativity, curiosity and fun will spark new ideas, knowledge and concepts. With in-vivo e-Plant circuits, a matrix of sensor and delivery devices will enable accurate control and recording of plant physiology. Connecting the e-Plants to computational power, future approaches of artificial fertilizers, surveillance and monitoring of plants and also for forest refineries will be possible.

Further, E-Plants represent a platform to explore generic concepts regarding in-vivo manufacturing of electronics and iontronics with living tissue, which may be of importance for the area of Organic Bioelectronics in medical applications such as towards new prosthesis and therapy methods.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of said element, device, component, means, step, etc., unless explicitly stated otherwise.

### Brief description of the drawings

The above objects, as well as additional objects, features and advantages of the present invention, will be more fully appreciated by reference to the following illustrative and non-limiting detailed description of preferred embodiments of the present invention, when taken in conjunction with the accompanying drawings, wherein:
Figure 1 is a schematic view of a plant, and the vascular system in a plant.
Figure 2 shows chemical structures explored within at least one embodiment of the invention;
Figure 3A is a schematic perspective view of root and graft contacts connected to the plant in accordance with at least one embodiment of the invention;
Figure 3B is a schematic perspective view of wires and contacts manufactured in the vascular system of a plant in accordance with at least one embodiment of the invention;
Figure 3C is a schematic perspective view of devices formed and distributed throughout the plant in accordance with at least one embodiment of the invention;
Figure 4 and 5 are schematic views of circuitries and/or circuit(s) and/or device(s) in a plant in accordance with at least one embodiment of the invention;
Figure 6A-E is a schematic view schematically showing the idea and of an experiment where electronically conducting wires were produced inside the xylem vascular system of roses;
Figure 7 shows electronically conducting xylem wires, transistors, and digital logic in accordance with at least one embodiment of the invention;
Figure 8 shows PEDOT-loaded leaves and leaf-OECDs in accordance with at least one embodiment of the invention;
Figure 9 shows PEDOT-loading and electrochromic leaves on living rose in accordance with at least one embodiment of the invention;
Figure 10 shows chemical structures of the monomers and polymers which have been utilized in experiments in accordance with at least one embodiment of the invention;
Figure 11A-E includes photographs of different parts of a rose which has been immersed in a trimer solution in accordance with at least one embodiment of the invention;
Figure 12 shows photographs of wires formed in the xylem of the rose in accordance with at least one embodiment of the invention;
Figure 13 shows scanning electron micrographs (SEM) of a cross-section of a rose stem with polymer filled and coated xylem in accordance with at least one embodiment of the invention;
Figure 14 and 15 shows images of petrioles with polymer filled transport channels in accordance with at least one embodiment of the invention;
Figure 16A-E are photographs of an experiment where roots of a young bean plant were immersed of in a trimer solution;
Figure 17 and 18 are magnified views of the roots of a bean plant after immersion in trimer solution;
Figure 19A-D are magnified views of root hairs growing from a root which have been immersed in trimer solution;
Figure 20A-C shows photographs and magnified views of a root and cuts of a root which was immersed in trimer solution;
Figure 21A-F are photographs outlining an experiment step-by-step for creating an electrically conducting wire inside a plant with roots in accordance with at least one embodiment of the invention;
Figure 22A-C are photographs of a bean plant with roots immersed in trimer solution in accordance with at least one embodiment of the invention;
Figure 23A-C shows conductivity measurements along a vein in a petiole a leaf;
Figure 24A-C shows external conductivity measurements along a petiole;
Figure 25A-C shows external conductivity measurements along the stem of the petiole of figure 24;
Figure 26A-B shows external conductivity measurements along a stem of a petiole;
Figure 27A-C shows external conductivity measurement between a stem and petiole junction;
Figure 28A-C shows an external conductivity measurement between a stem and petiole junction;
Figure 29A-C shows a conductivity measurement for an electrically conducting wire formed in a xylem;
Figure 30A-C shows the electrochemical charachterization of the onset potential of the electropolymerization of a polymerized compound 1 and the charachterization of the resulting film;
Figure 31A-C shows are phototgraphs outlining an experiment step by step for investigating the polymerization of the compound 1 on different parts of a plant;
Figure 32A-C are photographs outlining an experiment step by step for investigating the effect of oxygen on the polymerization of compound 1; and
Figure 33 shows a time resolved absorption spectra of a compound 1 solution with and without plant material.
Figures 34 - 43 shows images related to a super-capacitor embodiment.

### Detailed description

In the following detailed description, the general inventive concept of electrical components, such as electronic devices comprising organic part(s), and methods of introducing electrical components into an organic part, as well as compounds facilitating this will be explained and are mainly discussed with reference to figures showing either schematical representations or experiments. It should be noted that this by no means limits the scope of the invention, which is also applicable in other circumstances for instance with other types or variants of materials, plants or method for introducing materials forming electric components into plants. Further, that specific features or details are mentioned in connection to embodiments while explained in conjunction with the figures does not mean that those components, steps, methods, materials and so on cannot be used to an advantage together with other embodiments of the invention.

### Introduction

Figure 1 is a schematic view of a plant, and the vascular system in a plant. A plant typically has roots which extend below the soil. The roots may have root hairs which further increase the surface area of the roots towards the soil, and thereby allows the plant to draw in, or absorb water and macro- and micronutrients. In more detail; a plant may be started by sowing a seed into the soil; oxygen, temperature and water, along with various chemicals and pH, are prime triggers of germination and embryonic growth. Eventually, the seedling becomes a matured structure where different signals, components and pathways work together to regulate plant functions. In the root, which is composed of the primary root and root hair, water is taken up in addition to an array of macro-(e.g. NO3-, N2PO4-, K+) and micro-nutrients (e.g. Fe2+, Zn2+), see figure 1. Water solution is pumped upwards, according to the cohesion-tension mechanism, along the xylem vascular system towards the leaves. The leaves are comprised of the upper and lower epidermis, the stoma-guard cell pathways that gates CO₂-O₂ and the palisade and spongy parenchyma (mesophyll) that includes the photosynthetic system and the CO2-O2 exchanger, respectively. In the leaves, the production of hydrocarbons is powered by sunlight with H₂O and CO₂ as the reactants. The primary and secondary products from photosynthesis are many; sucrose and a vast array of plant hormones such as auxin, cytokinin etc. These products are administrated throughout the plant along the phloem vascular system by hydrostatic pressure, which delivers plant hormones and sugar to sinks and growth sites. In addition, there are several fine-structured veins and different gates that regulate the dispensing and exposure of compounds [1].

Hence, carbohydrates, plant hormones and nutrients are long-distance transported along the phloem and the xylem vascular systems to selectively trigger and modulate processes at high spatiotemporal resolution. Hence, water and macro-and micronutrients absorbed by the roots may be transported to the other various parts of the plant such as the stem and leaves. Note that the Xylem vascular structures normally only transports materials from the root, or other parts to the outer ends of the plant, whereas the Phloem may transport materials in both direction. In any case, further details on the biology and functionality of plants as presently understood may for example be found in reference [1].

However, artificial regulation, as interaction by man directly or indirectly, of plant processes are in 2015 generally achieved by exposing plants to chemicals (nutrients and hormones) in a highly systemic manner. Hence, many long-standing questions in plant biology are still left unanswered due to the lack of means or technological advancement focused on precisely regulating plant function, in vivo.

It is therefore desired to record, address and regulate local and isolated, but also connected, plant functions (even at the single cell level) in a highly complex and spatiotemporal manner. Specifically, plants having electronic components therein, henceforth known as electronic plants or e-plants, that locally convert plant chemistry into electricity and other chemical end-products may enable new ways to harvest energy from photosynthesis and nature in general. Further, molecular-genetic modification techniques aim at dictating and modifying plant physiology in an effort to enhance plants themselves or to improve their performance e.g. the time a plant sets fruit may be regulated forwards or backwards in time. It is suggested that electronic plant technology may provide a new platform and pathway as a complement or alternative to existing genetic techniques in plant biology.

Natural and synthetic organic electronic materials (molecules and polymers) and devices can conduct and process electronic and ionic signals in a tightly coupled fashion. Based on this, organic devices and circuits than can convert electronic addressing signals into a highly specific and complex delivery of chemicals, and vice versa, in order to regulate and record various functions and processes in biology [3, 4], may be built, manufactured or generated.

This may allow the regulation and control of plant physiology and functions, from the event of seeding to the state of a fully matured plant, and to electroactively convert plant chemicals into desired products, e.g. electrical energy and chemical end products.

Organic electronic materials - amorphous or ordered electronic and iontronic polymers and molecules - can be manufactured into devices or systems that exhibit a unique combination of properties and can be shaped into almost any form using soft, and even living systems [107] as the template [108].

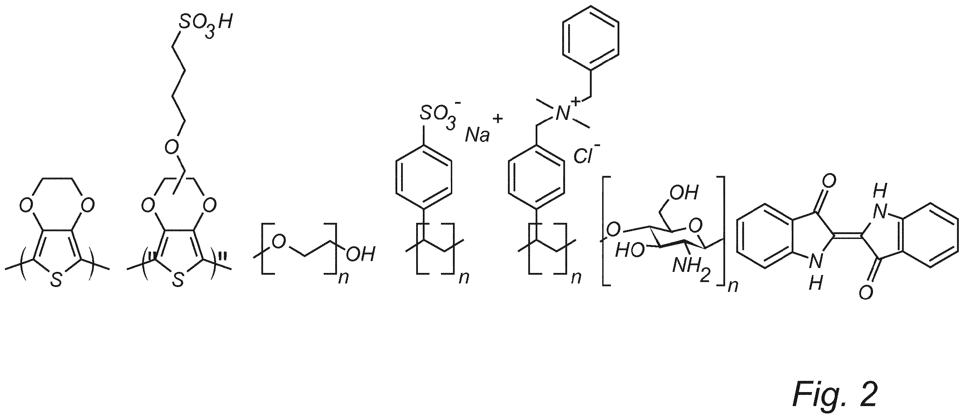

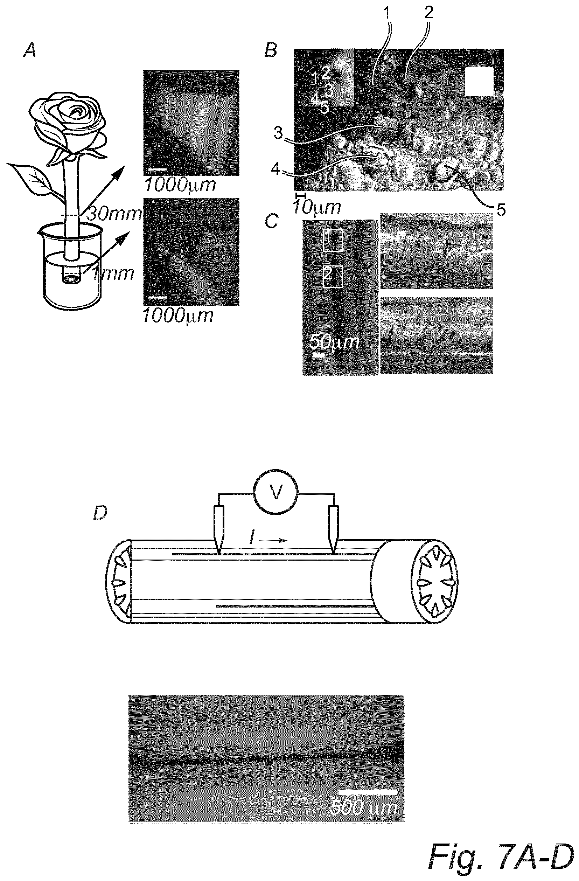

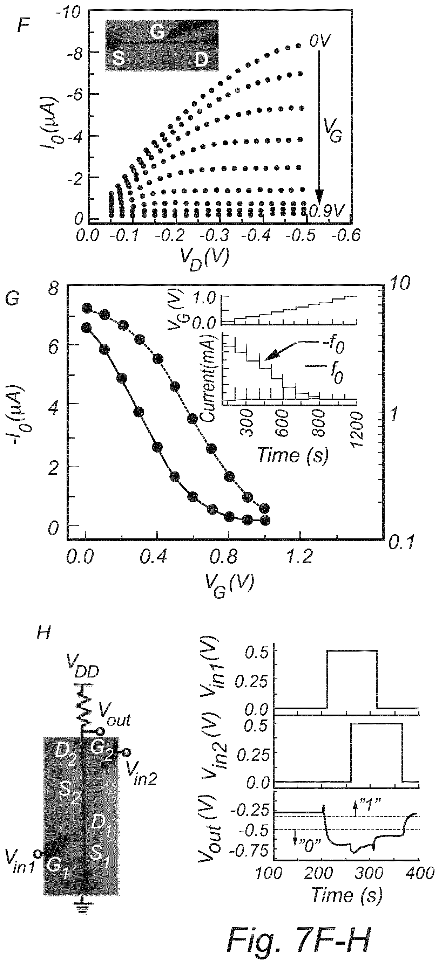

The electronically conducting polymer poly(3,4-ethylenedioxythiophene) (PEDOT) [109], either doped with polystyrene sulfonate (PEDOT:PSS) or self-doped [1010] via a covalently attached anionic side group (e.g., PEDOT-S:H [108]) is one of the most studied and explored organic electronic materials, see figure 2. The various PEDOT material systems typically exhibit high combined electronic and ionic conductivity in the hydrated state. Its electronic performance and characteristics are tightly coupled to charge doping, where the electronically conducting and highly charged regions of PEDOT+ require compensation by anions, and the neutral regions of PEDOT0 are uncompensated. This "electrochemical" activity has been extensively utilized as the principle of operation in various organic electrochemical transistors (OECTs) [1012], sensors [1013], electrodes [1014], super-capacitors [1015], energy conversion devices [1016] and electrochromic display (OECD) cells [109, 1017]. PEDOT is versatile from a circuitry fabrication point of view, as contacts, interconnects, wires, and devices, all based on PEDOT:PSS, have been integrated into both digital and analogue circuits, exemplified by OECT-based logical NOR gates [1018] and OECT-driven large-area matrix-addressed OECD displays [1017], see figure 7b.

Electronically and electrochemically active organic materials, i.e. semiconducting, semi-metallic and metallic polymers and molecules, are today widely explored in various applications within ICT (information and communication technology). The organic materials are soft, flexible, combine fast ionic and electronic conductivity and can be decorated with chemical functionality. As the bridge between electronics and biology; various kinds of sensors, neuronal electrodes, chemical circuits, drug delivery devices [8] and active scaffolds [9] have been developed to record and regulate physiology, at high chemical specificity and spatiotemporal resolution.

Scientists around the world have considered or explored introducing electronic materials, not devices nor circuits, into plants in the past. For instance, metal nanoparticles [10], nanotubes [11] and quantum dots [12] have been applied to plant cells, seedlings and/or matured plants to impact various properties and functions, related to growth, photosynthesis and antifungal efficacy [13]. None of these discloses a continous electronic conductor arranged in an organic part. In e.g. [11] a nanotube is arranged withing the cell, but consequently there is no continuous electronic conductor formed as nanotubes are separated from each other.

It is suggested that e.g. synthetic [14] and natural [15, 16] organic electronic and iontronic [17] materials, such as the (semi)conductors (e.g. PEDOT, Indigo), polyelectrolytes (e.g. PSS:H; q-PVBC) and polymer electrolytes (e.g. PEO, chitosan), combining desired ionic, chemical, electronic and/or electrochemical functionality may be utilized to make devices and circuits in vivo. Figure 2 shows materials which were explored, or were suggested to be explored, during the research project related to electronic plants. In figure 2, from left to right is shown: PEDOT (conjugated polymer), PEDOT-S:H (conjugated polyelectrolyte), polyethylene glycol (polymer electrolyte), PSS (polyanion), q-PVBC (polycation), chitosan (natural polymer electrolyte) and indigo (natural (semi)conductor). Moreover, other synthetic and natural organic electronic materials may be designed and developed.

Selected materials can be manufactured into desired geometries and circuitries e.g. using the different structures and components of the plant as the template and also as an integral part of the device functionality. Thereby forming e.g. hydrogel, dendrite, wire, bi-continuous network and self-organized/assembled geometries using e.g. flow-based delivery, dry-phase-, electro- or chemical polymerization strategies in vivo, or within close proximity to, the plant structures.

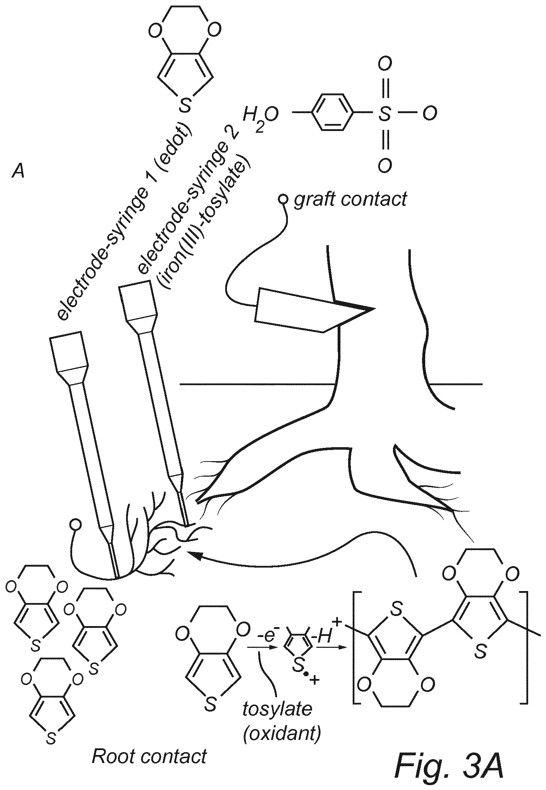

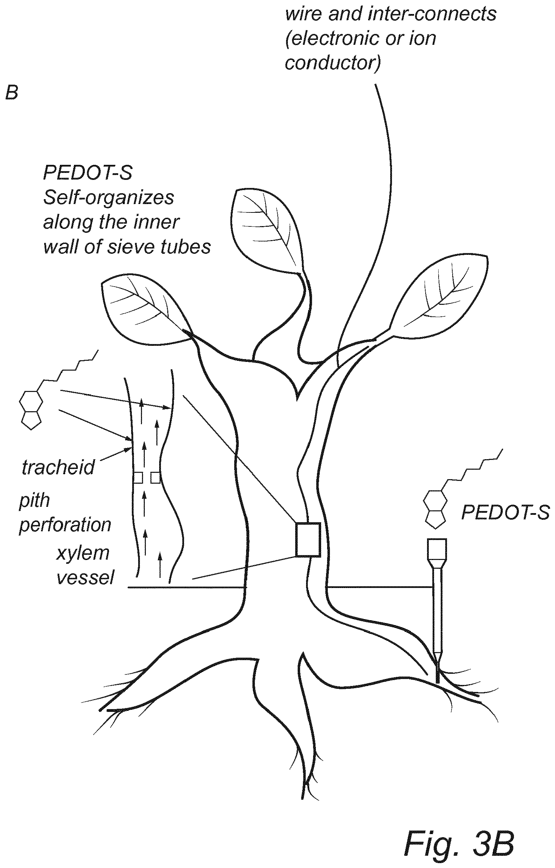

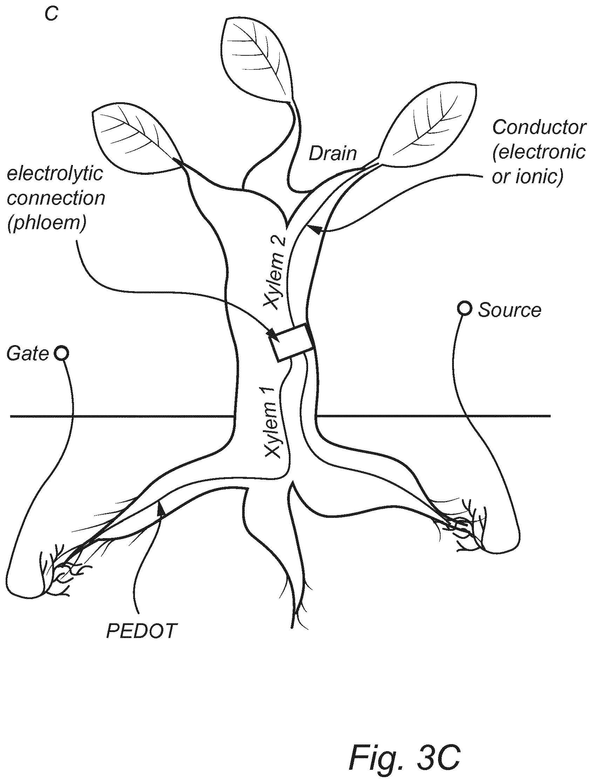

It is suggested to derive the four key-components of an integrated circuit in plants, in vivo, and those are: electronic and/or electrochemical and/or iontronic contacts, wires and inter-connects and devices. This idea is shown in figure 3 a-c. In figure 3 a-c electronic and electrochemical contacts is achieved using for instance dendrite electrodes manufactured in the soil and also by grafting inoculated electrodes into the stem of plants. Distributed wires and interconnect networks is achieved along the vascular systems of plants by arranging electronic and ionic conductors along the xylem and/or phloem channels.

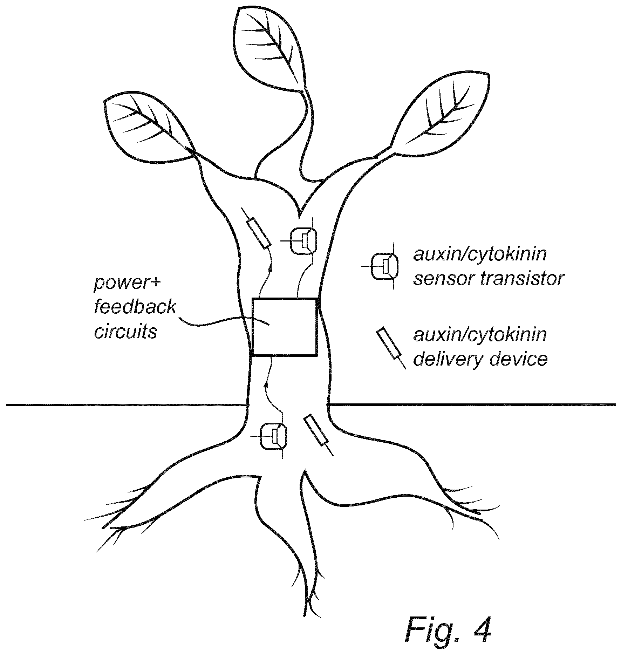

It is suggested to achieve self-organization of conductors along the inner wall, i.e. the periphery, or inner wall, of extended xylem channels and/or phloem channels. Electronic devices are thus formed and distributed along the various constructs of the plant, e.g. in the leaf along the stem and in the root. For instance, electrochemical or ion transistors, for signal routing, dispensing or sensing in the stem can be defined at specific positions along the stem, by combining two conducting xylem wires with a current modulating junction, see e.g. figure 4c.

Devices may also include enzymes, ion conductor membranes, electrochemical electrodes, to enable selective translation between chemical and electronic signals [18]. Electrodes and devices of various kinds can be manufactured, such as transistors, sensors and capacitors, rectifiers and fuel cells [19, 20]. Moreover, various organic chemical and electronic circuit systems can be constructed to convert electronic addressing signals into complex delivery of chemical signal patterns, and vice versa. The circuit or circuit systems can include devices and circuits that transport and/or adapt electronic and/or ionic signals, i.e. forming electronic, electrochemical and/or iontronic organic devices. Electronic devices are preferably based on solid-state organic semiconductors, and electrochemical devices are typically based on the combination of organic semiconductors and ion conductors (electrolytes), and the iontronic devices are built up from the combination of ion selective (bipolar) membranes. Hence, in its full scale, the plant may host a distributed and integrated system composed of contacts, interconnects, devices and circuit wiring. Soil

It is suggested in a first experiment PEDOT fibre scaffolds will be polymerized in the soil to enable electronic control over the potential and also the charge polarization state of a 3D soil electrode scaffold. Those electrodes will also be combined with anchored enzymes to achieve chemical-to-electronic energy conversion and also sensing.

The efficacy of ion diodes to electronically control pH of the soil may be explored via water splitting. For example, at high reverse potentials, water dissociation occurs along polyanionic (PSS:H)-polycationic (q-PVBC) interfaces, thus producing electronic control over pH, locally and as gradients. This can be achieved also by use of other substances.

Coaxial ion conductor (PSS, q-PVBC, i.e. the same materials as in the foregoing; polyanionic (PSS:H)-polycationic (q-PVBC) interfaces) fibres, having repeating outlets, may be constructed to provide active control of delivery and the harvesting of nutrients and plant products. In other words, according to one example, by varying the potential applied to the electrically conducting coaxial ion conductor fiber arragned in the transport channel of the organic part, the substances transported in the transport channel may be varied and/or selected. In an extension, each outlet may include ion bipolar membrane transistors for exclusive and addressed gating of delivery into the soil (spatiotemporal resolution), more information on such devices may for example e.g. be found in WO2010119069.

### Roots

It is suggested that electrode dendrites may be grown, along conductor wires immersed into soil, to serve as charge collector and stimulating scaffold for root (hairs). These "e-root hairs" twin and intermix with the plant's natural roots, where growth of the electrode dendrites is potentially also guided by an electric potential applied to the root of the plant. See e.g. figure 3A.

The same polymerisation techniques may be used to generate electrode and conductor networks within the vascular system of roots and hairs. Electrodes can be made along with the inclusion of *e.g.* nanoparticles as immobilizing anchors, to make enzymatic energy conversion or sensing possible. Specific enzymes, e.g. sucrose oxidase/invertase, cytokinin dehydrogenase, will be harboured at the anchoring sites to derive sucrose-to-electricity and hormone-to-sensor-signal conversion at "*e*-root hair" electrodes.

Ion fibre diodes and conductors, coated with an impermeable cladding, are constructed inside the vascular pathways of the roots. Those channels and components may serve as transportation highways, rectifiers and water splitters, respectively, to deliver and express gradients and signals of nutrients and pH inside roots.

### Stems

According to one example, the vascular pathways of the xylem and phloem are typically comprised of ∼100 µm wide vessels. The xylem system is normally entirely unidirectional, and for many plants each vessel bundle exclusively connects the bottom compartment with selective structures at the top. This opens up for the construction of well-isolated and exclusively addressable pathways for electronic and ionic signals within plants.

Interconnects and contacts may transfer addressing signals from the outside through both the peripheral coatings of the plant and the phloem to connect with individual bundles or even individual vessels of the xylem. Electronic as well as electrochemical electrode grafts may be manufactured together with ion conductors, diodes and transistors. Similar interconnects may also connect to the phloem vascular system, then targeting systemic delivery and conduction of signals within plants.

Electronic signals and also reactants are introduced into the vascular system of plants to in vivo-synthesize electronic and ion conduction pathways throughout the plant. These pathways may be utilized to deliver and receive signals, and to define distributed e-Plant circuit boards.

### New functionality

The leaves are versatile antennas and factories that e.g. collect, emit and produce e.g. elctromagnetic energy, CO2, O2, H₂0 and carbohydrates. The components, structures and vascular systems of the leaves offers an array of opportunities to define, distribute and connect electronic devices within the leaves and in fact within the entire plant, and to use naturally occuring products and components of the leaves as an integral part of device functionality, i.e. to offer new functionality.

Along the xylem pathway, monomeric units (EDOT), enzymes, and oxidation agents can be administrated within the veins, and further also delivered out into the to the palisade and spongy parenchyma. Through the stomas, being typically 1-5 µm in diameter, an array of vapour-phase or dissolved chemicals can be introduced to initiate polymerisation or to integrate and complete existing device architectures.

According to one example, the structure and functions of the veins, the spongy and palisade parenchyma and the stomas, together defines a very interesting platform for device architecture and functionality. Several device concepts may be obtained for energy conversion, production of chemicals and to twin biological compounds with synthetic materials to define devices and systems.

Plants are lacking central and peripheral nervous systems that emit, transport, receive and process various signals that collectively dictate functions in a coordinated manner. However, plants utilize the coupling of different hormones via specific feedback loops to regulate, for instance, growth and to combat various traumas that a plant might experience. For instance, auxin, generated by the apical buds, travels down the shoots to inhibit axillary bud growth. This stimulates the growth of shoots while at the same time suppressing lateral branching. Conversely, cytokinin moves upwards from the roots into the shoots to stimulate lateral bud growth. The competition of the auxin-to-cytokinin ratio is known to regulate the balance between apical and axillary growth. Cytokinins in plants have also been found to aid or control disease resistance and combat traumas. Here, generic integrated electronic-chemical circuitry or circuits are proposed that are composed of one or more of transport wires, feedback circuits, together with enzymatic sensors and delivery devices for plant hormones as shown in figure 4. The circuits may provide fast and electronically controlled regulation of the balance between different plant hormones to regulate growth, prevent diseases and to promote the expression of specific structures of plants.

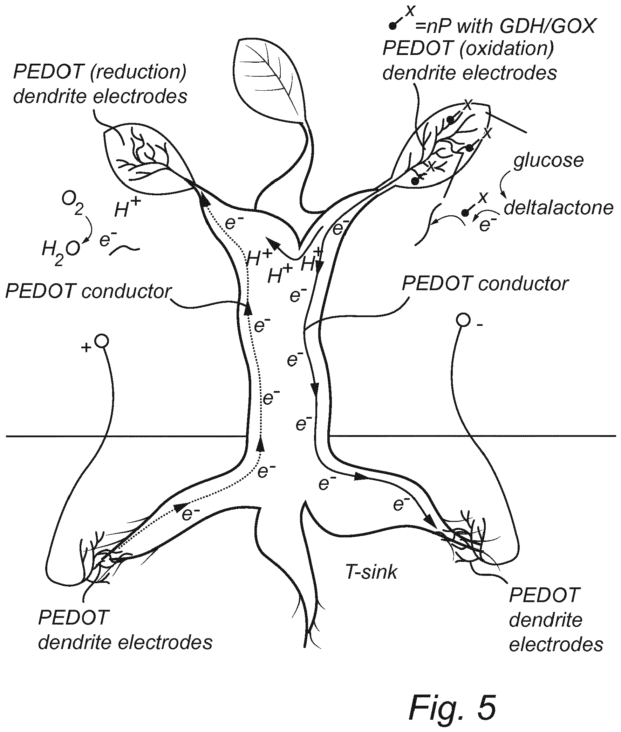

Simple sugars such as glucose, sucrose etc. are produced in the palisade parenchyma and released into the phloem. In an enzymatic fuel cell, glucose can be oxidized into gluconolactone via enzymatic oxidase reaction accompanied by the generation of protons and the collection of electrons at the electrodes. The counter reaction includes reduction of oxygen, electron transfer and the consumption of protons to produce H₂O. Such enzymatic electrode systems inside leaves, in the roots and/or close to the auxiliary junctions of the plant, i.e. at components that contain high levels of sugar and/or O2. Swift transfer channels of protons along phloem gradients may also be developed. Establishing electronic wiring along the tranport channel, e.g. the xylem, enables the xylem to transfer charges to external electrodes. The resulting system will represent a generic platform to realize energy conversion technology inside living plants, that e.g. by consuming the natural products of the plant may be used to produce electricity, as shown in figure 5.

Plants produce cellulose, lignin and hemicellulose together with an array of chemicals. The balance and outcome of the production of these materials and compounds are governed by a combination of different environmental parameters, the availability of nutrients, and internal and external signals. It is suggested that distributed electrodes and solid-state, electrochemical and iontronic device circuits are used to regulate the production and expression of some of the products of plants. Such as the expression and exposure of electric potential, pH, plant hormones and nutrients to regulate the production of natural materials will be explored. As an extension, the distributed electronic plant circuits or circuitry may also enable the production of completely new compounds and materials from plants.

An electronic plant may thus be used as batteries and/or energy/charge storage devices. Electronic functionality may also be introduced into plants that is maintained and further utilized in converted wood or cellulose-based products. The electronic technology also provides a regulator tool complementary to existing genetic and molecular techniques used in plant science. Furthermore, thermoelectric plants may convert natural temperature gradients in a forest into electricity.

New materials, devices and systems are needed to make electronics a truly recyclable technology. e-Plants defines one new path towards a green intelligent electronics technology.

With e-Plants, in-vivo regulation and promotion of specific end products is possible. It is foreseen that such technology then can also provide a new regulation technology to enhance the production of specific existing or completely new end products from plants. With an electroactive regulation of, for instance, carbohydrate chemistry, one could envision the possibility of plants becoming factories of new materials and compounds as well - e-Plant Factories.

Plants are natural converters of solar energy into chemical energy. Several materials and technologies exist that convert chemical energy into electricity. Fuel cell concepts fall short due to a lack of proper efficiency or capacity. It is foreseen that the e-Plants concept can be further refined to promote plants to produce higher quality chemicals to be utilized in high-performing fuel cells or energy converting/storing systems.

### Initial experiments

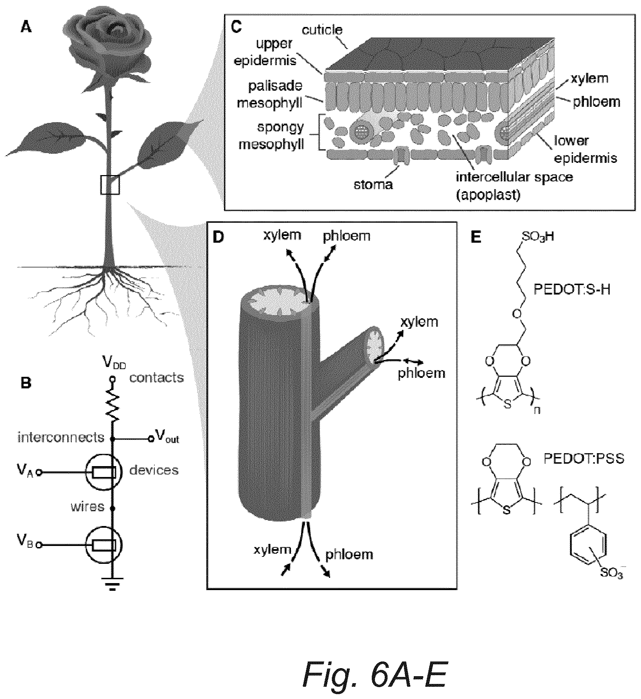

A few initial experiments related to the e-Plant research is presented here. In a first experiment, electronic ion pumps were manufactured as stand alone devices and then applied to the root of Arabidopsis seedlings. From this early experiment we record a control of the growth pattern of the seedling. In a second experiment, we produced several centimetre long electronically conducting wires inside the xylem vascular system of roses, see Figure 6A-H. Further, PEDOT:PSS has successfully been administrated inside the leaves of roses, see Figure 7A-H.

### Detailed description of experiments and example embodiments performed

In the past, artificial electro-active materials have been introduced and dispensed into living plants. For instance, metal nanoparticles [1019], nanotubes [1020] and quantum dots [1021] have been applied to plant cells and the vascular systems [1022] of seedlings and/or matured plants to impact various properties and functions related to growth, photosynthesis and antifungal efficacy [1023]. However, the complex internal structure of plants has never been used as a template for in situ fabrication of electronic circuitry. Given the versatility of organic electronic materials - both in terms of fabrication and function - the first tests and experiments were made to investigate introducing electronic functionality into plants by means of PEDOT.

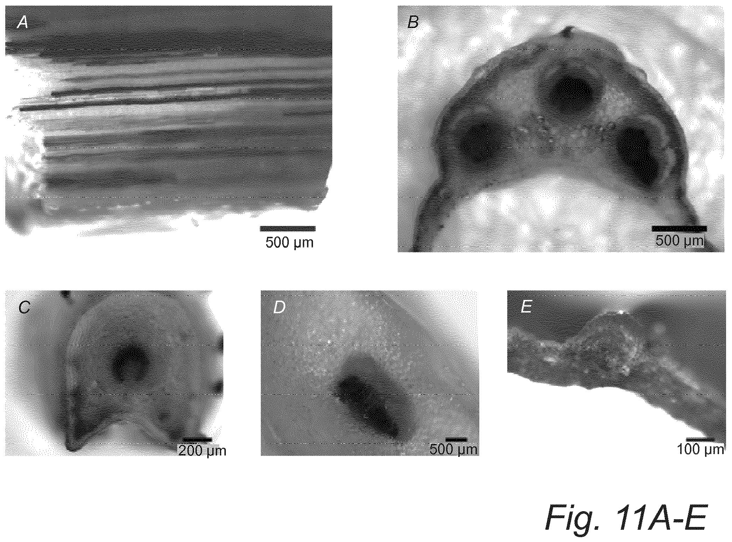

Figure 7A-H, with references to the alphabetical parts of figure in parenthesis, shows: Electronically conducting xylem wires, transistors, and digital logic. (B) Forming PEDOT-S:H wires in the xylem. A cut rose was immersed in PEDOT-S:H aqueous solution, and PEDOT-S:H is taken up self-organizes along the xylem forming conducting wires. The optical micrographs show the wires 1 mm and 30 mm above the bottom of the stem (bark and phloem were peeled off to reveal the xylem). (B) SEM image of freeze-dried rose stem cross section showing xylem (1-5) filled with PEDOT-S:H. The inset shows the corresponding optical micrograph, where the filled xylem have the distinctive dark blue color of PEDOT. (C) SEM images (with corresponding micrograph at left) of freeze-dried stem along xylem, showing hydrogel-like PEDOT-S structure. (D) Schematic of conductivity measurement using Au probes as contacts. (E) I-V characteristics of PEDOT-S xylem wires of different lengths, L₁ = 2.15 mm, L₂ = 0.9 mm, L₃ = 0.17 mm. The inset shows resistance vs length/area and linear fit giving conductivity of 0.13 S/cm. (F) Output characteristics of the xylem-OECT. Inset shows the xylem wire as source, S, and drain, D, with gate, G, contacted through the plant tissue. (G) Transfer curve of a typical xylem-OECT for *V*_{D} = -0.3 V (solid line corresponds to linear axis, dashed line to log axis). The inset show temporal response of *I*_{D} and *I*_{G} relative to increasing *V*_{G}. (H) Logical NOR gate constructed along a single xylem wire. The circuit diagram indicates the location of the two xylem-OECTs and external connections (compare with circuit in Fig. 11B). Voltage traces for *V*ᵢₙ₁, *V*ᵢₙ₂, and *V*ₒᵤₜ illustrate NOR function. The dashed lines on the *V*ₒᵤₜ plot indicate thresholds for defining logical 0 and 1.

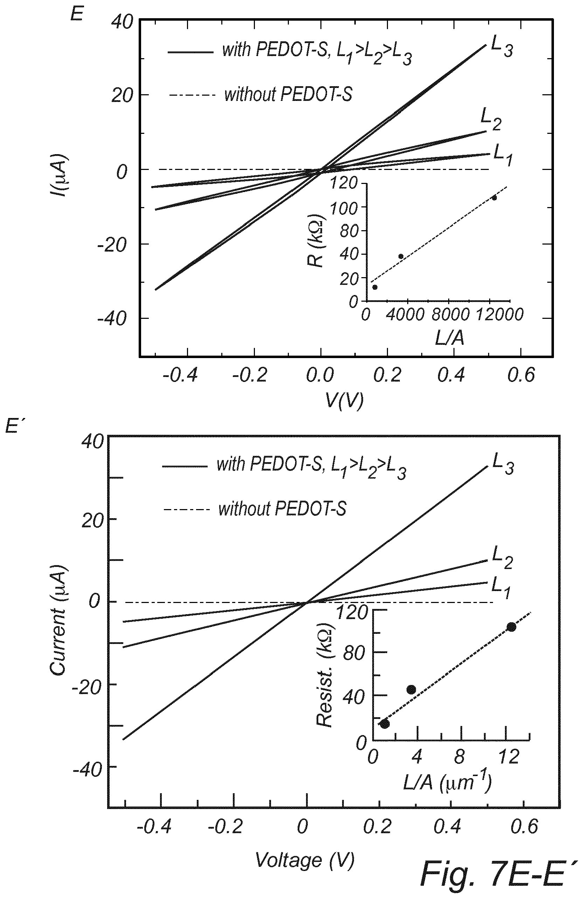

We chose to use cuttings of *Rosa floribunda* (garden rose) as our model plant system. The lower part of a rose stem was cut and the fresh cross section was immersed in an aqueous PEDOT-S:H solution for 24-48 h (Fig. 7A), during which time the PEDOT-S:H solution was taken up into the xylem vascular channel and transported apically. The rose was taken from the solution and rinsed in water. The outer bark, cortex and phloem of the bottom part of the stem were then gently peeled off, exposing dark continuous lines along individual 20-100 µm wide xylem channels (Fig. 7B-D). These "wires" extended more than 5 cm along the stem. From optical and scanning electron microscope images of fresh and freeze-dried stems, we conclude that the PEDOT-S:H formed dense and homogenous wires occupying the entire xylem tubular channel. PEDOT-S:H is known to form hydrogels in aqueous-rich environments, in particular in the presence of divalent cations, and we assume that this is also the case for the wires established along the xylem channels of rose stems. The conductivity of PEDOT-S:H wires was measured using two Au probes applied into individual PEDOT-S:H xylem wires along the stem (Fig. 7D). From the linear fit of resistance versus distance between the contacts, we found electrical conductivity reading 0.13 S/cm with contact resistance ∼10 kΩ (Fig. 7E). To form a solid and continuous wire along the inner surface and volume of a tubular structure, such as a xylem channel, by exposing only its tiny inlet to a solution, one must rely on a subtle thermodynamic balance of transport and kinetics. The generation of the initial monolayer along the inner wall of the xylem, along with the subsequent reduction in free energy of PEDOT-S:H upon formation of a continuous hydrogel, should preferably be in proper balance with respect to the unidirectional flow, entropy and diffusion properties of the solution in the xylem. Initially, we explored an array of different conducting polymer systems to generate wires along the rose stems. For some of these materials clogging of the materials already at the inlet, or no adsorption of the conducting material along the xylem was observed. In those cases, we conclude that the balance between transport, thermodynamics and kinetics does not favor the formation of wires inside live xylem vessels.

After immersing the rose stem into the aqueous solution, dissolved PEDOT-S:H chains migrated along the xylem channels, probably driven by the upward cohesion-tension transportation of water. The stem was then immersed in 1 mg/ml PEDOT-S:H in DI water, and kept at approx. 40 % humidity and 23 °C. Experiments were also performed at 70 % humidity, but no significant difference on the absorption was observed. The rose was kept in the PEDOT-S solution for approx. 48 h.We hypothesize that a net influx of divalent cations into the xylem occurred, which then increases the chemical kinetics for PEDOT-S:H to form a homogenous and long-range hydrogel conductor phase along the xylem circuitry. The surprisingly high conductivity of >0.1 S/cm of these extended PEDOT-S:H wires suggests that swift transport and distribution of dissolved PEDOT-S:H chains along the xylem preceded the formation of the actual conductive hydrogel wires.

These long-range conducting PEDOT-S:H-xylem wires, surrounded with cellular domains including confined electrolytic compartments, are promising components for developing in situ OECT devices and other electrochemical devices and circuitry. We therefore proceeded to investigate transistor functionality in the xylem wires. A single PEDOT-S:H xylem wire served simultaneously as the transistor channel, source, and drain of an OECT. The gate comprised a PEDOT:PSS-coated Au probe coupled electrolytically through the plant cells and extracellular medium surrounding the xylem (Fig. 7F, inset). Two additional PEDOT:PSS-coated Au probes defined the source and the drain contacts. By applying a positive potential to the gate electrode (VG) with respect to the grounded source, the number of charge carriers in the OECT channel is depleted, via ion exchange with the extracellular medium and charge compensation at the gate electrode. This mechanism defines the principle of operation of the xylem-OECT. The device exhibited the expected output characteristics of an OECT (Fig. 7F). *I*_{D} saturation is also seen, caused by pinch-off within the channel near the drain electrode.

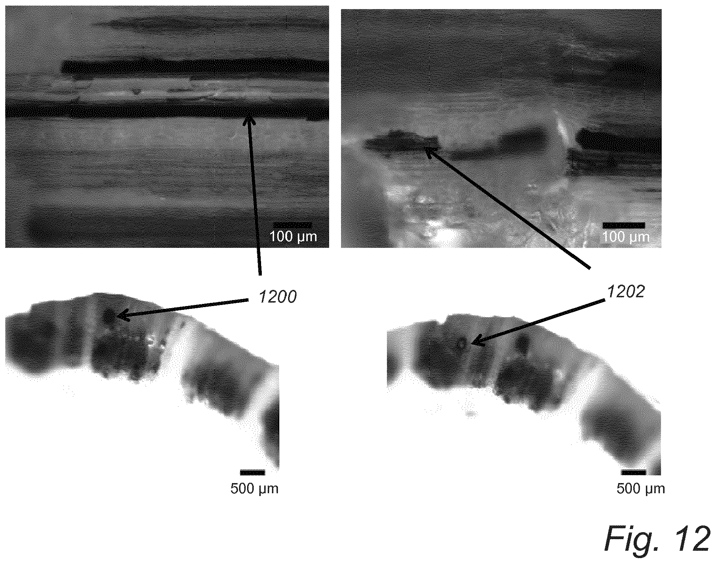

Figure 7G shows the transfer curve, along with the evolution of *I*_{D} and IG as VG was stepped up. From these measurements we calculate an *I*_{D} on/off ratio of -40, a transconductance (Δ*I*_{D}/Δ*V*_{G}) reaching 14 µS at *V*_{G} = 0.3 V, and very little current leakage from the gate into the channel and drain (∂*I*_{D}/∂*I*_{G} > 100 at *V*_{G} = 0.1 V). With OECTs demonstrated, we proceeded to investigate more complex xylem-templated circuitry, namely xylem-logic. Two xylem-OECTs were formed in series by applying two PEDOT:PSS-coated Au gate probes at different positions along the same PEDOT-S:H xylem wire. The two OECTs were then connected, via two PEDOT:PSS-coated Au-probes, to an external 800 kΩ resistor connected to a supply voltage (*V*_{DD} = -1.5 V) on one side, and to electric ground on the other side (Fig. 12H). The two gate electrodes defined separate input terminals, while the output terminal coincides with the drain contact of the "top" OECT (that is, the potential between the external resistor and the OECT). By applying different combinations of input signals (0 V as digital "0" or +0.5 V as "1"), we observed NOR logic at the output, in the form of voltage below -0.5 V as "0" and above -0.3 V as "1".

In addition to xylem and phloem vascular circuitry, leaves additionally comprise the palisade and spongy mesophyll, sandwiched between thin upper and lower epidermal layers (Fig. 7C). The spongy mesophyll, distributed along the abaxial side of the leaf, contains photosynthetically active cells surrounded by the apoplast: the heavily hydrated space between cell walls essential to several metabolic processes, such as sucrose transport and gas exchange. Finally, the stomata and their parenchymal guard cells gate the connection between the surrounding air and the spongy mesophyll and apoplast, and regulate the important O₂-CO₂ exchange. Together, these structures and functions of the abaxial side of the leaf encouraged us to explore the possibility of establishing areal - and potentially segmented - electrodes in leaves in vivo.

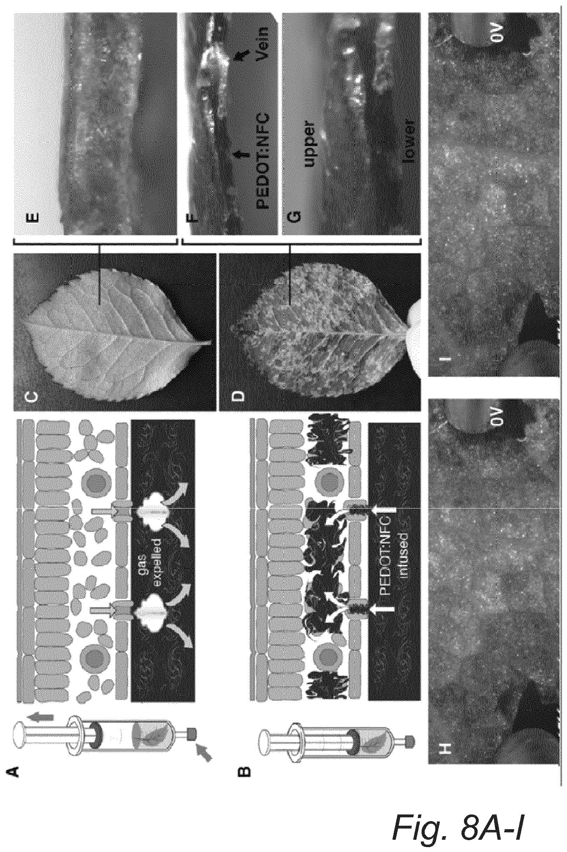

Figure 8A-I, with references to the alphabetical parts of figure in parenthesis, shows: PEDOT-loaded leaves and leaf-OECDs. (A) is vacuum infiltration, a leaf placed in PEDOT:PSS-NFC solution in a syringe with air removed. The syringe is pulled up creating negative pressure causing the gas inside the spongy mesophyll to be expelled. (B,B') When the syringe returns to standard pressure, PEDOT:PSS-NFC is infused through the stomata filling the spongy mesophyll between the veins. (C) Photograph of the bottom and (D) cross section of a pristine rose leaf before infiltration. (D) Photograph of the bottom and (F,F',G) cross section of leaf after PEDOT:PSS-NFC infusion. (H, I) Electrochromism in PEDOT:PSS-NFC infused leaf. (J, K) False-color map of change in grayscale intensity between application of (J) +15 V and (K) -15 V. The gray areas, i.e. lighter colors, represent green areas in a color picture, and the light areas represents a positive increase (light to dark). (L, M) Grayscale values of pixel intensity along the lines indicated in (J) and (K) showing successive oxidation/reduction gradients, and a plot of the change in grayscale intensity over a fixed line showing the change and oxidation/reduction gradations versus distance. (N) Visualization of the electric field in the leaf-OECD via the induced electrochromic gradient directions (cf. Ref. [30]). (O) Electrical schematic representation of n-compartments modeling both electrical and ionic components of the current.

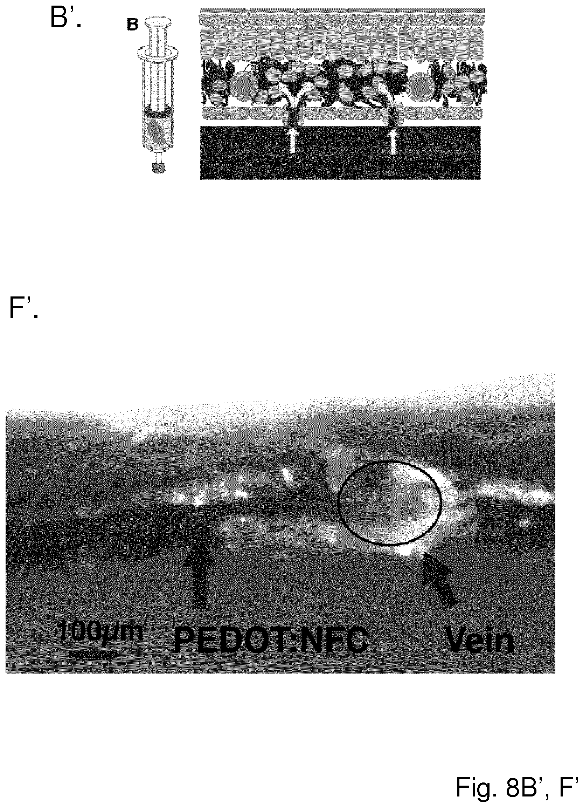

Vacuum infiltration [1025, 1026] is a technique commonly used in plant biology to study metabolite [1027] and ion concentrations in the apoplastic fluid of leaves. We used this technique to "deposit" PEDOT:PSS, combined with nanofibrillar cellulose (PEDOT:PSS-NFC) into the apoplast of rose leaves. PEDOT:PSS-NFC is a conformable, self-supporting, and self-organized electrode system that combines high electronic and ionic conductivity [1028]. A rose leaf was submerged in a syringe containing said aqueous PEDOT:PSS-NFC solution. The syringe was plunged to remove air, sealed at the nozzle, and the plunger was then gently pulled to create vacuum, see figure 8A, thus forcing air out of the leaf through the stomata. As the syringe returned to its original position, PEDOT:PSS-NFC was drawn in through the stomata to reside in the spongy mesophyll, see figure 8B. A photograph of a pristine leaf and microscopy of its cross section, see figure 8C and 8D, are compared to a leaf infiltrated with PEDOT:PSS-NFC, see figure 8E and 8F. PEDOT:PSS-NFC appeared to be confined in compartments, along the abaxial side of the leaf, delineated by the vascular network in the mesophyll, see figure 8F. The result was a leaf comprised of a 2D network of compartments filled - or at least partially filled - with the electronic-ionic PEDOT:PSS-NFC electrode material. Some compartments appeared darker and some entirely unchanged in color, suggesting that the amount of PEDOT:PSS-NFC differed between compartments.

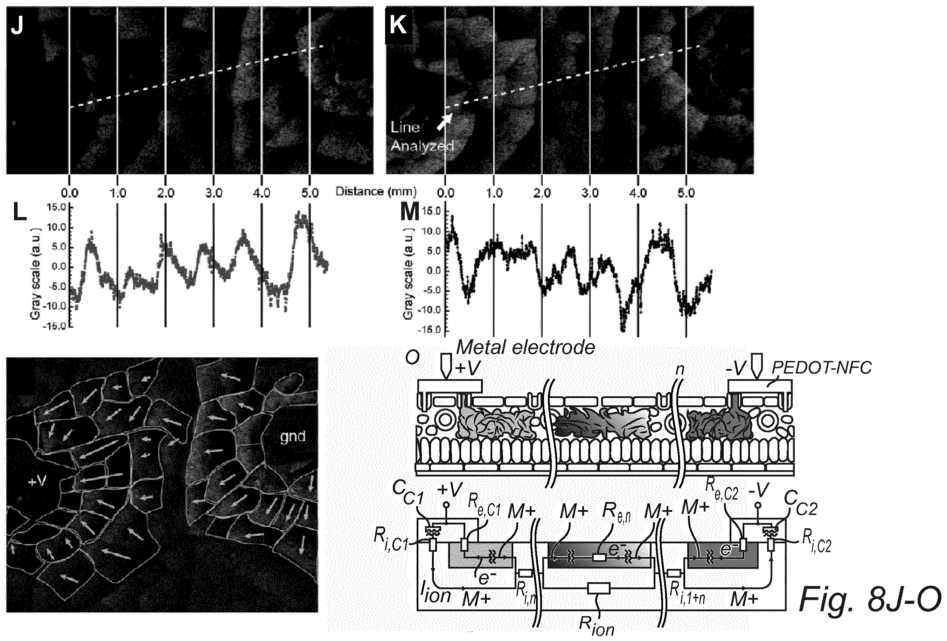

We proceeded to investigate the electrochemical properties of this 2D circuit network using free-standing PEDOT:PSS-NFC films (1-2 mm², 90 µm thick, ∼19 S/cm conductivity, ionic charge capacity ∼0.1 F) placed on the outside of the leaf, providing electrical contacts through the stomata to the material inside the leaf. We observed typical charging-discharging characteristics of a two-electrode electrochemical cell, while observing clear electrochromism compartmentalized by the mesophyll vasculature, see figure 8G and 8H. Upon applying a constant bias, steady-state electrochromic switching of all active compartment typically took less than 20 s, and the effect could be maintained over extended period of times (> 10 min). Likewise, when the voltage was reversed, the observed light-dark pattern was flipped within 20 s. The electrochromism can be quantified by mapping the difference in grayscale intensity between the two voltage states , see figure 8I-L. The analysis shows homogeneous electrochromism in the compartments in direct stomatal contact with the external PEDOT:PSS-NFC electrodes. This is to be expected, as stomatal contact provides both ionic and electronic pathways to the external electrodes, allowing continuous electronic charging/discharging of the PEDOT and subsequent ionic compensation. However, for the intermediate compartments not in direct stomatal contact with the external electrodes, we observed electrochromic gradients with the dark-colored side (PEDOT⁰) pointing towards the positively biased electrode. This behavior can be explained by a lack of electronic contact between these compartments - that is the infiltrated PEDOT:PSS-NFC did not cross between compartments. As such, these intermediate compartments operate as bipolar electrodes [1029], exhibiting so-called induced electrochromism [1030]. Indeed, the direction of the electrochromic gradients, reflecting the electric potential gradients inside the electrolyte of each compartment, exactly matches the expected pattern of induced electrochromism figure 8M.

In figure 8N, we propose a circuit diagram to describe the impedance characteristics and current pathways of the leaf-OECD, taking into account both the electronic and iontronic current pathways. The fact that both electrochromic and potential gradients are established in electronically isolated but ionically connected areal compartments along the leaf suggests that the ionic (*R*_{i,Cn}) and electronic (*R*_{e,Cn}) contact resistances across the stomata do not limit the charge transfer and transport. Even though electrochromic switching takes less than 20 s, a constant current (due to charge compensation and ion exchange) can be maintained for extended periods of time, suggesting that the capacitance for ion compensation within the electrodes (*C*_{Cn}) is very large, thus not limiting the current and transient behaviour either. We also found that the induced electrochromism vanished shortly after the two outer electrodes were grounded, suggesting that the electronic resistance (*R*_{e,n}) of the infused PEDOT:PSS-NFC is lower than that of the parallel ionic resistance (*R*_{n,ion}). Our conclusion is that the switch rate of directly and indirectly (induced) compartments of the leaf-OECD is limited by ionic - rather than electronic - transport.

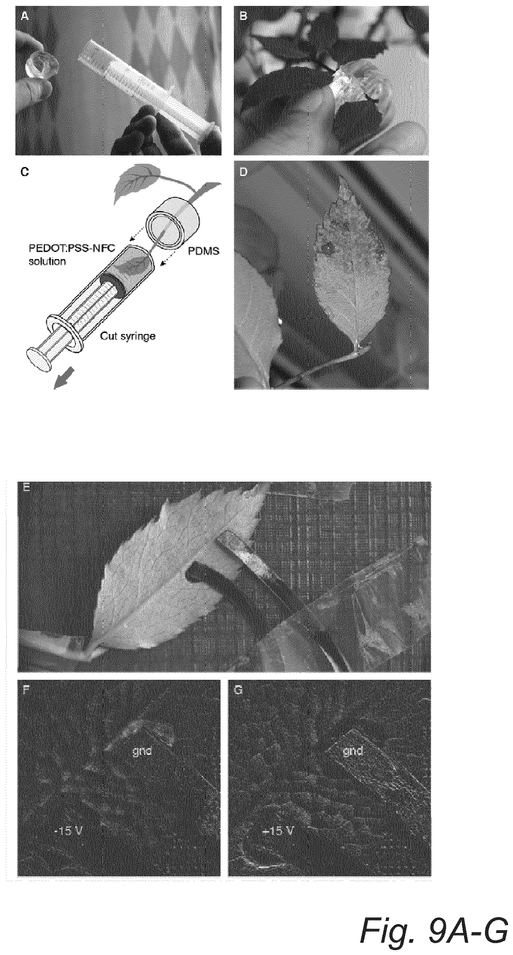

The fact that electrochromically-visualized potential gradients are established along leaf compartments indicates ion conduction across veins is efficient and does not limit the overall charge transport. Indeed, we demonstrate above that induced electrochromism and optical image analysis are powerful tools to investigate ion migration pathways within a leaf. However, many technological opportunities and tools require extended electronic conduction along the entire leaf. Our next target will therefore include development of conductive bridges that can transport electronic charges also across leaf veins.
All experiments on OECT and OECD circuits, in the xylem and in leaves, have been carried out on plant systems where the root or leaves have been detached from the plant. In another experiment, we investigated infusion of PEDOT:PSS-NFC into a single leaf still attached to a living rose, with maintained root, stem, branches and leaves. We found infusion of PEDOT:PSS-NFC successful and observed OECD switching similar to the isolated leaf experiments (Supplementary Material and figure 9). Figure 9A-G, with references to the alphabetical parts of figure in parenthesis, shows: PEDOT-loading and electrochromic leaves on living rose. (A) PDMS seal (left) and cut syringe used for on-plant vacuum infiltration of PEDOT:PSS-NFC. (B) PDMS seal mounted on attached rose leaf. (C) Vacuum infiltration schematic for live leaf. (D) Bottom side of PEDOT:PSS-NFC infused leaf. The dark regions indicate filled compartments. Compare with figure 8E. (E) Free-standing PEDOT:PSS-NFC "wires" mounted on abaxial side of leaf. (F) Color-enhanced live-leaf electrochromism (as in figure 8I and J) and (G) in reversed bias.

Integrated organic electronic analog and digital circuits manufactured in plants are reported. The vascular circuitry, components and signals of Rosa floribunda plants have been intermixed with those of in vivo manufactured PEDOT structures. Our findings pave the way for new technologies and tools based on the amalgamation of organic electronics and plants in general. For future electronic plant technologies, we identify integrated and distributed delivery and sensor devices as a particularly interesting e-Plant concept for feedback-regulated control of plant physiology, possibly serving as a complement to existing molecular genetic techniques used in plant science and agriculture. Distributed conducting wires and electrodes along the stem, root and in the leaves are preludes to electrochemical fuel cells, charge transport and storage systems that convert sugar produced from photosynthesis into electricity, in vivo.

### Supplementary Materials:

### Materials and Methods:

### PEDOT-S wires formation in rose xylem

We used stems directly cut from a young 'Pink Cloud' *Rosa floribunda*, with and without flowers, purchased from a local flower shop. The stems with flowers were kept in water and under refrigeration until they were used for the experiment. The stems were cleaned with tap water and then a fresh cut was made to the bottom of the stem with a sterilized scalpel under DI water. The stem was then immersed in 1 mg/ml PEDOT-S:H in DI water, and kept at approx. 40 % humidity and 23 °C. Experiments were also performed at 70 % humidity, but no significant difference on the absorption was observed. The rose was kept in the PEDOT-S solution for approx. 48 h. During the absorption, fresh 2-3 mm cuts to the bottom of the stem were done every 12 h. After absorption, the bark and phloem were peeled off to reveal the xylem. The dissected stem was kept in DI water under refrigeration fridge until used for characterization and device fabrication.

### Xylem-wire device fabrication and characterization

The piece of stem was mounted on a petri dish using UHU Patafix and was surrounded by DI water to prevent drying out during the experiment. For all the measurements, Au-plated tungsten plated probe tips (Signatone SE-TG) with a tip diameter of 10 um were used. Using micromanipulators and viewing under a stereo microscope (Nikon SM Z1500), the probe tips were brought into contact with the wire, and a very small pressure was applied in order for the tips to penetrate the xylem and make contact with the PEDOT-S inside.

### Xylem-wire conductivity measurement

Measurements were performed in the same wire for three different lengths starting from the longest and then placing one contact closer to the other. We used a Keithley 2602 SourceMeter controlled by a custom LabVIEW program. The voltage was swept from 0.5 - -0.5 V with a rate of 50 mV/s.

### Xylem-OECT construction

The channel, source, and drain of the OECT are defined by the PEDOT-S wire in the xylem. Contact with source and drain was made done using Au-plated tungsten probe tips. A Ag/AgCl pellet electrode or a PEDOT:PSS (Clevios PH1000 with 10% ethylene glycol and 1 % 3-(glycidyloxypropyl)trimethoxysilane) coated probe tip were used as the gate. In the Ag/AgCl case, the gate was in contact with the channel through an agarose gel electrolyte (10 % agarose in 0.01 M KCl(aq)). For the PEDOT:PSS-coated probe, the tip penetrated the tissue in the vicinity of the channel. All measurements were performed using a Keithley 2602B SourceMeter controlled by via a custom LabVIEW program.

### NOR gate construction

The NOR gate consisted of two xylem-OECTs and a resistor in series. The two transistors were based on the same PEDOT-S xylem wire and were defined by two gates (PEDOT:PSS coated Au-probe tips), placed in different positions near the PEDOT-S xylem. Using probes, the transistors (xylem wire) were connected with an external 800 kΩ resistor and a supply voltage (*V*_{DD} = -1.5 V) on one side, and grounded the other side. All measurements were performed using two Keithley 2600 series SourceMeters that were controlled using a custom LabVIEW program, and one Keithley 2400 SourceMeter controlled manually.

### Preparation of PEDOT:PSS-NFC material

The preparation of the PEDOT:PSS-NFC material follows a previously reported recipe (28). Briefly, PEDOT:PSS (Clevios PH 1000, Heraeus) was mixed with DMSO (Merck-Schuchardt OHG), glycerol (Sigma-Aldrich), and cellulose nanofiber (Innventia, aqueous solution at 0.59 wt%) in the following (aqueous) ratio: 0.54 : 0.030 : 0.0037 : 0.42, respectively. The mixture was homogenized (VWR VDI 12 Homogenizer) at a speed setting of 3 for 3 min and degassed for 20 min in a vacuum chamber. To make the dry film electrode, 20 mL of the solution was dried overnight at 50 °C in a plastic dish (5 cm in diameter), resulting in a thickness of 90 µm.

### Leaf infusion and contact.

A leaf was excised from a cut rose stem that was kept in the refrigerator (9 °C, 35% relative humidity). The leaf was washed with DI water and blotted dried. The leaf was placed in a syringe containing PEDOT:PSS-NFC and then plunged to remove air. Afterwards, the nozzle was sealed with a rubber cap. The plunger was gently pulled a difference of 10 mL thereby creating a vacuum in the syringe. The plunger was held for 10 s, and then slowly returned to its resting position for an additional 20 s. The process was repeated 10 times. After the 10th repetition, the leaf rested in the solution for 10 min. The leaf was removed, rinsed under running DI water, and gently blotted dry. Infusion was evident by darker green areas on the abaxial side of the leaf surface. As the leaf dried, the color remained dark indicating a successful infusion of the material. To make contact to the leaf, small drops (1 µL) of PEDOT:PSS-NFC solution were dispensed on the abaxial side of the leaf. PEDOT:PSS-NFC film electrodes were placed on top of the drop air dried for approximately 1 h, while the leaf remained wrapped in moist cloth.

### Electrochromic measurements

Metal electrodes were placed on top of the PEDOT:PSS-NFC film and optical images (Nikon SM Z1500) were taken every 2 s. A positive voltage potential was applied (Keithley 2400) and the current recorded by a LabVIEW program every 250 ms for 6 minutes. The time stamp was correlated with the optical images. The voltage potential was reversed and the process repeated. Electrochromic effects were observed between ±2 and ±15 V.

### Image analysis

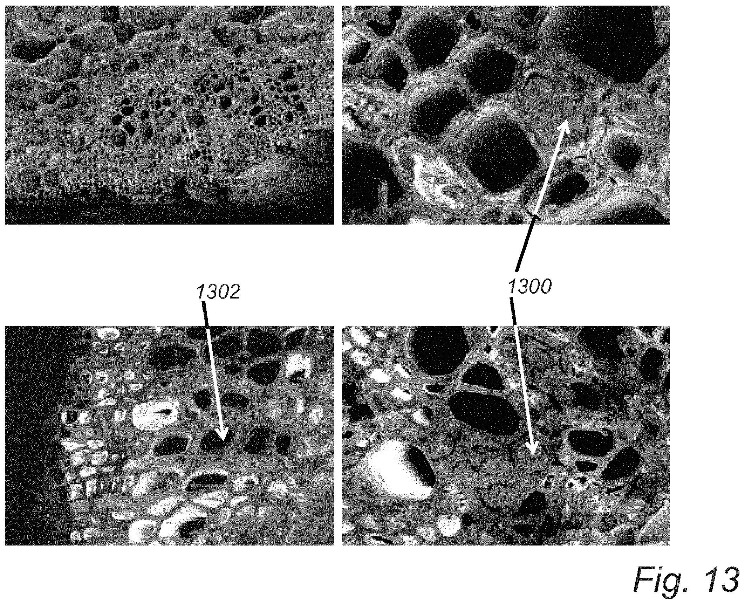

The optical images were converted to TIFF using the microscope software (NIS-Elements BR) and opened in Image J and used without further image processing. The gray scale pixel intensity (0-255) was recorded for pixels along a straight line (Fig. 13I and J) by taking the final image (*i.e*., the image after 6 min) for each state: *V*₁ = +15 V, *V*₂ = -15 V, and *V*₃ = +15 V. Each respective image for the 3 states were sampled 10 times and averaged together. Afterwards, those averaged gray scale values were subtracted from the averaged values of the previous state (*i.*e.*, V*₂ from *V*₁, and *V*₃ from *V*₂) representing the changes due to electrochromism plotted in Fig. 3K and L.

To observe estimated electric field path between the electrodes the final image of the second run (*V*₂) was subtracted from the final image of the first run (*V*₁) in ImageJ to create a false color image of the changes shown in Fig. 3M. Additionally, the final image of the third run (*V*₃) was subtracted from the second run (*V*₂). The false color images were increased in brightness and contrast, and noise reduction was applied to reveal the changes in oxidized and reduced states.

### Leaf infusion, in vivo

Infusion on a leaf attached to a rose bush was carried out by cutting the nozzle off of a syringe and filling the syringe part way with PEDOT:PSS-NFC, leaving a little room to pull vacuum. A PDMS mold was constructed to fit around the end of the cut syringe (see Fig. S1). The PDMS cap had a slit where the branch of the leaf could be inserted, while still able to retain vacuum. As much air was removed in the syringe as possible and the leaf in the PDMS cap was inserted into the PEDOT:PSS-NFC and sealed. Vacuum could be pulled as described above. The leaf and cap were removed and rinsed and electrochromism was observed taking images using a Canon 5D with a macro-lens filter, while the leaf was still attached to the plant.

### Chemical synthesis (overview)

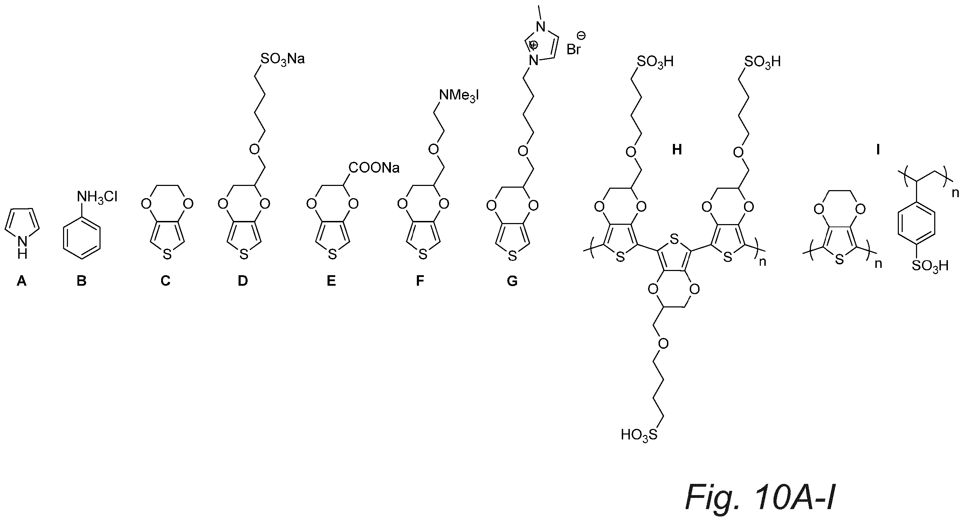

Figure 10A-I, with references to the alphabetical parts of figure 10 in parenthesis, are listed all the monomers (A-G) and polymers (H, I) used and investigated as suitable electrode materials inside plants. Molecular charge, size, oxidation potential (on monomers in xylems) and pH, were the variables explored. Monomers (A-G) were either chemically or electrochemically polymerized inside xylems, while (H) and (I) was allowed to be absorbed into xylems from their respective water dispersion. Attempts to chemically polymerize the monomers into water soluble electronically conducting polymers was successful for (D) (EDOT-S). The resulting polymer (H) (PEDOT-S) was synthesized according to previous publications [1001-1003].

Electrochemically and chemically polymerizations was performed first after the plants have been standing in a solution of the monomers for 6-12 hours. Electropolymerization was performed at varied onset potentials based on previous reports and literature of monomer oxidation potentials [1004]. After the stems had absorbed the monomers (with or without) additional electrolyte salts, the stems was split in halves, and potentio-static/dynamic polarization was applied to gold electrodes with varied points of contact with the xylem interiors.

Due to plants abilities to adjust salt gradients inside xylem and phloem through ionic exchange mechanisms with H⁺, Ca²⁺, Mg²⁺ SO4²⁻, NO₃⁻ being the ions mostly involved, we also investigated the effect of pH on chemically/electrochemically polymerizations and solution absorbance of polymers (H, I). In the case of EDOT-S and PEDOT-S (D, H), previous reported results have shown cross-linking (sulphonate interactions with divalent metal ions), conformational changes (aggregation/clustering) and conductivity being some properties influenced by changes in pH or salt concentrations [1006].

Compound (F) and (G) have to our knowledge never been published and was synthesized with the aim to investigate the effects from ionic charge, and type of charge (e.g. aromatic) on EDOT/PEDOT derivatives and there assembly in xylems. Structure (F) and (G) could not be chemically polymerized with the synthetic protocols used, probably due to charge repulsion/stabilization effects between intermediate radical structures and positive functionalities.

### General synthesis methods

Compound (D), (F), (G) and (H) were synthesized in-house. Compound (I) (PEDOT:PSS (PH1000)) was purchased from HC Starck and used as received. Compound (A-C), (E), and all other reagents were purchased from Sigma-Aldrich and used as received. Proton nuclear magnetic resonance ¹H were recorded on Varian (300 MHz) spectrometer. The deuterated solvent was used as internal standard, for CDCl₃ (¹H, δ = 7.26) and DMSO-*d*₆ (¹H, δ = 2.50) were used as references [1005].

### In Situ Chemical Polymerization

First, the monomer, given by the entry in table SX below, was dissolved in deionized water in concentrations of 1-5 mg/ml and allowed to be absorbed by the xylems for 6-12h. After that, the stems where put to a freshly made solution containing diluted Fe(NO₃)₃, FeCl₃ or FeCl₂/Na₂S₂O_{8,} FeCl₃/Na₂S₂O₈. Oxidant concentrations varied from 1 µM to 1 mM. Solution pH was changed from neutral to acidic/alkaline with addition of HCl or NaOH respectively.

### In Situ Electrochemical Polymerization

The monomers was dissolved in deionized water in concentrations of 1-5 mg/ml and allowed to be absorbed by the xylems for a period of 6-12 h. Then the stems were cut in halves and contacted with gold-electrodes at different positions along the xylem Potentiostatic/dynamic controlled polarization of the electrodes was used, with onset potentials based on reported oxidation potentials of the monomers [1005].

### Solution Absorption

Successfully chemically polymerized PEDOT-derivatives were dissolved in deionized water at concentrations of 1-5 mg/ml and subsequently used for direct absorption into xylems for different periods in time, usually 6-12 h.

### Synthesis of Compound D and H

EDOT-S and PEDOT-S was synthesized according to previous work. [1001, 1002].

### Synthesis of Compound F

To a stirred solution of Hydroxymethyl EDOT (1g, 5.81 mmol) in dry DMF (50 ml, 0°C) was added NaH (1.63g, 40.7 mmol) in small portions over 15 min. 2-chloro-N,N dimethylamine hydrochloride (2.51g, 17.4 mmol) was then added in portions over five minutes. The resulting mixture was allowed to reach RT and stand for 3h. The reaction was quenched with distilled water (50 ml) and extracted with EtOAc (3×50 ml). The separated organic layer was then extracted with BRINE (50 ml), distilled water (50 ml) and dried with MgSO₄. Evaporation of EtOAc resulted in an orange oil that was purified with FC (EtOAc 1% trimethylamine) to give 1.05g (74%) as yellow oil. ¹HNMR (CD₃OD) δ: 2.47(s, 6H), 2.75(s, 2H), 3.43(t, 2H), 3.72(m, 2H), 4.05(dd, 2H), 4.28(dd, 1H), 4.3(dd(b), 2H), 6.4(s, 2H).

To the resulting oil above, CH3I (neat, 4 ml) was added to an airtight vessel at 0°C and allowed to stand 12h at 60°C. The resulting yellow salt was repeatedly washed with diethylether and filtrated. After drying, compound F was obtained quantatively ¹HNMR (DMSO-d6) δ: 1.37(t, 2H), 1.39(t, 2H), 3.43(t, 2H), 3.52(t, 2H), 3.6(dd, 2H), 4.08(dt, 1H), 4.3(dd(b), 2H), 6.4(s, 2H).

### Synthesis of Compound G

To a stirred solution of Hydroxymethyl EDOT (1g, 5.81 mmol) in dry DMF (50 ml, 0°C) was added NaH (240 mg, 6 mmol) in small portions over 15 min. 1,4 dibromobutane (2.1 ml, 17.4 mmol) was then added in one portion and the resulting mixture was allowed to reach RT and stand for 12h. The reaction was diluted with EtOAc (50 ml), and the organic layer was then extracted with BRINE (2×50 ml), distilled water (2×50 ml) and dried with MgSO₄. Evaporation of EtOAc resulted in an orange oil that was purified with FC (EtOAc/Toluene (9:1),) to give 1.23g (69%) as yellow oil. ¹HNMR (CD₃OD) δ: 2.03(t, 2H), 2.11(t, 2H), 2.4(m, 4H) 3.43(t, 2H), 3.49(t, 2H), 3.52(t, 2H), 3.6(dd, 2H), 4.08(dt, 1H), 4.15(t, 2H), 4.3(dd(b), 2H), 6.4(s, 2H).

Methyl Imidazole (neat, 4ml) was then added to the oil in a airtight vessel and allowed to stand 12h at 70 0°C under argon. The resulting yellow salt was repeatedly washed with diethylether and filtrated. After drying, compound F was obtained in 96% (1.5 g)) ¹HNMR (DMSO-d₆)) δ: 1.7(d, 2H), 2.11(d, 2H), 3.61(s, 1H), 3.73(s, 4H), 4.12(s, 2H), 4.25-4.44(m, 4H), 6.35(s, 2H), 6.91(s, 1H), 7.09(s, 1H), 7.51(s, 1H).

**Table SX**

| Entry | Monomer/Polymer concentration and pH | Xylem deposition method |
|---|---|---|
| A | 1-5 mg at pH 3 | *In Situ* chemical and electrochemical polymerization |
| B | 1-5 mg, pH 3, SDS as surfactant. | *In Situ* chemical/electrochemical polymerization |
| C | 1-5 mg, pH 7, SDS as surfactant. | *In Situ* chemical/electrochemical polymerization |
| D | 1-5 mg/ml, pH 7 | *In Situ* chemical/electrochemical polymerization |
| E | 1-5 mg/ml, pH 7 | *In Situ* chemical polymerization |
| F | 1-5 mg/ml, pH 7 | *In Situ* chemical polymerization |
| G | 1-5 mg/ml, pH 7 | *In Situ* chemical polymerization |
| H ^{a} | 0.1-2 mg/ml, pH (3-8). | Solution absorption |
| I | 0.1-2 mg/ml, pH (3-7) | Solution absorption |
| ^{a}PEDOT-S (Entry H) was synthesized with H₃O⁺, Na⁺, K⁺ and NH₄⁺ as counter ions for the negative sulphonates respectively. | | |

### Further experiments using the Compound of formula I

In the following section, experiments and results from experiments which used compound 1 outlined in the section "compound 1" are shown. Throughout the following figures depicting parts of a rose, or a young bean plant, after or during an experiment, areas of the rose or the bean plant where a polymerized compound 1 has formed and is present within the plant often appears as the darker or black areas in the photographs. Further, the magnification of each image may be shown by a graphical element representing a distance in the figures.

Turning to figure 11A-E, with references to the alphabetical parts of figure in parenthesis, figure 11A-E shows cross sectional cuts of a rose plant. The stem of the rose plant, i.e. 1-3 cm of the bottom of the stem, was immersed in an aqueous solution of compound 1 (1 mg/mL). The plant was left in the solution in order to draw in or uptake the solution, and the cross sectional cuts were made after less than twelve hours. Although the initial color of the compound 1 solution used is yellow, inside the plant the solution appears dark blue which suggests that the compound 1 polymerizes inside the plant. Note, the polymerization inside the plant is quicker than the polymerization of compound 1 outside the plant. Hence, the plant is speeding up the process of the polymerization.

The different cross sectional cuts shown are (A) the stem, (B) a petiole, (C) a stem of a leaf, (D) a petal vein, and (E) a leaf vein. Note that the compound 1 is present in all different parts (A-E) after approximately just six hours of immersing, or at least less than twelve hours of immersing, of the bottom of the stem in the compound 1 solution and is interconnected through the different parts (A-E). Hence, the solution polymerizes within the xylem of the rose and forms a continuous polymerized structure. In other words, in this example after approximately less than twelve hours the solution has spread and polymerized throughout the rose. The polymerized solution thus forms continuous wires, which are electrically conducting due to the polymerized compound 1, through at least several parts of the plant.

Figures 12 and 13 shows polymerized compound 1 formed in the xylem of the rose having at least two different structures; a condensed structure where the polymer fills the xylem tube 1200, 1300, and a thin film structure 1202, 1302 where the polymer coats the inner wall of the xylem. Note that this is similar to the color and properties as the previous experiment(s) showed using PEDOT. The inherent properties, such as enzymatic defense mechanism, of the plant itself may be the reason why in some cases the resulting polymer fills a transport channel or coats the inner wall of the transport channel by forming a thin-film or layer. Note that the different photographs in figure 13 has different degrees of magnification, the top left photo is magnified 500 times, the top right photo is magnified 5000 times, the bottom left photo is magnified 1500 times, the bottom right photo is magnified 2000 times.

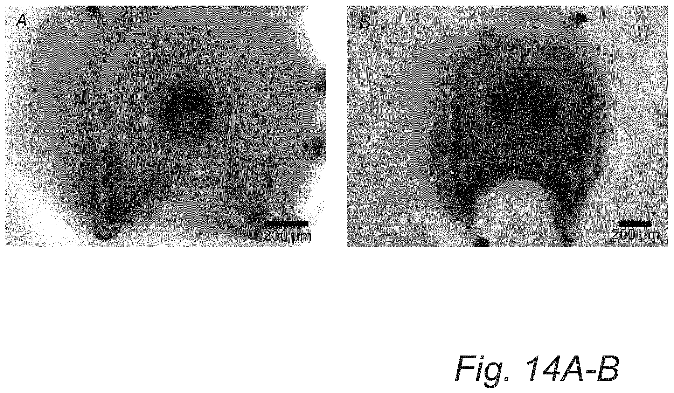

Figure 14A and 14B shows two cross sectional cuts made at two different parts of a petiole of the rose shown in figures 11-13. The petiole extends from the stem and the leaves of the plant are attached to the petiole. The cross sectional cut shown in figure 14A has been made at the first leaf of petiole, and the cut shown in figure 14B is made at the top leaf of the petiole which is at least 9 cm from the stem. Hence, the compound 1 also polymerizes into the veins of the leaves, reaching the top leaf of the petiole that is 9cm from the main stem of the rose, i.e. forming an at least 9 cm long continuous electronic conductor. The conductor formed by polymerized compound 1 is the darker area in the center of the petiole cuts shown in figure 14A and 14B.

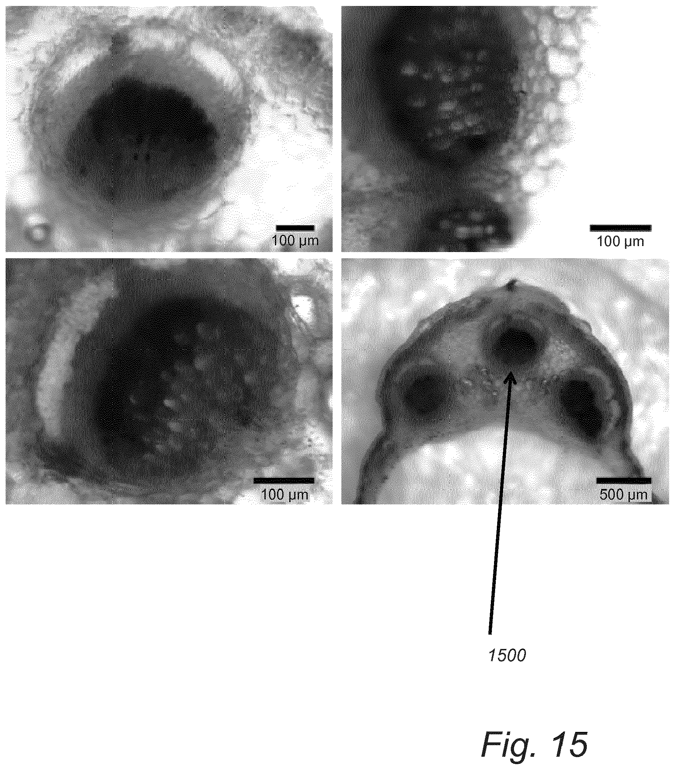

Figure 15 are other cuts made along the petiole of the same plant as shown in figure 11 to 14. Note that the polymer formed by the compound 1 in the xylems of the plant, i.e. the darker areas shown. That the polymerized compound 1 is present in the different parts, for example in the xylems 1500 close to the stem means that the polymerized compound 1 extends from the petiole into at least the stem of the plant as well.

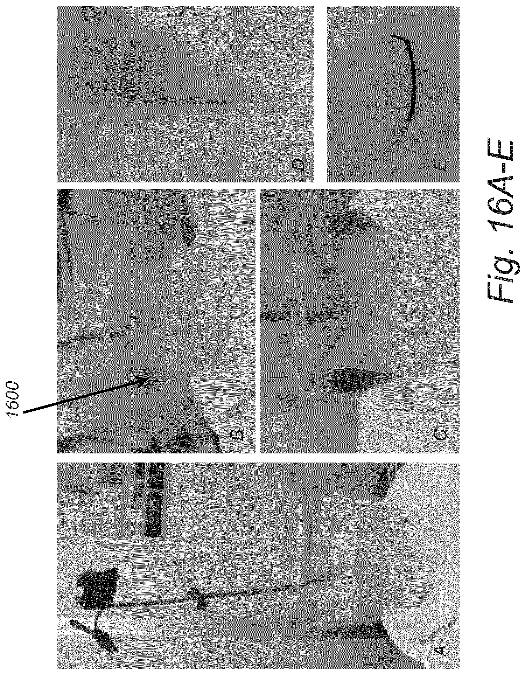

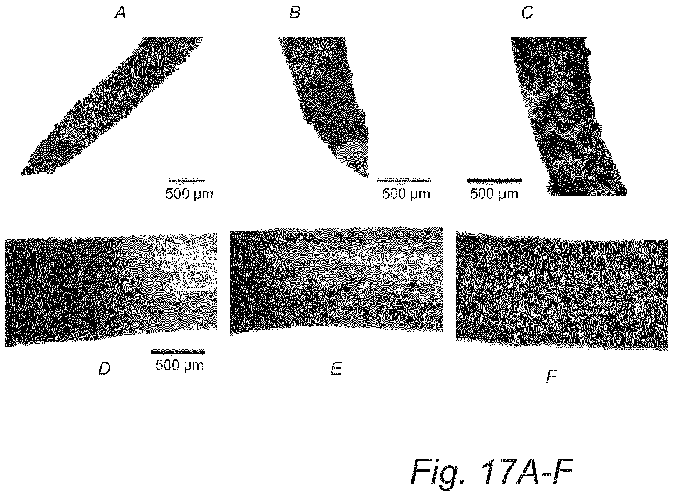

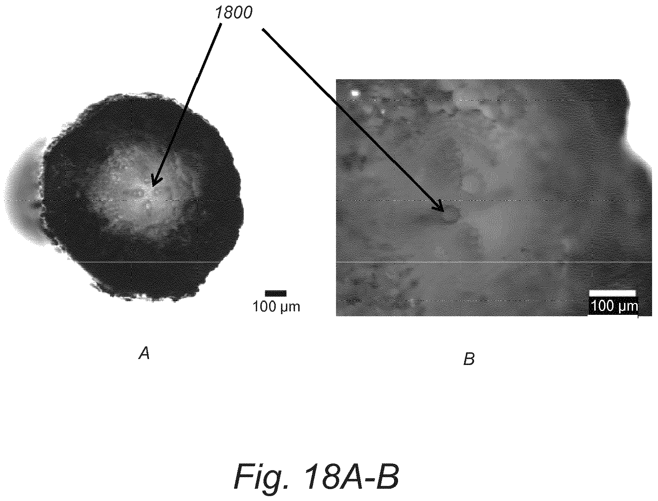

Figure 16A-E illustrates another experiment, with references to the alphabetical parts of figure in parenthesis. A young bean plant having a plurality of roots was used as a test specimen. In (A) the roots of the young bean plant were planted in a plastic container having Seedhru® gel as a soil substitute.. Two of the roots of the bean plant were immersed in a vial 1600 (B) containing aqueous solution of compound 1 (1 mg/mL). Figure 16B and 16C shows a close up of the plastic container, where the two vials with compound 1 solution are seen as darker claw-like shapes. As soon as or shortly after the two roots were immersed the color of the compound 1 solution shifted from white to pink (D) and then back to white again. The reason of the color shift is still being investigated. After 24 hours the compound 1 solution has turned dark blue indicating that a polymer has been formed from the compound 1. After 24 hours one of the roots was removed from the compound 1 solution and can be seen in (E) and in figure 17. The other root was left immersed in the compound 1 solution for 24 more hours (total 48hours). After 48 hours the second root was cut of the bean plant and can be seen in figure 18.. Note that the part of the root which was immersed in compound 1 is now coated with polymerized compound 1 as indicated by the darker color. It is clear from the start of the experiment that the polymerization starts in the plant prior to within the vial, at the start of the experiment the color on the stem is dark, which means that compound I has polymerized on the stem while the color of the compound I solution in the vial is still yellow which means that no significant polymerization has occurred yet.

Figure 17A-F shows a magnified view of a bean root which has been coated with polymerized compound 1 from the experiment in figure 16, and shown in figure 16E, corresponding to an 24 hours immersion in the compound 1 solution. In figure 17A-C shows different longitudinal cross sectional cuts of the bean root. Note that the polymerized compound 1 is not only present on the external surface, but has also penetrated into the bean root. Figures 17D-F shows a gradient of polymerized compound 1 being present along the longitudinal extension of the root. Figure 17D is a view close to the part of the root which was immersed in the compound 1 solution, figure 17E is an area further up, and figure 17F is another area yet further up along the root. Note that the darker area, with the polymerized compound 1, recedes as the figure moves further up the root, and in figure 17F there is no observable polymerized compound 1 present.

Figure 18A-B shows a cross sectional cut made along the root discussed in figure 16, i.e. after 24 hours of immersion, at two different magnifications. In figure 18A the polymerized compound 1 covers the external surface and reaches xylem(s) in the root. Also note that the center 1800 of the bean root is clear and may still provide sufficient biological function for the plant to survive. Hence, electrical contact may be made from the outside, the external surface of the root to the xylem(s) of the root.

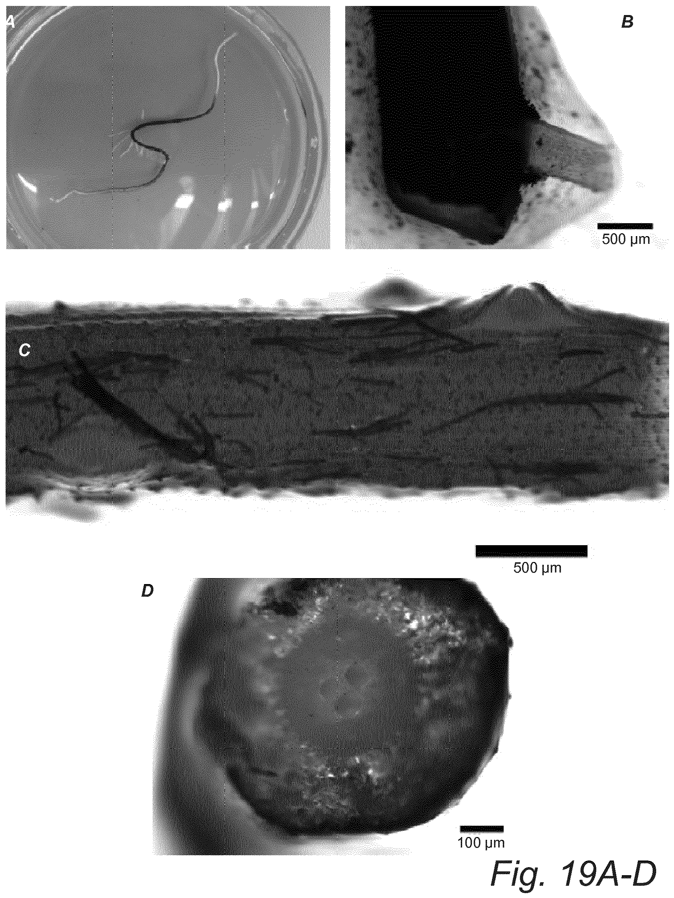

Figures 19A-D shows different views of a root which was immersed in compound 1 in accordance with the experiment discussed in figure 16. As seen in figure 19A, the root has polymerized compound 1 on the external surface thereof, but is still growing in length and is forming root hairs. Figure 19B is a magnification of a root hair which extends from the polymerized external surface. Figure 19C is a longitudinal cross sectional cut of the root shown in figure 19A, the elongated darker shapes are large polymer shapes, or accumulations of polymers. Figure 19D shows a cross sectional cut of the root. However, there seems to be no polymer within the xylem(s) of this root only on the surface. In any case, it is clear from figures 19A-D that the polymerized compound 1 does not significantly inhibit the growth or survival of the plant.

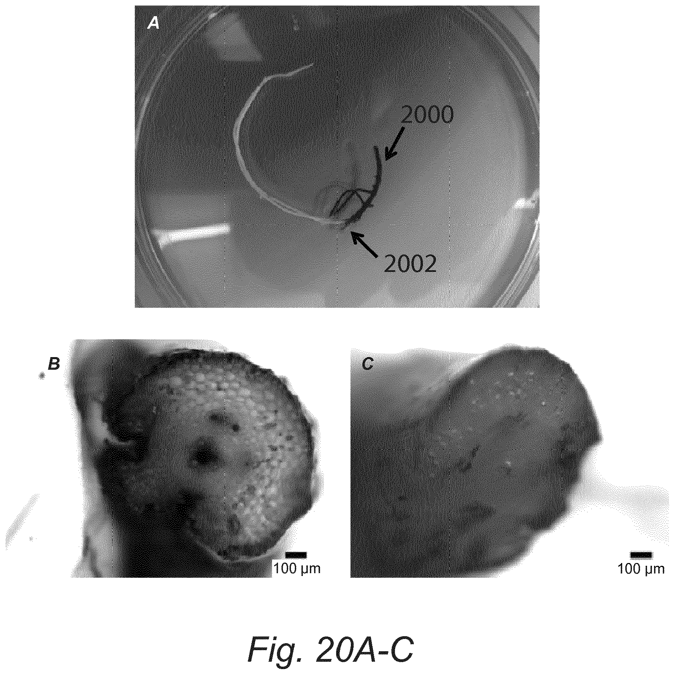

Figures 20A-C shows different views of a root which cut towards the end of the root. The root, still being attached to the bean plant, was then immersed in a compound 1 solution similar to the experiment discussed in figure 16. As seen in figure 20A, the end of the root has polymerized compound 1 on the external surface thereof. The arrow 2002 indicated where the cross sectional cut is made to provide figure 20C, and arrow 2000 indicated where the cross sectional cut is made to provide figure 20B. Figure 20A and 20B are magnifications of the respective cross section cut made at 2002 and 2000 in figure 20A. Note that in figure 20B compound 1 has polymerized on the external surface and on in the transport channel(s) of the root. In figure 20C compound 1 polymerization has not continued and reached further up into the plant.

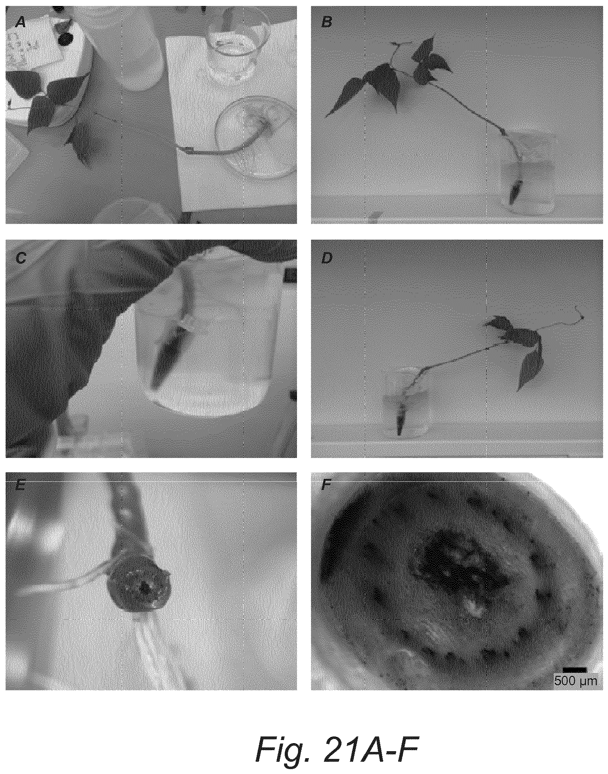

Figure 21A-F shows step by step how electronic wires are created in a bean plant in another experiment with references to the alphabetical parts of figure in parenthesis. The young bean plant including roots is shown in (A). A majority of the roots of the plant are cut off, leaving the stem clean, with some side roots left untouched. The side roots and the bottom part of the stem are immersed in the compound 1 solution (B) similar to the ones used in previous experiments. The compound 1 solution turns pink instantly upon immersion (C). After 24 hours the compound 1 solution has turned dark blue indicating that a polymer has been formed from the compound 1 (D). (E) shows a cross sectional cut of the stem, and that the xylem(s) of the stem is provided with polymer (E). A close up of the stem is shown in (F). Further, the young bean plant was replanted in Seedthru® gel without any other interaction and survives for at least two weeks. Hence, even if at least a portion of the xylem(s) of stem is/are provided, filled or coated with polymer, biological function of the plant is not hindered as the plant survives.

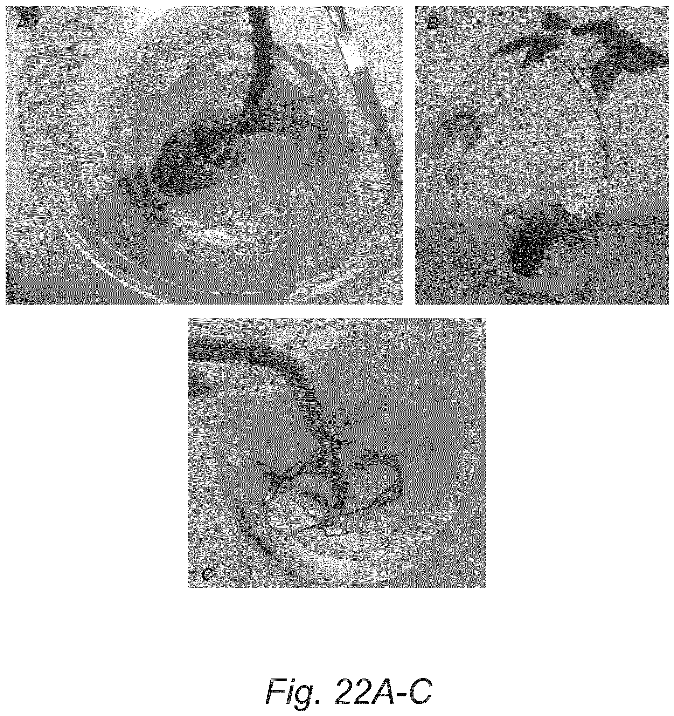

Figure 22A-C shows the same experimental setup as in figure 21A-F but here are half of the roots immersed in the compound 1 solution having a concentration of 1mg/ml and dissolved in water. The solution turns pink, and then back to the original yellow color. After approximately four days, the plant was removed, as seen in figure 22C, the roots which were immersed in the compound 1 solution are then coated with a polymerized compound 1. The plant was replanted in Seedthru® gel after absorbing the compound 1 and showed further growth. This means that not only do the plant survive, but may also continue to grow after wires have been formed in the transports channel(s).

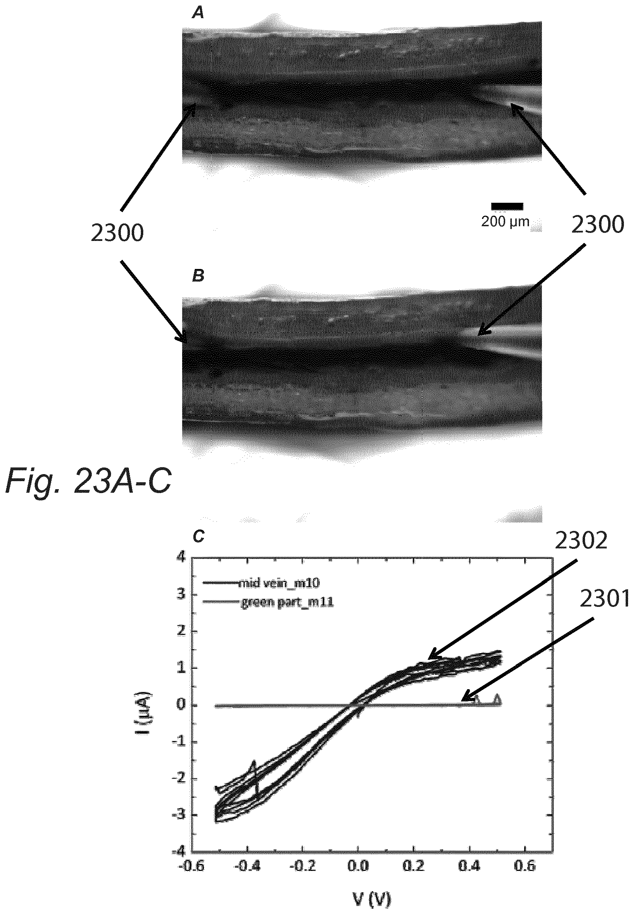

Figure 23A-B shows an experiment for measuring the conductivity along a vein in a leaf. Figure 23A and 23B is a longitudinal cross sectional cut along a vein in a petiole from an experiment similar to what is described in conjunction with figure 11. Two gold plated microelectrodes 2300 are used to contact the surfaces of the vein in figure 23A, and the outside between the vein and the outer surface of the petiole in figure 23B. Stated differently, figure 23A shows a measurement in the middle of the vein, and figure 23B shows a measurement outside the vein. Only the electrodes 2300 in figure 23A is in contact with the polymerized compound 1. Figure 23C is a plot of the current I (µA) versus the potential (V). The curve 2302 represents the measurement in the vein; hence, the vein coated with a polymerized compound 1 is conducting as an increasing potential provides an increasing current. The outside of the vein however, represented by the curve 2301, is not conducting as no current is measured for a varying potential.

Further, note that the measurement performed in figure 23A may be construed as measuring the conductivity along the longitudinal direction of the transport channel of the organic part. This means that the electrodes 2300 are distanced along the longitudinal direction of the vein, i.e. transport channel.

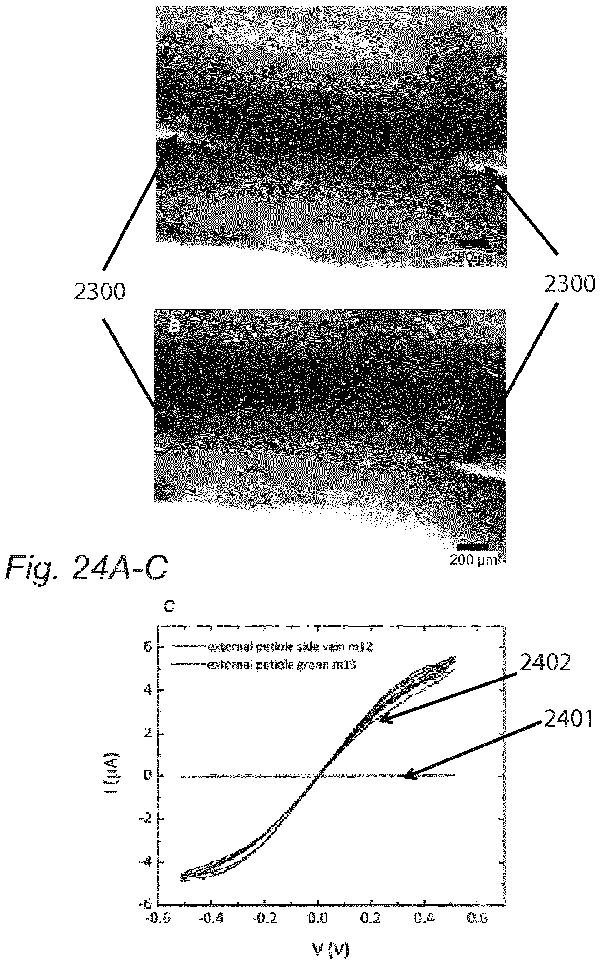

Figure 24A-B shows an experiment for measuring the conductivity along a vein in the petiole. Figure 23A and 23B is a longitudinal cross sectional cut along a petiole. Two gold plated microelectrodes 2300 are used to contact and penetrate petiole and order to reach the vein (or transport channel) of the petiole in in figure 24A, and the outside between the transport channel and the outer surface of the petiole in figure 23B. Stated differently, figure 23A shows a measurement in the middle of a petiole, and figure 23B shows a measurement outside the transport channel. Figure 24C is a plot of the current I (µA) versus the potential (V). The curve 2402 represents the measurement in the transport channel; hence, the transport channel coated with a polymerized compound 1 is conducting as an increasing potential provides an increasing current. The outside of the transport channel however, represented by the curve 2401, is not conducting as no current is measured for a varying potential.

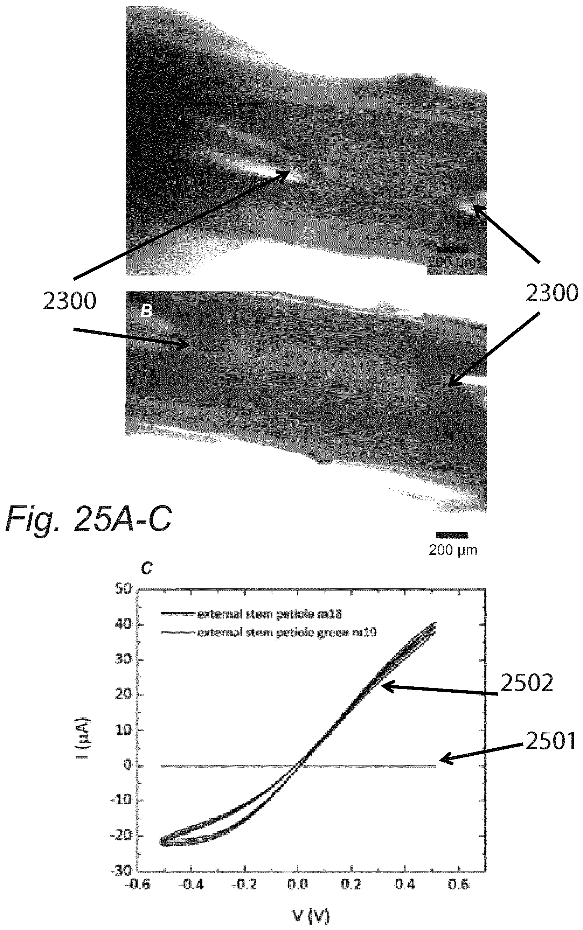

Figure 25A-B shows an experiment for measuring the conductivity along the stem of the petiole. Figure 25A and 25B are external views of a petiole. Two gold plated microelectrodes 2300 are used to contact and penetrate the stem of the petiole in figure 25A in order to reach a transport channel, and in figure 25B the two microelectrodes 2300 are used to contact the area outside the transport channel of the petiole. Figure 25C is a plot of the current I (µA) versus the potential (V). The curve 2502 represents the measurement in the transport channel by penetrating the external surface of the petiole. The area outside the transport channel is represented by the curve 2501. Hence, it is possible to reach the transport channel through the external surface of the petiole simply by penetrating the surface with microelectrodes, and the transport channel is conducting. Note the outside of the transport channel is not conducting as no current is measured for a varying potential.

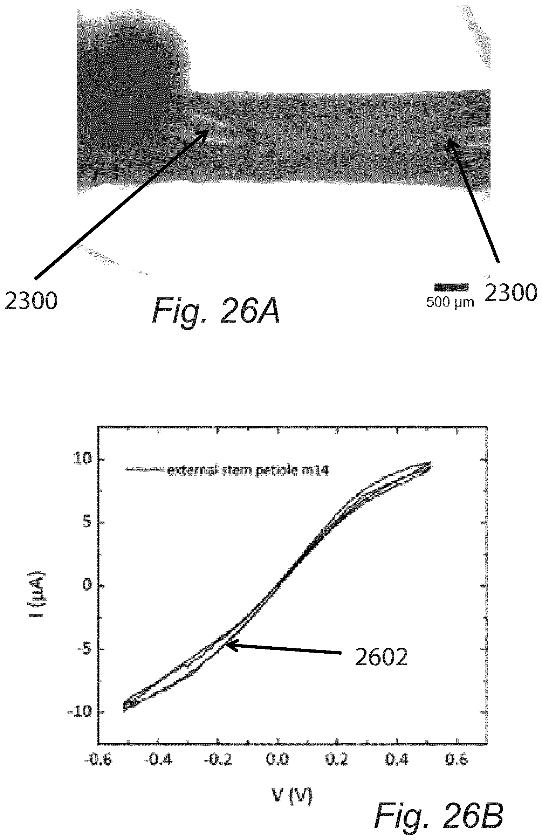

Figure 26A shows an experiment for measuring the conductivity along a stem of a petiole from the outside. Figure 26A is an external view of a petiole. Two gold plated microelectrodes 2300 are used to contact and penetrate a petiole surface in figure 26A in order to reach a transport channel. Figure 26B is a plot of the current I (µA) versus the potential (V). The curve 2602 represents the measurement in the transport channel by penetrating the external surface of the petiole. Hence, it is possible to reach the transport channel through the external surface of the petiole simply by penetrating the surface with microelectrodes, and the transport channel is conducting. Note the scale of the experiment in figure 26A, the conductivity is thus measured over several millimeters in length.

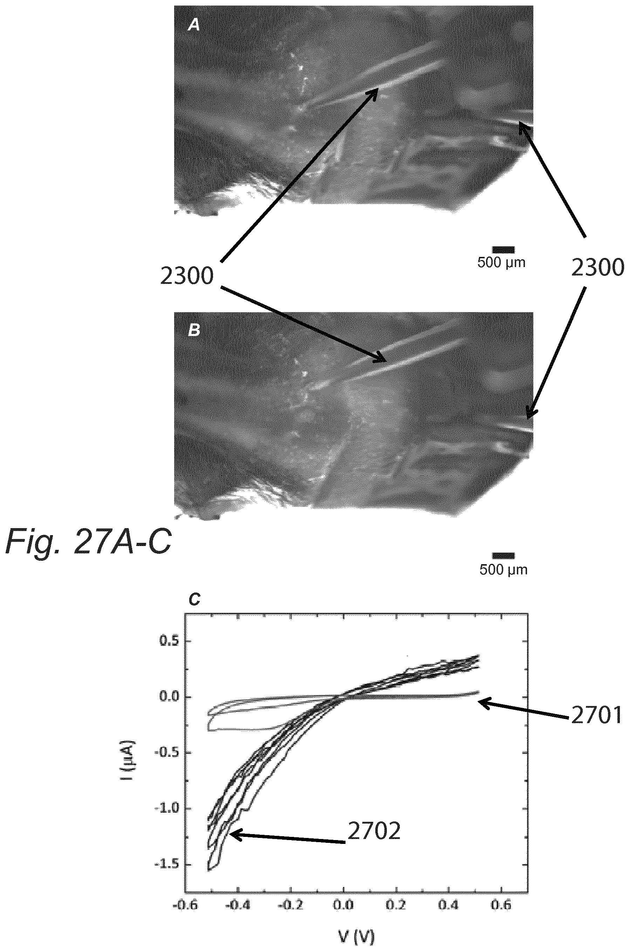

Figure 27A-B shows an experiment for measuring the conductivity between a petiole and a stem of a plant from the outside. Figure 27A and 27B are external views of a petiole and stem junction. Two gold plated microelectrodes 2300 are used to contact and penetrate a petiole surface and a stem surface in figure 27A in order to reach a transport channel, and in figure 27B the two microelectrodes 2300 are used to contact and penetrate the petiole surface and the stem surface in order to reach an area outside the transport channel. Figure 27C is a plot of the current I (µA) versus the potential (V). The curve 2702 represents the measurement in the transport channel, between the petiole and the stem, The area outside the transport channel is represented by the curve 2701. Hence, contact may be established also through, or between, a stem-petiole junction and the polymer also coats the inside of the transport channels therein. The larger current for the curve 2702 shows us that we have conducting wires from the stem to the petiole. The conducting wires enable electronic transport from stem to petiole.

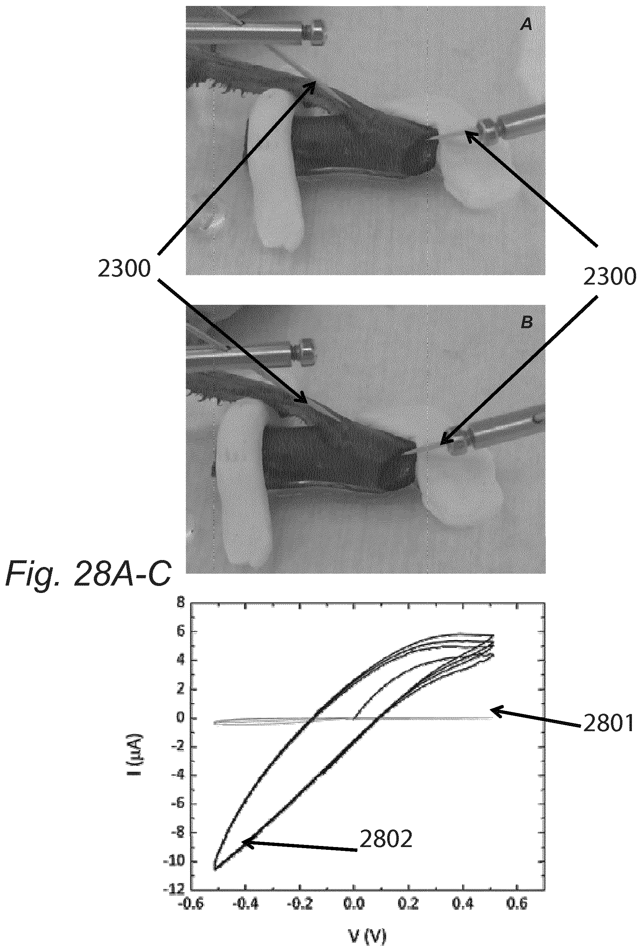

Figure 28A-B shows an experiment for measuring the conductivity from the bottom of a stem that has been immersed in a compound 1 solution and a petiole from the outside. Figure 28A and 28B are external views of a petiole, stem and a petiole-stem junction. Two gold plated microelectrodes 2300 are used to contact and penetrate a petiole surface in order to reach a transport channel in figure 28A and also to contact the bottom of the stem where the transport channel(s) is located, and in figure 28B the two microelectrodes 2300 are used to contact and penetrate the petiole surface in order to reach an area outside the transport channel and the bottom of the stem space apart from where the transport channel(s) is located. Figure 28C is a plot of the current I (µA) versus the potential (V). The curve 2802 represents the measurement in the transport channel, between the petiole and the stem, the area outside the transport channel is represented by the curve 2801. Hence, contact may be established also through, or between, a stem-petiole junction and the polymer also coats the inside of the transport channels therein. The larger current for the curve 2802 shows us that we have conducting wires from the stem to the petiole. The conducting wires enable electronic transport from stem to petiole.

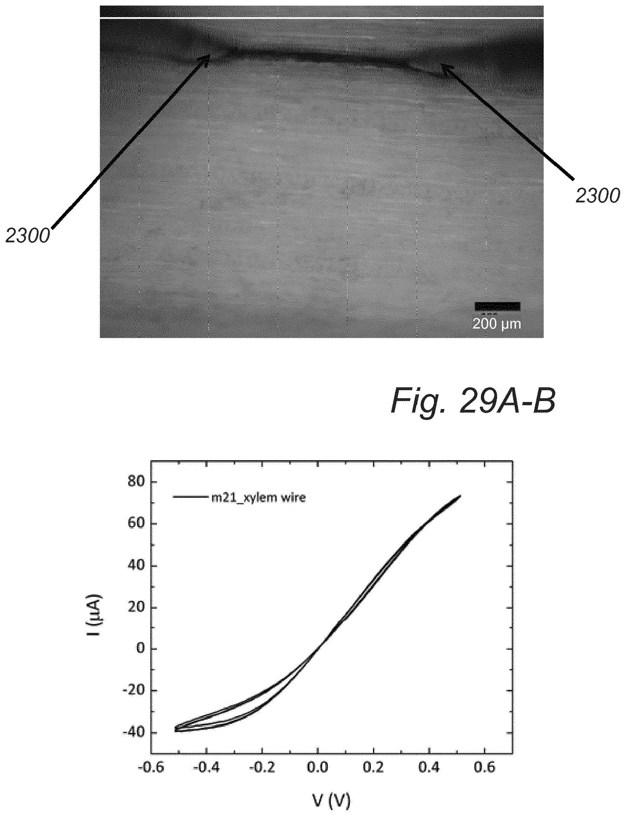

Figure 29A shows an experiment which measures the conductivity of a electrically conducting wire formed in a xylem channel of a rose plant. Figure 29A is an external views of a plant part, in this case the steam where the epidermis has been peeled off, also showing two gold plated microelectrodes 2300 used to contact and penetrate a surface of the stem in order to reach the xylem channel. Figure 29B is a plot of the current I (µA) versus the potential (V). This is the currently highest measured electronic conductivity in a plant. The resulting conductivity is approximately 1.5 S/cm, i.e. σ=1.5S/cm.

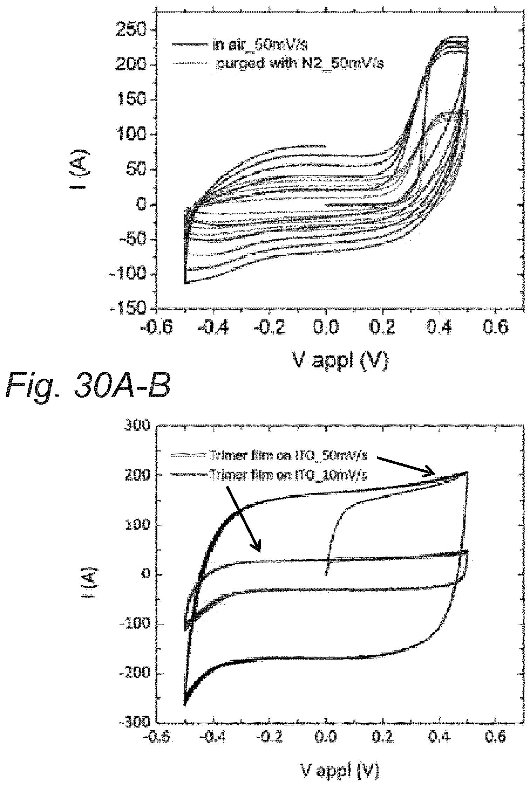

Figure 30A-B shows the on-set potential of compound 1. In order to define the on-set potential of the polymerization of the trimer a three electrode setup was used. The working electrode is an ITO coated glass, the counter electrode is a Platinum sheet and the reference electrode is an Ag/AgCl saturated electrode. The electrolyte was 1mg/ml of compound 1 in 0.01M NaCl. The active area of the ITO was 21mm x 8mm. A cyclic voltametry was performed in order to define the onset potential. The potential was applied between the working and the reference electrode while the current was measured between the working and the counter electrode. The potential was scanned from +0.5V to -0.5V, starting from 0V with a scan rate of 50mV/s. During the first scan current increases at around +0.3V defining the onset potential of the polymerization were observed. The polymer is deposited on the active area of the ITO coated glass.

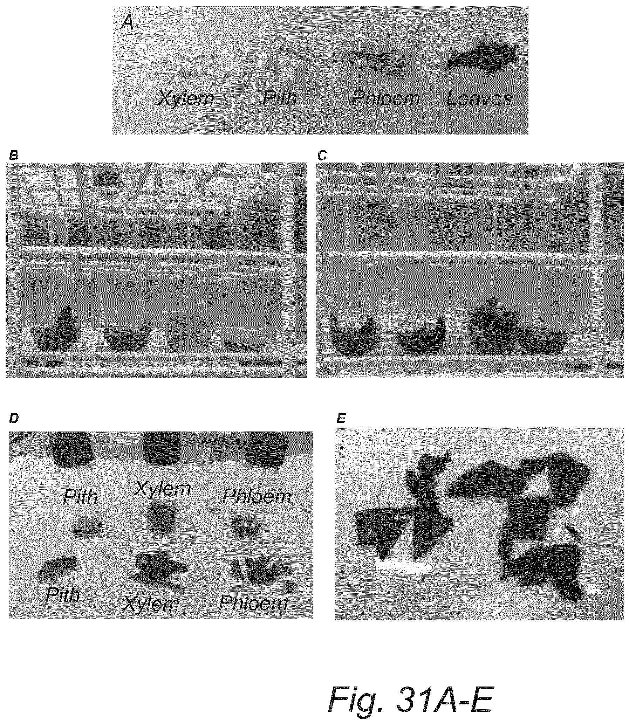

Figure 31A-F shows the reaction of compound 1 with different parts of the plant (xylem, pith, phloem and leaves). An aqueous solution of compound 1 was prepared (1 mg/mL). The plant tissues used were xylem, pith, phloem and leaves, shown from left to right in figure 31A. 2 mL of the prepared solution was added to the xylem whereas 0.5 mL was added to the pith, the phloem and the leaves (Fig B. leaves, phloem, xylem, pith, shown from left to right). Figure C show the tissues after 2 hours (leaves, phloem, xylem, pith, shown from left to right). Figures D show the pith, xylem and phloem after 5 hours, whereas figure E shows the leaves after 48 hours. Note that the polymerization of compound 1 is sped up by the plant tissue. The polymerization is faster on the plant tissue than in the solution.

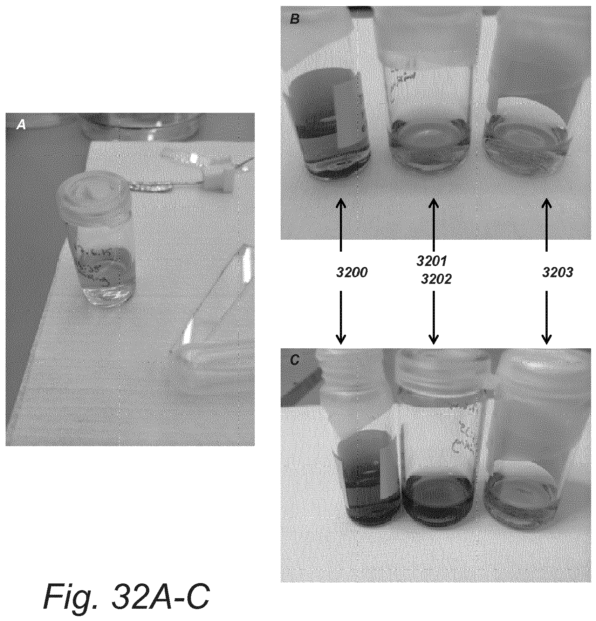

Figure 32A-C shows compound 1's solution oxygen sensitivity. Fig 32A shows after mixing compound 1 powder with de-ionized water. Vial 3200 was left in room temperature under an air atmosphere. Vial 3201 was sealed and left in fridge (4°C). Vial 3202: was sealed and left in fridge (4°C), but with leaves immersed in the mixture for half an hour. Vial 3203 was purged with N2, and then sealed in fridge. Figure 32B shows the effect after 18 hours, and figure 32C shows the effect after 24 hours.

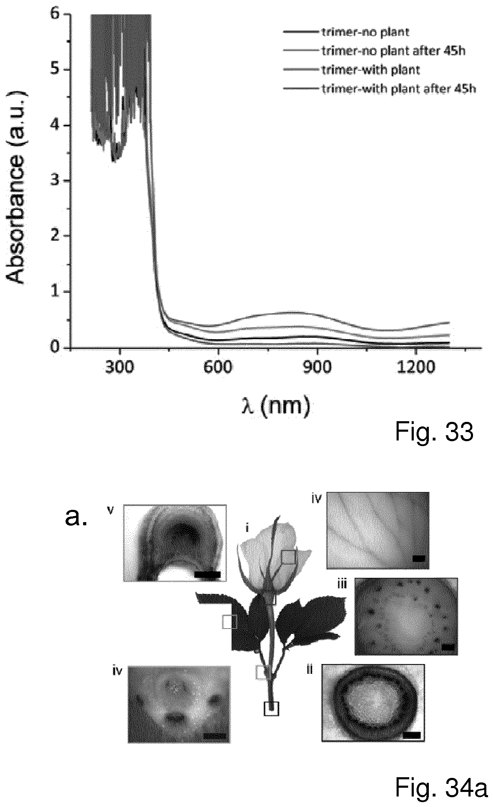

Figure 33 shows a comparison of polymerization with and without the effect the plant has on compound 1. Two solutions of compound 1 in water were prepared, one of which had a plant part immersed in it. UV-Vis spectra were recorded for the two solutions at t = 0 and after 45 hours. The resulting UV-Vis spectra is shown in figure 33. The spectra shows that more polymerization has occurred in the solution in which the plant was immersed, since the "trimer-with-plant" curve has the highest absorbance at 850 nm and 1200nm which also indicates that a polaron is formed and polymerization has occurred or is occurring. In other words, plant material speeds up the polymerization process compared to i.e. compound 1 without the plant, which is shown "trimer-no-plant" and has the second lowest absorbance at the relevant wavelengths.

### Detailed Example

### In vivo polymerization of electrodes in Plants

Plants are indispensible for our lives as they consist our primary source of food, release oxygen, regulate the water cycle and the climate, and provide us with a wealth of materials. A technology that regulates and controls plant processes with high spatiotemporal resolution can transform basic research and agriculture industry. In addition a technology that directly harvests energy from plants will allow us to exploit more efficiently the already absorbed solar energy. Nowadays the most sophisticated methods for altering and optimizing plant functions in plant science and biotechnology are based on genetic engineering. However, deploying these techniques is limited by governing and societal conditions. As an alternative to genetic modification smart materials such as carbon nanotubes and metal nanoparticles have been distributed in plants to enhance and regulate their functions. And most recently, we have reported a new approach where we augment electronic functionality to plants.

The E-plant technology is based on functionalizing the plant with organic electronic materials and manufacturing electronic devices in vivo. Organic electronic materials can conduct both electronic and ionic carriers making them ideal active components in bioelectronic devices for translating addressing electronic signals to complex ionic outputs and vice versa. However, organic bioelectronics have been mainly oriented towards biomedical applications e.g. neural prosthesis, in vitro diagnostics, therapy and tissue engineering and thus have been interfaced with mammalian organisms. Such technology can find several applications in plants with the recently introduced concept of electronic plants being the first example. Integrated electronic circuits and devices in plants open new pathways for sensing and regulating physiology, plant optimization and increase of productivity as well as energy harvesting.

Self-organization of materials in the internal structure of the plant is a key aspect of the E-plant technology. As a first example we have used PEDOT-S, a self doped p-type conducting polymer to fabricate conducting wires in the vascular tissue of the plant using the natural process of transpiration. Pedot-S chains self organize in the xylem, the water transport channels, and form hydrogel like conducting wires along the stem. Using the xylem-wires and the surrounding cellular and extracellular domains we constructed organic electrochemical transistors and more complex digital circuits. Our previous attempts based on other conducting polymers and in vivo chemical and electro- polymerization of monomers have failed either due to clogging and toxicity or have resulted to very localized polymerization. In order to gain versatility on device fabrication we need new materials that can be distributed in every part of the plant not only in the stem, like Pedot-S, without affecting the plant physiological processes. Connecting roots and stem with photosynthetic centers in the leaves and reproductive organs in flowers allow us to envisage a range of applications under the concept of electronic plants.

Molecular design of materials is the answer to many technological bottlenecks as materials are custom made meeting the demands of the specific application. Water-soluble conjugated oligomers that can self organize and polymerize in the internal structure of the plant upon distribution will be an ideal solution for e-plant technology. In this work we report the design and synthesis of a new water soluble oligomer, ETE-S (exo-EDOT-Thiophene-EDOT-alkoxylfonate) for E- plant technology. The ETE-S oligomer has low oxidation potential enabling air oxidation and consequent polymerization in the xylem of the plant. After studying NMR-spectra the inventors believes that the ETE-S characterizations in polar solutions, show strong proton exchange equilibriums, with many conformations present already at RT, but with more specific conformations upon different time dependent parameters, such as, concentration, pH, UV-VIS-IR exposure and/or oxidants present, and also that one of the EDOT-rings can be in syn-configuration as compared with the thiophene in the oligiomer. Without being bound to this theory, the inventors believe that this syn/trans relationship have an important impact on the parameters behind polymerization of the trimer, eg. oxidation potential, pH and/or optical transitions, and thereby enables the polymerization. Due to its small molecular size it can be distributed in every part of the vascular tissue of a rose cutting, from stem to flower and self polymerize forming long conducting wires. In addition the oligomer can overpass the veins and enter the apoplastic pathway in the leaves. Good electrical and charging capacity properties enable us to use the conducting xylem wires and the natural architecture of the plant to form super capacitors along the stem.

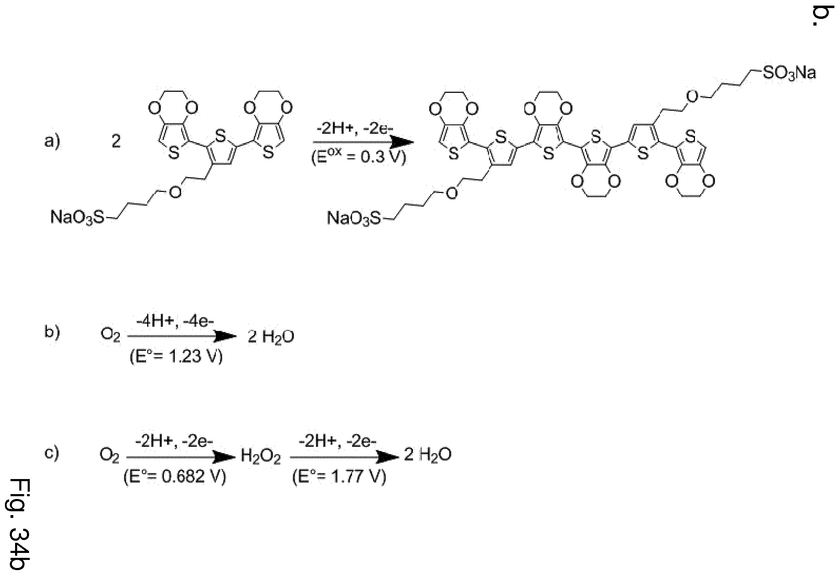

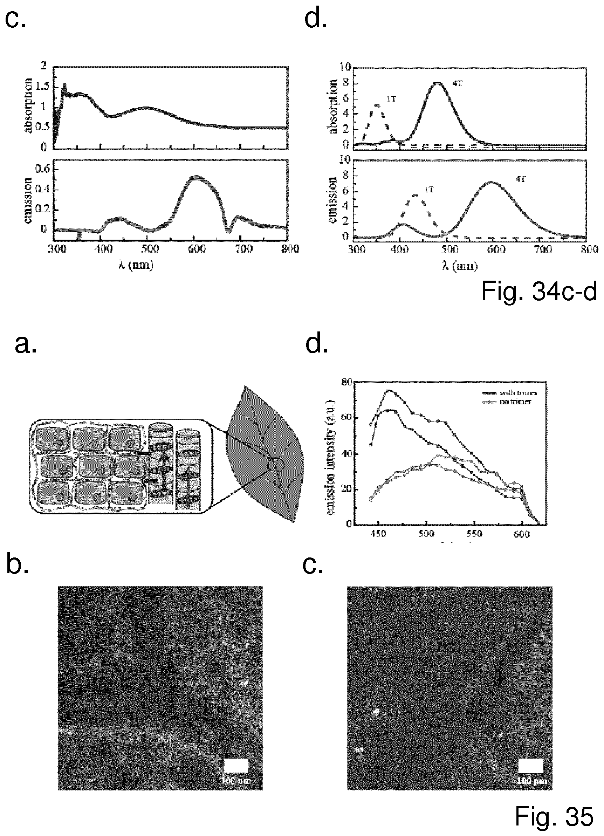

ETE-S was dissolved in DI water and a rose cutting with a fresh cross section was immersed in the solution for about 24hours. During this time the ETE-S solution was transported apically through the vascular tissue of xylem according to tranpiration. Two hours after the immersion of the rose in solution, the vascular bundles that were exposed appeared to be darker indicating initiation of the polymerization process. It must be noted that the ETE-S solution didn't polymerize at the same rate as the polymerization occurred in the vascular tissue of the plant. After 24 hours of immersion we removed the rose and rinsed it with water (Fig. 34.a.i). Peeling off the outer bark, cortex and phloem of the rose stem we exposed dark continuous lines along the whole length of the stem, length of more than 10cm. In Fig. 34.a.ii we show the cross section of the bottom part of the stem and the cross section of the top part of the stem just before the flower (Fig. 34.a.iii). The xylem tissue has a dark color suggesting that the ETE-S has been transported and polymerized from bottom to the top of the stem. From SEM and optical microscopy we see that the xylem in some cases is filled while in others is coated with the polymer (SI info). The ETE-S molecule is favorably deposited and polymerized in the inner wall of xylem, forming an initial layer. Depending on the flow and local concentration of the ETE-S we have different coverage of the inner volume of the xylem vessels.

The vascular system of a plant spans from the roots to the stem, to the leaves and flowers, existing in every part of it. In Figs. 34.a.iv-vi we show optical micrographs of the veins of the petal, the main vascular bundle of the leaf and the vascular bundles of the petiole. ETE-S, due to its small size, travels through the xylem in every part of the rose cutting and polymerizes in stem, petioles, leaves and petals. Even in the case where the leaf extended several cm from the stem (more than 10cm), ETE-S is effectively transported and polymerized in the vascular tissue of the leaves.

**Figure 34****:** ETE-S distribution and polymerization in rose cutting. a. **i.** Rose after immersion in ETE-S aqueous solution for 24hours, **ii-iii**. Bottom and top cross section of the stem with vascular bundles being dark due to deposition of the polymer, **iv.** White rose petal where veins appear dark due to the polymer, **v-vi**. leaf stem and petiole where vascular bundles are dark due to polymer. **b.** Synthetic route / polymerization of ETE-S, **c.** UV-Vis absorption and emission spectra upon excitation with 355nm laser of xylem filled with polymer extract in DMSO. D. Theoretical calculations of the absorption and emission spectra for ETE-S molecule and polymer (4 ETE-S units).

Fig. 34b shows one possible synthetic route/polymerization of ETE-S. However, the inventors have indications that there are also other routes. Photocatalysis involves the generation of charge carriers-that is, excess electrons (e⁻) and holes (h⁺), and the catalytic reactions induced by these species. When the trimer is excited with photons with energy higher than (or equal to) the bandgap, electrons are injected in the conduction band. Consequently, the electrons and holes migrate, when they escape recombination, to the semiconductor surface and generate highly oxidative radicals (in the presence of oxygen and water). Under irradiation, electrons are injected from the excited trimer and react with oxygen to form the oxidizing O2·⁻ superoxide radical (E⁰, O₂/O₂·⁻ = - 0.33V_{SHE})

O₂ + *e⁻* → O₂⁻ (1)

The calculated energy levels of the conduction band in the trimer (electronic energy levels) makes this reaction thermodynamically favorable. Therefore, the effects of xylem SOD processes on the photocatalytic excitations of the trimer have been examined to investigate the role of excess charge carriers and the contribution of the reactive oxygen species (ROS) in this process.

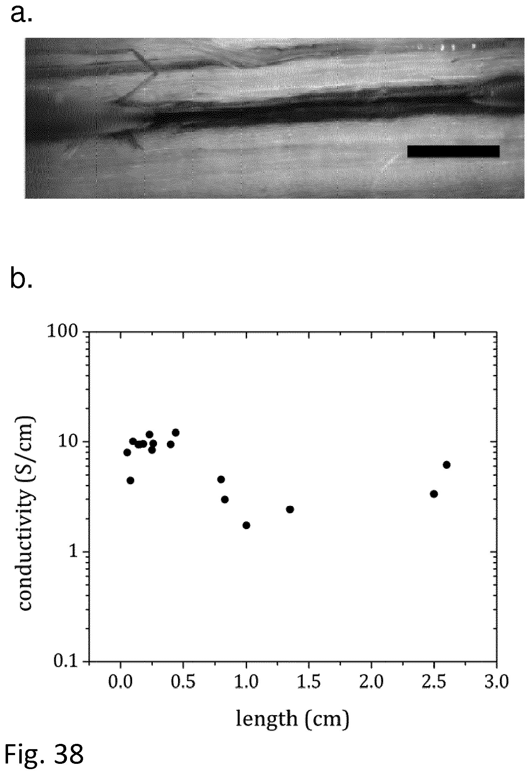

The conductivity of ETE-S based wires was measured using two Au probes applied into individual xylem wires along the stem (Fig. 38) and found to be equal to 7.25 +- 3.38 S/cm (n=17). Pedot-S based xylem wires exhibit two orders magnitude lower conductivity. The high conductivity was retained for cm scale wires showing that good interconnectivity is achieved along the xylem wires. ETE-S polymerized in air exhibits conductivity that varies with the initial concentration of the solution. The conductivity of 10mg/ml solution is 10⁻⁵ S/cm while for 50mg/ml is on the order of 10⁻³S/CM. In the plants we typically administrate 5ml solution of 1mg/ml concentration. An estimation of the total volume of the xylem tubing in a rose cutting of 15cm is 0.15ml taking into account an average diameter of xylem to be 20um. For this volume and since all the solution is taken up form the plant we end up with concentrations of the ETE-S molecule in the order of 30mg/ml. From microscopy images we see that approximately only 10% of the xylem is filled, while the majority of the rest is coated with the material and for that volume we have concentrations up to 300mg/ml. This high concentration in the plant allows the material to organize and polymerize in a way that creates highly conducting wires.

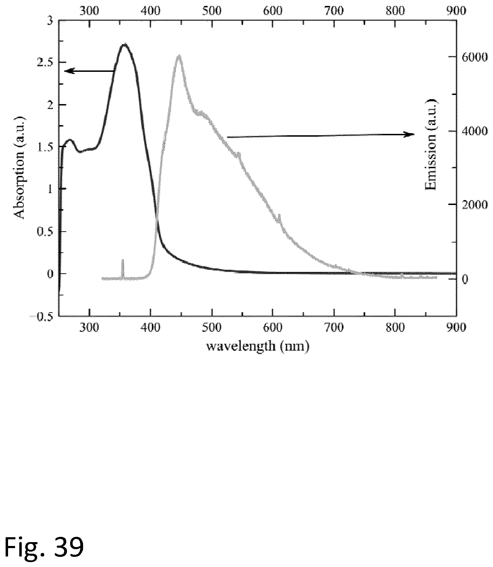

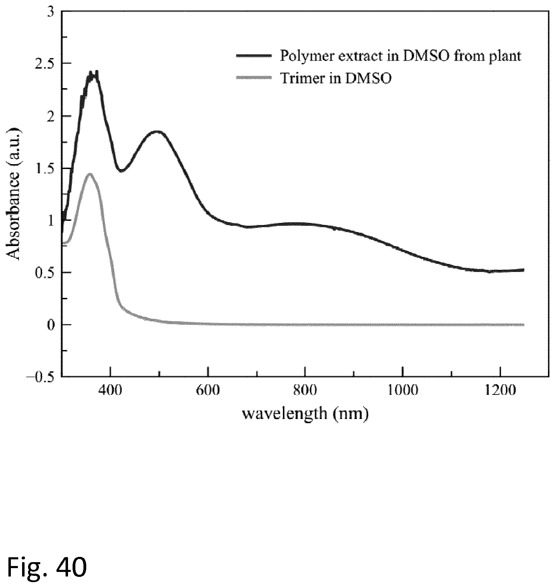

In order to elucidate the polymerization degree of the ETE-S in the plant we extracted material from the xylem and characterize its photophysical properties. We took xylem pieces filled with material and mechanically crashed them in DMSO solvent. In Fig. 34.c. we show the absorption and emission spectra for a typical xylem wire extract after normalization to the plant tissue contribution. The ETE-S oligomer shows absorption at 350nm and emission at 450nm as shown in the spectra taken for 1mg/ml in DMSO solution (Fig. 39). The ETE-S peak is present in the xylem-wire extract suggesting that we have non polymerized molecules present in the vascular tissue of the plant. To reveal the origin of the absorption and emission features of the xylem wires extract in higher wavelengths we performed theoretical calculations based on time dependent density functional theory. Theoretical calculations with sufficient accuracy are used to identify the length and excitation state of oligomers by comparing the calculated photophysical properties with experimental observations. The theoretical calculations for the neutral trimer species, Fig. 34.d reveal strong HOMO- LUMO transitions at 350 nm for absorption and at 433 nm for emission spectra. These transitions saturate towards higher wavelength at 481 nm for absorption and 597 nm for emission as the length of the polymer chain increases to dodecamer and beyond. Therefore we conclude that ETE-S polymerizes in the plant and form polymer chains of 12 and more units. The experimental transition at 800 nm (full spectra is shown in Fig. 40) indicates the presence of polaron and bipoloaron charge carriers in the polymer. Detailed theoretical study of charge carriers in given polymer system is out of scope of this work.

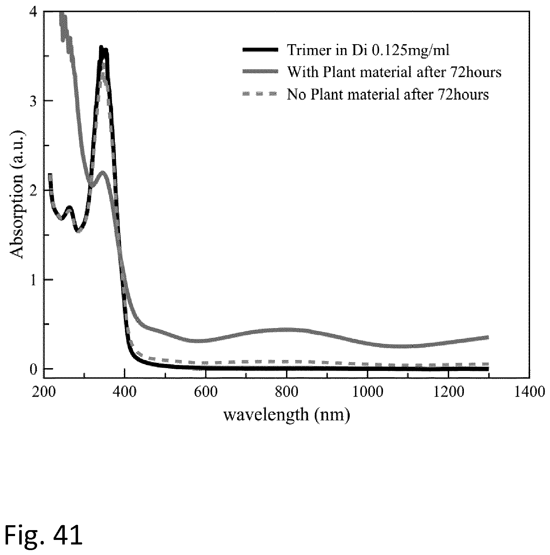

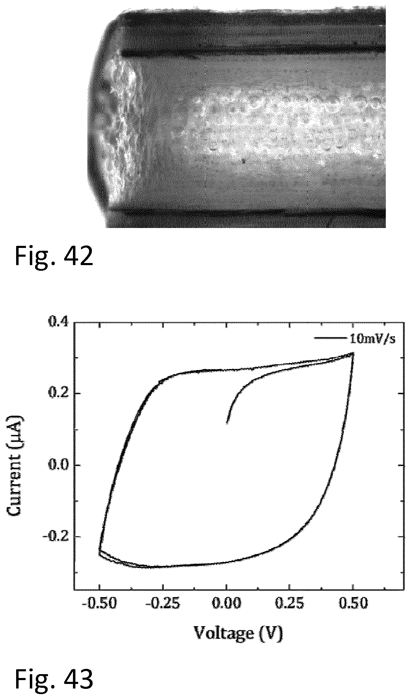

As a control experiment we performed time resolved UV-VIS absorption spectrometry of a solution with and without any plant tissue. The solution that contained the plant tissue polymerized faster than the solution without the plant Fig. 41. This suggests that the plant releases species that contribute to the polymerization beyond the air oxidation and have a catalytic effect. In addition the polymerization on the plant tissue after 18 hours of immersion in the solution was localized in the xylem even though the whole area was exposed in the solution Fig. 42. This exhibits the preferential absorption of the ETE-S on the xylem surface most probably due to its surface energy. We have also observed localized polymerization along the cuts of the plant tissue in the area of bark and phloem. It is known that the wounded part of the plant releases reactive oxygen species (ROS) as a defense response and those species can act as oxidants for the polymerization reaction.

The polymerization of the ETE-S in the plant is driven by the local physicochemical environment in the xylem vessels. Lignin that is one of the main components of the xylem is hydrophobic and favors the interaction with the backbone of the ETE-S, an interaction aided by the confined space of the vessel (and interaction with the charged sulfonate group). In addition proton fluxes are present in the xylem sap, while it is possible to have ROS. We hypothesize that the ETE-S oligomer appears as an invader to the sophisticated defense system of the plant triggering the generation of ROS. Subsequently ROS are released from the parenchyma cells to the adjacent xylem vessels and catalyze the polymerization reaction.

The polymerization of ETE-S was localized in the xylem vessels. However the ETE-S molecule was able to pass the xylem walls and enter the apoplastic pathway in the leaves reaching the spongy mesophyll (Fig. 35.a.). This route is followed by water molecules during the process of transpiration. Dyes like brilliant blue (793 g/mol) and indigo carmine (466g/mol) have molecular weights similar to the ETE-S (536 g/mol) and are commonly used in flower industry since they can pass the vessels and enter the apoplast, coloring leaves and flowers. In Fig. 35.b we show fluorescence confocal images of a leaf that has been attached to a rose cutting that absorbed the ETE-S solution as well as an image of a control leaf (Fig. 35.c.) upon excitation with a 405 nm laser. The blue color on the fluorescent images corresponds to emission from 424-500nm while the green color from 510-607nm. From the images and the spectra shown in Fig 35.d we conclude that the functionalized rose leaf exhibits additional fluorescence in the range of 424-500nm (Fig. 35.c.). Comparing the spectra with the theritical and experimental emission spectra of the ETE-S molecule and polymer we can attribute the additional fluorescence to the presence of ETE-S molecule. As a control experiment we vacuum infused leaves with freshly prepared ETE-S solution and performed spectral analysis. The spectra are similar to the ones from the leaf that was functionalized with the ETE-S molecule through the stem. The fact that an electronic material can be delivered from the vascular tissue of the plant to the apoplast of the leaf creates new promises for the E-plant technology. Electronic circuitry in the veins combined with bio-functionalized molecules in the leaf are preludes to energy harvesting devices, sensors and localized addressing components for plant control and optimization.

**Figure 35****:** Distribution of ETE-S molecule from the stem to the veins and extracellular space of leaves. a. Schematic showing the transport path from the xylem vessels in the leaf to the apoplast and spongy mesophyll space, Confocal fluorescent image of leaf with (b.) and without the ETE-S molecule (c.), d. Spectra from selected locations on a leaf with (blue) and without (green) ETE-S molecule upon excitation with 405nm laser.

Energy harvesting from plants is one of the main visions of E-plant technology. Other direct or indirect strategies are exemplified by extraction of photosynthetic current using nanoprobes and microbial fuel cells that utilize the organic substances released from the roots. A more versatile approach will require an autonomous system where energy conversion and storage co-exist in the same plant. Towards this direction we explored the xylem wires for supercapacitor applications.

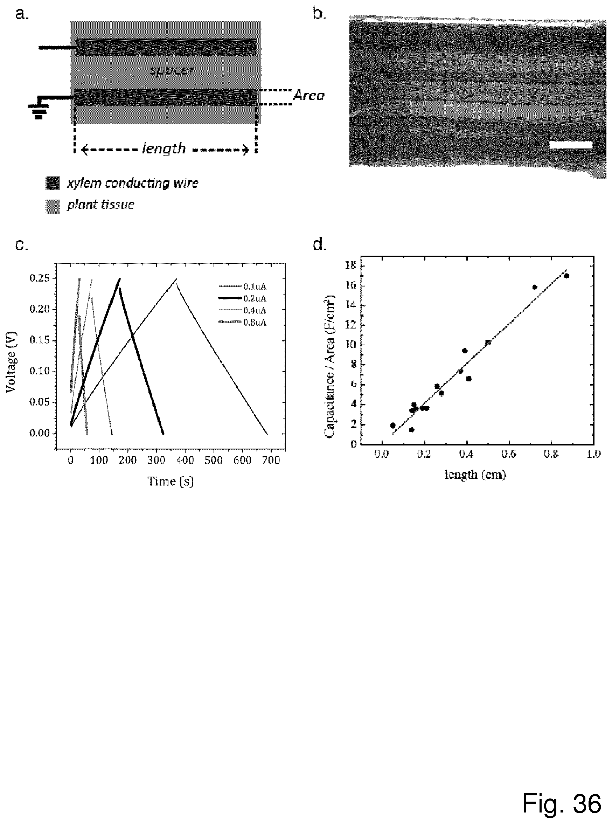

Plant's anatomy upon functionalization with the conducting material provides an ideal architecture for developing supercapacitors in vivo. The long-range conducting xylem wires are parallel and electronically isolated to each other while are surrounded with cellular domains and extracellular space rich in electrolyte. Therefore we explored the charge storage capacity of the xylem wires. Two parallel xylem wires served as the electrodes (capacitor plates) and the plant tissue in between them the spacer. Two Au probes defined the electrodes contacts. In the schematic of Fig. 36.a we show the general structure and the wiring of a supercapacitor along with the optical micrograph of the corresponding supercapacitor in the plant Fig. 36.b.

The xylem supercapacitor exhibits typical behavior as shown in the galvanostatic charging and discharging curves in Fig. 36.c. From the slope of the discharging curve we calculate the capacitance of the device equal to 1.3 10⁻⁴ F The capacitive behavior of the material is indicated and by the square shaped voltamogramm Fig. 43 in the potential range of -0.5V - 0.5V. In order to extract the specific capacitance of the xylem electrodes we measure devices of various dimensions. Xylem wires have typically diameter of 30-50um and lengths in the cm scale. In Fig. 36.d. we show the capacitance per area (diameter of xylem wire) vs the length. From the slope we calculated the specific capacitance and found it equal to 20F/cm3, a value in the typical range for conducting polymers. The fact that the capacitance/area scales with the length of the wire confirms that the total volume of the wire participates in the charge storage as expected for conducting polymers As the polymer is being doped or de-doped ions move in and out of the material resulting in a pseudocapacitive charge storage reaction. The ions are able to penetrate the material resulting in a volumetric capacitance while in the case of a metal the capacitance is limited by the double layer and hence the area of the electrode. The highest charge that we were able to store in the capacitors with the longest wires is 0.65mC with corresponding capacitance of 0.25mF. In this case the length of the wire assuming that the specific capacity is constant is equal to 1.7cm. It is possible to use a parallel connection between the capacitors and achieve larger charge storage.

The high performing conducting wires with conductivity up to 10S/cm and specific capacity of 20F/g allows us to build supercapacitors in a plant for the first time. Combining these charge storage devices with energy conversion devices we can envisage an autonomous system of energy harvesting in the plant. The operation of the supercapacitors relies on mixed conductivity along the PETE-S (for polymer ETE-S) wires and pure ionic conduction along the plant tissue between the wires. During the charging and discharging of the capacitor ions are injected and extracted from the polymer matrix creating local ionic gradients to cellular and extracellular domains nearby. These devices can proof to be powerful tool for monitoring and controlling ion fluxes in the plant.

**Figure 36****:** Plant supercapacitor. **a.** Schematic and wiring of a plant supercapacitor where the xylem wires is the electrodes and the plant tissue in between them the spacer. The area marked in the figure is corresponding to the cross-sectional area of the xylem wires, and is an approximation of the charge storing area. The charge storing volume of the supercapacitor corresponds to area × length of the xylem wire. **b.** Optical micrograph of a rose supercapacitor. **c.** Typical charging - discharging curves for a rose supercapacitor. **d.** Capacitance per area versus length of xylem limiting wire- From the slope we calculate specific capacity of 20F/cm³.

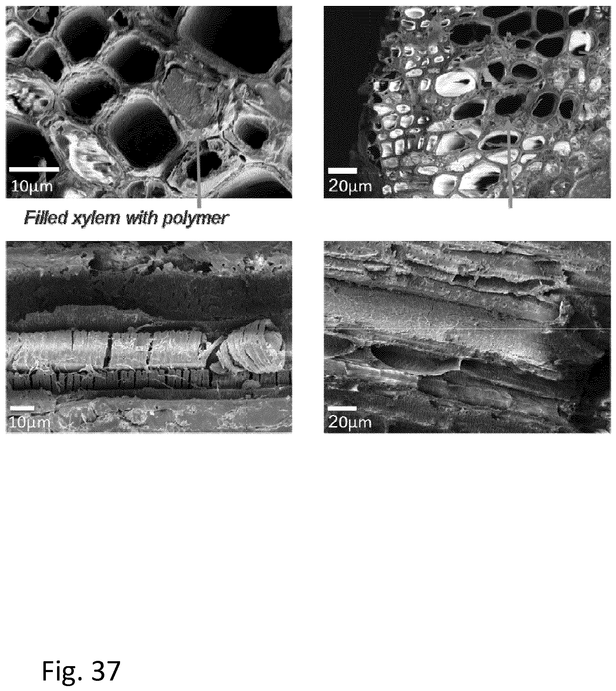

**Figure 37****:** SEM images show that the xylem in some cases is filled while in others is coated with the polymer of ETE-S

**Figure 38****:** a. Two probe conductivity measurement of xylem wires filled with ETE-S polymer, scale bar 500µm. b. Conductivity of the xylem wires filled with ETE-S polymer is in the range of 10 S/cm and is retained for cm length scale wires.

**Figure 39****:** ETE-S oligomer in DMSO absorption (dark blue) and emission (light blue) spectra

**Figure 40****:** Absorption spectra of ETE-S (trimer) in DMSO and polymer extract from the plant in DMSO.

**Figure 41****:** UV-Vis absorption spectra of a Trimer (ETE-S) solution of 0.125mg/ml in DI with and without plant material.

**Figure 42****:** Rose part that was immersed in a solution of 0.125mg/ml of ETE-S. After 18 hours we see that the ETE-S polymerized along the xylem and the edges of the rose part.

**Figure 43****:** Cyclic voltammetry of xylem-supercapacitor

### Conclusions

New materials will enable growth and development of E-Plant technology. In this work we established design parameters for organic electronic materials that can self organize and polymerize in living tissue without external stimuli. We have demonstrated a new water soluble conjugated oligomer that polymerizes in the xylem vascular tissue while as a molecule can overpass cellular junctions and reach the apoplast and spongy mesophyll of leaves. The polymeric wires exhibit high conductivity and specific capacity and together with the plants own architecture it is an ideal platform for in vivo manufacturing of supercapacitors. Moreover, an energy storage system is developed in living plants having the plant structure and physiology as integral part of the device. Bridging the electronic vascular circuitry with complex plants organs such as the leaf and flower paves the way for electronic accessing of photosynthetic and metabolic processes. In this way new technologies and tools can be develop for energy harvesting and plant control.

**Note that the following section A is a summary of suggested actions "A". Note that some of the therein suggested actions are already confirmed or at least partly confirmed in the above description by the performed experiments, that some of the information overlaps or repeats the above description, and that some remains to be performed.**
**A.**

### Introduction

Genetic modification techniques can be used to improve the performance of plants, in terms of resistance to external parameters and increased production. However, deploying these techniques is limited by regulatory and societal conditions. Complementary techniques that can record and regulate plant performance in vivo based on natural hormone chemistry and nutrients are needed. Furthermore, basic understanding of plant biology is hampered, in part, due to a lack of technology that can record and regulate plant physiology at high spatiotemporal resolution in vivo.

We aim to address these needs by building off of recent breakthroughs in bioelectronics, based largely on materials advancements in organic electronics and applied generally to the animal kingdom, to living plant systems.

### Objectives and primary goals of the e-Plants project includes:

- Study and regulate plant physiology through the development of new materials and methods; a complement to genetic techniques and new tools to study biology. (e-Plant-Physiology)
- Realize energy conversion and storage materials and technologies in plants, (e-Power-Plants)
- Electronically regulate the production of substances and materials within plants. (e-Plant-Factory)

### Background

Plants have developed from algae colonies into complex living systems. Growth and plant function are powered by photosynthesis and orchestrated by various internal and environmental physical and chemical parameters. Sucrose, plant hormones and nutrients are long-distance transported along the phloem and the xylem vascular systems to selectively trigger and modulate processes at high spatiotemporal resolution, see figure 1¹. Artificial regulation of plant processes is today typically achieved by exposing the plants to chemicals (nutrients and hormones) in a highly systemic manner². Many long-standing questions in plant biology are still left unanswered due to the lack of means or technological advancement focused on precisely regulating plant function, *in vivo.* A desire would be to record, address and
¹ Biology of Plants. (W.H.Freeman and Company Publishers, 2005).
² Plant biology: Unveiling the Casparian strip. Nature 473, 294-295 (2011). regulate local and isolated, but also connected, plant functions (even at the single cell level) in a highly complex manner and at high spatiotemporal resolution. Today, molecular genetics techniques are widely used to control plant physiology in an effort to enhance of improve plant performance. The proposed *e-Plants* technology is expected to provide a new platform and pathway as a complement to existing genetic techniques presently used in plant biology.

*Organic electronics* is a research and technology field in which electronically and electrochemically active organic materials, *i.e.* ion-conducting, semiconducting, semi-metallic and metallic polymers and molecules, are utilised to achieve components, circuitries and circuits. These materials are today widely explored within the areas of information and communication technology. Organic electronic materials can be manufactured into different electrode or device systems using soft biological and living systems as a template³.

In the proposed project, organic materials will be developed and explored in living plants to generate integrated electronic and chemical systems to achieve various forms of e-Plants circuits.

### Research program

### Organic electronics in plants - short introduction and general concepts

The objective is to develop and explore organic electronic and *iontronic* materials in order to derive the four key-components of an integrated circuit in plants, and those are: electronic/electrochemical/ *iontronic* **contacts, wires, inter-connects** and **components** see figure 3A-C. Electronic and electrochemical contacts will be defined using dendrite electrodes manufactured in the soil and also by grafting inoculated electrodes into the stem of plants. Distributed wires and interconnect networks will be achieved along the vascular systems of the plants by dispensing electronic and ionic conductors along the xylem channels. The goal is to achieve self-organization of conductors along the inner periphery of extended xylems. Devices will be formed and distributed along the various components and junctions of the plant, *i.e.* leaves, axils, stem etc.
³ In vivo polymerization of poly(3,4-ethylenedioxythiophene) in the living rat hippocampus does not cause a significant loss of performance in a delayed alternation task. Journal of Neural Engineering 11 (2014).

For instance, electrochemical or ion transistors, for high-speed signal routing, dispensing or sensing in the stem, can be defined at specific positions along the stem, by combining two conducting xylem wires with a current modulating junction, see figure 3C. Components will also include enzymes, ion conductor membranes and electrochemical electrodes, to enable selective translation between chemical and electronic signals⁴. Electrodes and components of various kinds will be manufactured, such as transistors, sensors, capacitors, rectifiers, and also fuel cell electrodes^{5,6}. Moreover, various organic chemical and electronic circuit systems will be constructed to convert electronic addressing signals into complex delivery of chemical signal patterns, and *vice versa.* All together, these technological augmentations - templated from the plant itself - will enable signalling and sensing at speeds never before seen in the plant kingdom. This will in turn, both enable deeper understanding of the underlying biology, as well as provide a route for efficiently leveraging modern technology to address important issues in agriculture and renewable energy.

The suggested circuit systems will include devices and circuits that process both electronic and ionic signals, also in combination, in electronic/electrochemical/*iontronic* organic devices. Electronic components are primarily based on solid state organic semiconductors; electrochemical devices are typically based on the combination of organic semiconductors and ion conductors (electrolytes); and *iontronic* devices are built up from the combination of different ion selective (mono-, bi- and tri-polar) membranes. In its full scale, the plant will host a distributed and integrated system composed of contacts, inter-connects, devices and circuit wiring. Initially, synthetic materials will be explored while, towards the end of the project, primarily natural materials will be utilized for devices and circuits.
⁴ Kergoat, L. et al. Detection of Glutamate and Acetylcholine with Organic Electrochemical Transistors Based on Conducting Polymer/Platinum Nanoparticle Composites. Advanced Materials 26, 5658-+, doi:10.1002/adma.201401608 (2014).
⁵ Tybrandt, K., Larsson, K. C., Richter-Dahlfors, A. & Berggren, M. Ion bipolar junction transistors. Proceedings of the National Academy of Sciences of the United States of America 107, 9929-9932, doi:10.1073/pnas.0913911107 (2010).
⁶ Bernards, D. A. et al. Enzymatic sensing with organic electrochemical transistors. Journal of Materials Chemistry 18, 116-120 (2008).

### ePlants circuit elements defined in the constructs of the plant

Organic electronic materials, defining circuit elements, will be distributed within the natural components of the plant and its immediate environment. The following concepts are envisioned:
***e-soil:*** The soil is the natural scaffold and source of water, nutrients, and stability for seeds and plants. Electro-active fibre networks and scaffolds, based on electronic/*iontronic* conductors and electrochemical charge collectors will be manufactured in the soil to enable electronic readout, harvesting and delivery of electrons, ions and chemicals to roots and hair.
***e-roots*:** Electrode dendrites will be grown, along wires immersed into the soil, to function as charge collector and stimulating scaffold for the plant's roots. Functionalized electrodes and conductor networks, as well as *iontronic* transportation and hormone delivery networks, will also be formed within the vascular system of roots.
***e-stem*:** The vascular pathways of plants are typically comprised of∼100 µm wide vessels. The xylem is entirely unidirectional, and for many plants each vessel bundle exclusively connects the bottom compartment with selective structures at the top. Conjugated polyelectrolytes, such as PEDOT-S, see figure 3B, will be used to form self-organized long-distance electronic/*iontronic* wires along these tubular biological templates. Interconnects, wires and contacts will be achieved to transfer electronic and chemical signals from the outside into the vessels via graft electrodes.
***e-leaves***: The leaves are versatile antennas and factories that collect, emit and produce optical energy, CO₂, O₂, H₂O and carbohydrates, respectively. The components, structures and vascular systems of the leaves offer an array of opportunities to define, distribute and connect electronic devices. Via the xylem or through the stoma gates, soluble polymers, monomeric units, enzymes and oxidation agents can be administrated into the veins and the palisade and spongy parenchyma. For example, the electronic/*iontronic* polymer system PEDOT:PSS, see figure 3A, combined with nano-fibre hydrogel-scaffolds can generate distributed electrode systems along the spongy parenchyma of leaves.
Plants lack central and peripheral nervous systems that emit, transport, receive, and process signals that coordinate plant functions with the speed and responsivity found in the animal kingdom. Here, generic integrated electronic-chemical circuits are proposed, composed of transport wires, feedback circuits together with enzymatic sensors, and (hormone) delivery devices to regulate intra- and even inter-plant physiology in hitherto impossible ways.

### Introduction

*e-Plant* circuits, and its included components, wires, interconnects and contacts, will be composed of linear (1D), areal (2D) and bulky (3D) *iontronic-* and electronic conducting and electro-active structures that are *in vivo*-processed and -manufactured inside the various constructs of plants. First, materials will be dispensed into compartments and vascular pathways via grafted inlets, the roots and hairs and also through the stoma gateways. Then, immediately after entrance, the electro-active materials, in the form as dissolved oligomers and polymers, will distribute and organize promoted by transpiration, cohesion-tension transport and the plant tissues as the templates, respectively. Finally, the stress responsive mechanism of the plant will trigger gelling and also polymerization of the electro-active materials into desired architectures and device structures.

### WP1: Electronic materials, in vivo synthesis and processing

Lignin is the product of lignification and is a cross-linked network providing necessary mechanical strength and resistance (stress response) against pathogens, and makes also cell walls impermeable to water. Peroxidase enzymes produce reactive oxygen species that in part regulates the overall lignification process^{7,8}. The high redox potential of the peroxidases provides excellent conditions for template-assisted polymerization of electro-active materials, *in vivo.* Here, we suggest establishing a library of water-soluble oligothiophenes (*e.g*. EDOT-Th-EDOT tri-mers, Fig. 3') that polymerizes via oxygen catalysis, and governed by the lignification process. In addition to obtain high electronic conductivity, the oxidation level of the peroxidases also provide desired morphology, adhesion, cross-linking and anchoring characteristics of the resulting electro-active polymer.
⁷ The xylem as battleground for plant hosts and vascular wilt pathogens, Koste A. Yadeta1 † and Bart P. H. J. Thomma, Front. Plant Sci., 23 April 2013, dx.doi.org/10.3389/fpls.2013.00097
⁸ Journal of Experimental Botany, Vol. 60, No. 2, pp. 367-376, 2009 doi:10.1093/jxb/ern27

Then, we employ peroxidase-triggered polymerization of oligo-EDOTs (biEDOT, triEDOT and terEDOT, see Fig. 3'e) to generate wires throughout the roots, xylem, axils, petioles, petals and leaves of the plant. The confined space of for instance the xylem expresses template characteristics similar to liquid crystalline (LC) phases. A LC-template approach will therefore be explored to derive conductors reaching >1000 S/cm in conductivity (comparable with the metallic phase of PEDOT:PSS⁴). Thus, with stiff metallic chains ordered in a LC phase, optimal conditions for conduction will here be obtained by supramolecular self-assembly throughout the long-range vascular systems.
Further, the low redox potential of the oligo-EDOTs and their amphiphilic character will be explored for oxygen-catalythic grafting of nano-particles, various molecular entities and enzymes to tailor-make device-specific electronic functions of the materials. These resulting transducers will then be structured into desired architectures that resemble organic electronic components and junctions.

Trimers with specific charges: sulfonate (a), ammonium (b) and carboxylate (c). PEDOT-S:Na with different chain lengths (n = 10-100) (d) and polymerization via reactive oxygen catalysis (e) are shown on the next page.

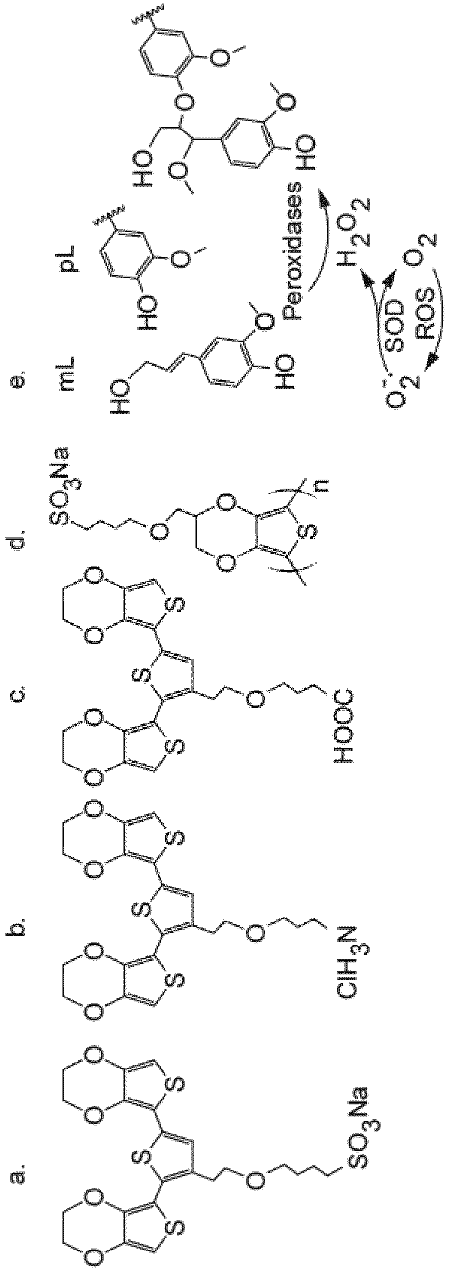

The vast array of physiological reactions of plants is in part dictated by the transport, delivery and recognition of various ionic species, nutrients and pH. Ions can selectively be transported, delivered and sensed using polymer membrane materials. While combined with electronic polymer electrodes, mono-, bi- and tri-polar membrane structures can define different components that process and amplify ion signals. For instance, sensors, fuel cells, drug delivery components/circuits and water splitters have successfully been realized based on the combination of PEDOT and polyelectrolytes.

Linear electrolyte polymers suffers from a random-coil geometry that prevents us to establish long-range assembly of *iontronic* membranes along the veins and vascular systems of plants, due to clogging at the natural pits and junctions of the plants. We propose to derive a novel class of electrolytes based on functionalized dendrimers, which is expected to swiftly transport across many of the tight junctions of *e.g.* the vascular system of the plant. Recent results achieved in our lab indicate a high electrophoretic transport and efficient processing of a vast array of ionic species (small ions, hormones, neurotransmitters and liposomes) as these *iontronic* dendrimers are included in devices.

The dendrimer electrolytes (*dendrolytes*) should include stationary charges to impart selectivity with respect to size and charge. The dendrolytes will provide an internal open space, thanks to their rigid spherical structure, which will promote high ion conductivity even for larger ions. The *dendrolytes* will also include cross-linking sites that will allow us to trigger dendrite-to-dendrite cross-linking at desired sites through peroxidase catalysis. They will also be optimized for efficient performance in mono-, bi- and tri-polar membrane-based devices to sense, deliver and process the ions of plants.

Hyperbranched polyglycerols (HBPG), functionalized with allylic crosslinkers and sulfonates, and how they further polymerize, in-vivo, via reactive oxygen/peroxidase catalysis is illustrated on the next page.

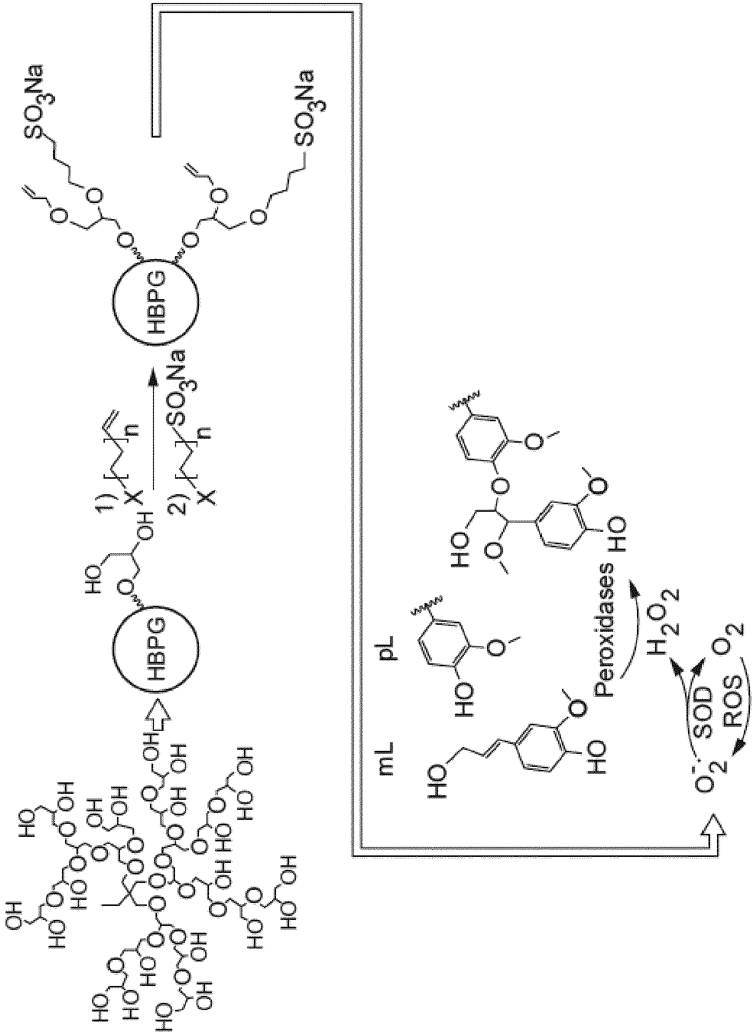

In plants, algae's and cyanobacteria's, thylakoid formation requires the action of vesicle-inducing protein in plastids 1 (VIPP1)⁹. Plants cannot survive without this protein, and typically a reduced VIPP1 level lead to slower growth and paler plants with reduced ability to photosynthesize. We will pursue integration techniques, similar to recent progress of OEIP delivery of lipids, for stabilizing, stimulating and over-expressing the lipid sensitive thylakoid formation. We will investigate plant-plastids response to delivery of phospholipids and galactolipids, not exclusively to obtain stronger, healthier and faster growth of plants, but also for future plant-symbiotic extraction of energy¹⁰.

### Electronic and iontronic polymers in 1D, 2D and 3D structures

*In vivo*-manufactured and -templated conjugated polymers (electronic) and the *dendrolytes* (*iontronic*) provide transport and processing of charged signals. These will be processed and organized into structures resembling the key-structures of components, circuitry and circuits.
- 1D-wires, contacts and interconnects, for electronic and *iontronic* transport and processing, will be derived that enables long-range transfer of signals from the root hairs all the way to the leaves
- Electronic materials forming (1D/2D) connections using the pits, axils, membranes and veins of the plants as the template and compartment to form electronic and *iontronic* pn(pnp)-junctions
- Areal (2D) and bulky (3D) electrodes will be structured along. the phloem and spongy parenchyma of stems and leaves, respectively, to generate electrodes and mixed ion-electron conductors

### Components, circuitry and circuits, in vivo

From the results derived in 2.4.4, including 1D, 2D and 3D electro-active and conducting structures, an array of different devices and electrodes will be manufactured and explored:
⁹ IM30 triggers membrane fusion in cyanobacteria and chloroplasts, R. Henning et al., Nature Communications 6, Article number: 7018 doi:10.1038/ncomms8018
¹⁰ Energy Environ. Sci., 2013,6, 1891-1900 DOI: 10.1039/C3EE40634B
- Linear and dendritic (e.g. contacts for the root and wires for the stem) and also areal and bulky (phloem and leaves) electrodes, wires and contacts
- Electronic components, based on conjugated polymers and dendrolytes to form electronic and electrochemical transistors and electrodes for addressing, signal processing and sensors
- *Iontronics,* based on p- and n-type dendrolytes combined with electrodes, to generate mono-, bi- and tri-polar devices for water splitting (pH) and delivery of *e.g*. hormones and nutrients
- Bulky and areal mixed ion-electron electrode systems for charge storage and conversion

### e-Plants demonstrators

The project will end with the achievement of the following demonstrators:
- Regulation of plant physiology using the integration of organic electronic sensors and *iontronic* delivery devices combined with control circuitry (*e-Plant-Physiology,*
- Combined energy conversion and storage in plants. (*e-Power-Plants,* figure 5)
- Electronic control over the production of materials in plants. (*e-Plant-Factory,*)

The above referred to "e-plants" project may be referred to as "trans-plant" below.

10% of earth's population is living in hunger conditions, at the same time earth's population is increasing and the land for agriculture is reducing due to desertification or urbanization (who). 85% of our food comes directly from plants while the rest 15% come indirectly form them (ref.WHO). In order to address the challenge of undernourishment we need to understand better how plants grow, how they interact with their environment, abiotic and biotic one and how to increase plants productivity. During the past decades important progress has been made in plant biology due to the discovery of genome sequencing and the development of genomic tools (transcriptomics, metabolomics, proteomics) that give insight on how genes, metabolites and proteins orchestrate the various processes in plants (ref.General biology book). Still many questions remain unanswered highlighting the need for the development of complementary technologies to genetic methods. Plant's growth and productivity are determined by photosynthesis and the way its products are distributed and utilized during the growth and development of the plant. Sucrose being one of the main products of photosynthesis is the fuel that drives all growth and development in the plant. It is also consider a secondary signal mechanism for various possesses such as flower timing (ref.S-S rel). Sucrose is transported though the vascular tissue (phloem) from the leaves (sources) to all growing part of the plant and it is stored in the sinks (e.g. root). The goal of Trans-Plant is the development of a complementary technology based on organic bioelectronics that will allow in vivo sucrose monitoring in phloem from source to sink and give new insight to the transport mechanism of sucrose. This technology will enable a direct measurement, real time monitoring and the macroscopic mapping of sucrose in various locations of the plant through a sensor network.

Organic bioelectronics is a field that couples organic electronics with the biological world. An organic electronic device can be used to induce a biological function or a biological event can be recorder by an organic electronic device. Based on soft materials that can conduct both electronic and ionic carriers these devices are ideal for translating addressing electronic signals to complex ionic outputs and vice versa.

This first effort involves the use of ion pumps,that are delivery devices with high spatiotemporal resolution to deliver hormones in the roots of a plant. This technology will enable plant biologists to control hormone's delivery very precisely and very locally. One can envisage many more applications of organic bioelectronic to plants; regulating and monitoring physiology and metabolic activities as a new tool for plant biologist and/or forest industry and agriculture, promoting growth in extreme conditions (drought, severe cold or even in space environment - microgravity), energy harvesting or even production of materials through the biochemical processes of plants.

### Research methodology

The ultimate objective of Trans-Plant is to integrate organic electronic materials and devices into a living plant for monitoring the sucrose transport of the plant in vivo. The plant structure will be used for delivering materials and as a template for manufacturing the devices. The project will focus on the development of a sensor network based on OECTs that are manufactured in vivo and the capability of this network to monitor the sucrose concentration in the phloem from source (leaf) to sink (root). In figure 3 the very basic concepts of the OECT sensor (3a) and sensors network (3b) are illustrated. The principle of operation of OECTs relies on the fact that the channel conductivity can be modulated upon application of gate bias through an electrolyte. The channel consist of a conducting polymer and when voltage is applied at the gate ions from the electrolyte penetrate the channel and compensate the dopants resulting in dedoping of the channel and decrease in the source drain current. In the case of the sensor network the channel of each transistor will be constructed in the plant structure and the gating of the transistor will be realized through the phloem where the sucrose is transported.
Task 1: Development of OECT channels utilizing the plant structure in three key locations: the leaf, the stem and the root. Dicots plants such as rose or beans will be used initially as model plants. The channel will consist of the conducting polymer Pedot-S that we have shown can be self-organized in the xylem (figure 6). In parallel new custom-made materials, from collaborators or the in house synthesis, will be tested as well. In order to introduce electronic materials into the plant and manufacture devices in vivo we will utilize the plant's functions, focusing on the ones that allow the plant to exchange substances with its environment. The vascular tissue of the plant creates a connecting network all over the plant with longitudinal and lateral transport making it ideal for introducing and delivering materials. Through the process of transpiration the water flows from roots to leaves in the xylem vessels due to cohesion of water molecules and hydrostatic pressure. We will create in-lets and out-lets in the plant stem and "feed" the plant with electronic materials forming the channel in various locations in the stem. A second environment of interest is the leaf. Leaves are complex plant organs where the production of sucrose occurs though photosynthesis. The gas exchange between the internal structure of the leaf and the environment occurs through the stomata, that are holes that open and close due to specialized guard cells. Electronic materials can be introduced in the leaf's spongy mesophyll with vacuum infiltration technique through the stomata (figure 8). A third area of focus will be the roots where communication between soil and plant takes place. Water molecules and macro- and micro-nutrients enter the plants vascular system through the roots following a symplastic () or apoplastic pathway (). Roots are considered a sink sink for sucrose. Focus will be given in the development of the transistor in the three areas: in the xylem in the stem, in the spongy mesophyll of leaves and surface or xylem of the root.
Task 2: Functionalise the gate probe for detection of sucrose. Preferably, to detect sucrose that is located in the phloem vascular tissue we will utilize probes with tips of few micrometers in diameter that will penetrate the epidermis of the plant and reach the phloem with minimal disruption of the phloem flow. When sucrose reacts with the enzymes of the probe, the potential of the gate will change and this will cause a change in the current of the channel. The transistor is an inherent amplification device where small changes in the gate voltage cause large changes in the source-drain current and output of the device. In combination with the close proximity of the channel and gate will ensure the ability of the sensor to monitor with precision the sucrose concentration. Parameters such as sensitivity, selectivity and dynamic range of the sensor will be thoroughly investigated. The probe may be be metallic, Pt or Au and will be functionalized with enzymes with appropriate surface chemistries. In order to be able to detect sucrose we will have to use two enzymes first the enzyme invartase that will break down sucrose into glucose and fructose and then the enzyme glucose oxidase that will react with glucose and change the potential of the gate. Another strategy will be to electro polymerize conducting polymers on the metallic tip and entrap the enzyme during polymerization.

The idea to manufacture organic electronic devices, inside a plant, in vivo, and establish communication pathways between the plant and the device, that is the main goal of this project, is not only original but also revolutionary. It will open up new possibilities for monitoring and controlling physiology in plants and result in the development of a state of the art device concept and technique for organic bioelectronics and plant science. The project has a strong multidisciplinary character and relies on developments at the edge of knowledge of organic bioelectronics () and plant science (), which combined will deliver a very powerful tool. Furthermore, independent of the final aim, the project is going to reveal new insights into the development of in vivo organic devices, that will be able to communicate with living tissue. In the future one can envisage devices that can harvest energy from the plant - based on fuel cell concepts or hacking directly into photosynthesis, and can influence the biochemical processes and produce materials from plants. These devices can also be coupled with delivery devices such as ion pumps in order to create a sensing-delivery coupling that can regulate plant's functions. From plant science perspective the technology can be established as a complementary to genetic tools. Hence the European research community in the fields of bioelectronics and plant science will in any case benefit tremendously from this project.

The skilled person realizes that a number of modifications of the embodiments described herein are possible without departing from the scope of the invention, which is defined in the appended claims.

For instance, it shall be noted that examples given above are detailed examples of how to achieve above mentioned electric components. It shall be understood that even though the electric components are provided with a certain electron conducting material it does not mean that the described material is the only material which can be used for providing the electric components.

### FURTHER INFORMATION ABOUT THE COMPOUND OF FORMULA I and II: POLYMERIZABLE COMPOUND

### FIELD OF THIS PART OF THE DISCLOSURE

This part of the disclosure relates to a compound and the use thereof for producing a conductive polymer. This part of the invention further relates to a method for producing a conductive polymer inside plant material.

### BACKGROUND

Conductive polymers have been the subject-matter of numerous publications in the last few decades. One of the most studied and explored conductive polymers is poly(3,4-ethylenedioxythiophene) (L. Groenendaal *et al*.), either doped with polystyrene sulfonate or self-doped (R. H. Karlsson *et al*.) via a covalently attached anionic side group (M. Hamedi *et al*.).

Hence, there is a desire to provide new compounds, devices, methods and materials with new propeties which may at least in part enable new technical implementation.

According to an example, there is provided a compound of formula I 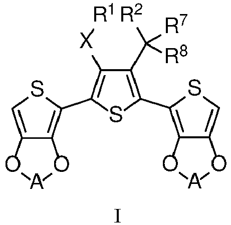 wherein
A represents a C₁-C₄-alkylene diradical optionally substituted with one to four R⁹;
X is hydrogen or CR⁵R⁶;
when X is hydrogen, R¹ is absent;
when X is CR⁵R⁶, R¹ is selected from the group C₃-C₆-cycloalkyl, aryl and C₁-C₈-alkyl in which one -CH₂- is optionally replaced by -O- or -S-, said C₃-C₆-cycloalkyl, C₁-C₈-alkyl or aryl optionally carrying one to three R³, said C₁-C₈-alkyl may be selected such that R¹ and R² form a 5-10 membered ring;
R² is selected from the group C₃-C₆-cycloalkyl, aryl and C₁-C₈-alkyl in which one -CH₂- is optionally replaced by -O- or -S-, said C₃-C₆-cycloalkyl, C₁-C₈-alkyl or aryl optionally carrying one to three R³, said C₁-C₈-alkyl may be selected such that R¹ and R² form a 5-10 membered ring;
R¹ and/or R² carries one or two R⁴;
each R³ and R⁹ is independently selected from hydrogen, hydroxy, halogen, C₁-C₃-alkyl, C₁-C₃-haloalkyl and C₁-C₃-alkoxy;
each R⁴ is independently selected from hydrogen, sulfonate, carboxylate, sulfinate, phosphonate and phosphinate or the corresponding acid or the corresponding sodium, potassium or lithium salt thereof; and R⁵, R⁶, R⁷ and R⁸ are each independently selected from hydrogen, halogen, C₁-C₃-alkyl, C₁-C₃-haloalkyl and C₁-C₃-alkoxy.
The respective atom, of the two substituents on the central thiophene, that connects to the thiophene i.e. position X and the carbon atom carrying R², R⁷ and R⁸, are either an sp³-hybridized carbon or a hydrogen. Hydrogen and sp³-hybridized carbons are not π-donating groups and do thus not contribute with electrons to the system of conjugated double bonds in that manner. For example an oxyxen or an sp²-hybridized carbon in the corresponding position would stabilize the conjugated π-system and the compound would be less prone to polymerization. R¹ and R² are linear or branched alkyl chains that are optionally substituted. At least one of R¹ and R², however, has an anionic group or an acidic function (R⁴), such as a sulphonate or a carboxylic acid function to increace the water solubility of the compound. A corresponding acid may also be used during synthesis of the compound as a change in pH may easily yield the anionic form. When X is hydrogen, R¹ is absent. R³, R⁵, R⁶, R⁷, R⁸ and R⁹ may be hydrogen, but may also be other suggested groups with which it is possible to finely tune the properties of the compound. The number as well as the type of R³ and R⁹ may influence such properties. The compound as a whole should be sufficiently easily polymerizable not to require external stimuli, but not so prone to polymerize so that it obstruct the spreading of the compound prior to polymerization. The compound is preferably water soluble.

R⁴ may be anionic for self-doping purposes according to some embodiments.

According to a detailed example, R² is selected from 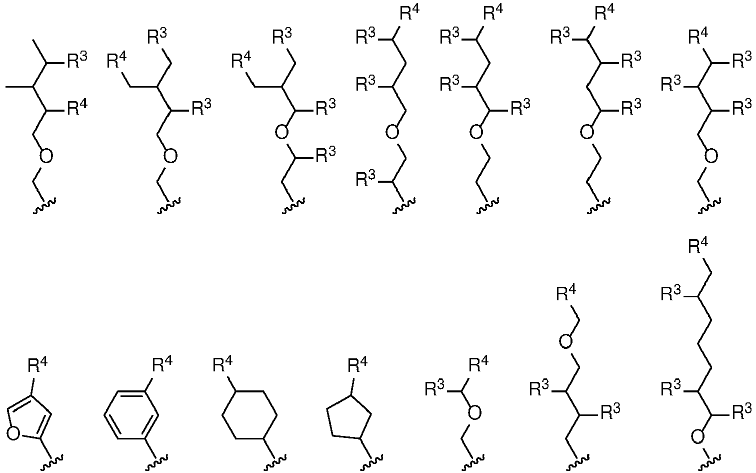 and R¹ is independently selected from the same group as R², when X is CR⁵R⁶.

According to a detailed example, R¹ is C₁-C₃-alkyl when X is CR⁵R⁶; and R² is C₁-C₈-alkyl wherein one -CH₂- is replaced by -O-, said C₁-C₈-alkyl carrying one R⁴ and optionally carrying one or two R³. The use of a short alkyl chain in R¹ may be used to slightly increace hydrophobicity. The presence of an ether function in R² may facilitate diverse syntheses.

According to a detailed example, R³, R⁵, R⁶, R⁷ and R⁸ are independently selected from hydrogen, fluorine, C₁-C₃-alkyl and trifluoromethyl. Using fluorine, C₁-C₃-alkyl or trifluoromethyl as substituents or hydrogen may be used not to alter the properties of the compound excessively.

According to a detailed example, R⁴ is selected from sulfonate, carboxylate or the corresponding acid or the corresponding sodium, potassium or lithium salt thereof.

According to a detailed example, A represents ethylene and R⁹ is selected from hydrogen and C₁-C₃-alkyl. Ethylene in EDOT is a common bridge in conductive polymers. Hydrogen and C₁-C₃-alkyl as R⁹ may be used to finely adjust the properties of the compound.

According to a detailed example, X is hydrogen. Using a hydrogen as X may facilitate synthesis and starting materials may be more readily available commercially. Further, in cases when a substituent is not necessary for the purpose at hand, hydrogen is sufficient.

According to a detailed example, R² is selected from 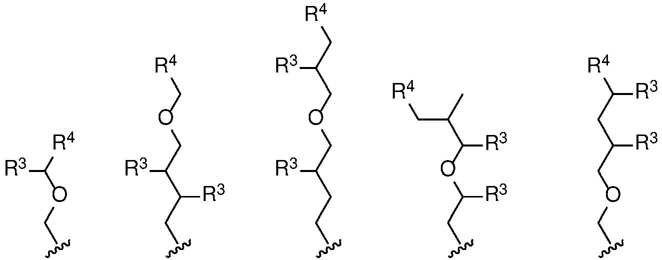 Alkoxyalkyl type groups have low reactivity.

According to a detailed example, R² is 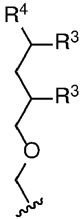 and R³ is selected from hydrogen, fluorine, methyl and trifluoromethyl.
An alkoxyalkyl type group with the length of approximately 8 atom length carrying hydrogen, fluorine, methyl and trifluoromethyl substituents and an appropriate anionic or easily deprotonatable group may be beneficial for self-doping, water solubility while maintaining low unnecessary reactivity.

According to a detailed example, the compound is

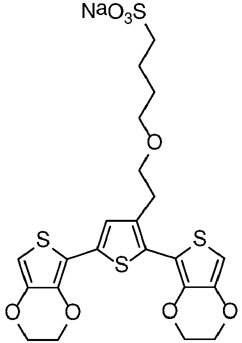

According to another aspect of this part of the disclosure, there is provided a use of a compound of formula I or an embodiment thereof, for producing a conductive polymer.

The compound may be used in the production of conductive polymers for any application. The water solubility of the compound of course makes it especially useful in applications requiring aqueous solutions. One application where the compound has been found particularly interesting is for polymerization in plants.
According to an aspect of this part of the invention, there is provided a method for producing a conductive polymer inside plant material comprising the step: a) bringing plant material into contact with a solution of a compound of formula I or an embodiment thereof. Bringing plant material into contact with a solution of a compound of formula I may spread the solution into the plant material, where it polymerizes.

According to an embodiment of this aspect, bringing plant material into contact with the solution is done by immersing a portion of said plant material in said solution such that said solution is spread via canals inside said plant material. By immersing a portion of the plant material in a solution of a compound according to this part of the invention, the solution may spread via canals inside said plant material and polymerize inside the plant material. Distributing an already produced conductive polymer inside a plant is difficult due to that a polymer is often a solid, an amorphous material or at least a gum. A solution may spread in canals suitable for distributing liquids.

According to an embodiment of this aspect, the plant material is a living plant or a part thereof. Spreading a solution of a compound according to an embodiment inside a plant or a part thereof may be done via canals inside a plant. The compound may then polymerize inside, such as in the xylem or the phloem. In applications where the compound is used in contact with a living plant or a part thereof, the compound is preferably selected such that it is not toxic to the plant.

According to an embodiment of this aspect, the method further comprises the step of:
a) waiting such that a polymerization of the compound occurs inside the plant material.
Actively or passively transporting a solution of a compound according to this part of the disclosure and then allowing the compound to polymerize inside leads to a conductive polymer inside the plant material.

According to an embodiment of this aspect, the polymerization occurs without external stimuli. A compound according to this part of the disclosure polymerizes more rapidly in contact with the plant and a conductive polymer may thus be produced without external stimuli e.g. by adding a radical initiator.

According to an embodiment of this aspect, the polymerization is accelerated by the chemical environment in the plant. The presence of the plant speeds up the polymerization of the compound.

According to another aspect of this part of the disclosure, there is provided a conductive polymer comprising the repeating unit of formula II 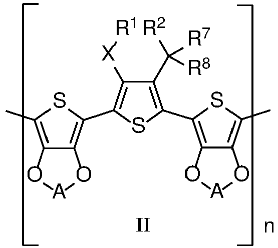 wherein
A represents a C₁-C₄-alkylene diradical optionally substituted with one to four R⁹;
X is hydrogen or CR⁵R⁶;
when X is hydrogen, R¹ is absent;
when X is CR⁵R⁶, R¹ is selected from the group C₃-C₆-cycloalkyl, aryl and C₁-C₈-alkyl in which one -CH₂- is optionally replaced by -O- or -S-, said C₃-C₆-cycloalkyl, C₁-C₈-alkyl or aryl optionally carrying one to three R³, said C₁-C₈-alkyl may be selected such that R¹ and R² form a 5-10 membered ring;
R² is selected from the group C₃-C₆-cycloalkyl, aryl and C₁-C₈-alkyl in which one -CH₂- is optionally replaced by -O- or -S-, said C₃-C₆-cycloalkyl, C₁-C₈-alkyl or aryl optionally carrying one to three R³, said C₁-C₈-alkyl may be selected such that R¹ and R² form a 5-10 membered ring;
R¹ and/or R² carries one or two R⁴;
each R³ and R⁹ is independently selected from hydrogen, hydroxy, halogen, C₁-C₃-alkyl, C₁-C₃-haloalkyl and C₁-C₃-alkoxy;
each R⁴ is independently selected from hydrogen, sulfonate, carboxylate, sulfinate, phosphonate and phosphinate or the corresponding acid or the corresponding sodium, potassium or lithium salt thereof; and
R⁵, R⁶, R⁷ and R⁸ are each independently selected from hydrogen, halogen, C₁-C₃-alkyl, C₁-C₃-haloalkyl and C₁-C₃-alkoxy. According to embodiments, X, A and R¹-R⁹ may be as defined above with reference to a compound of formula I.

According to an embodiment of this aspect the repeating unit is

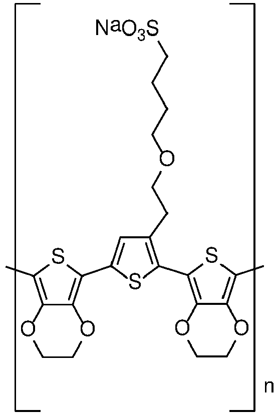

### EXAMPLES

### Materials and method

Nuclear magnetic resonance (NMR) on ¹H (600 MHz and 300 MHz) and ¹³C (75 MHz) were recorded on a Agilent(Varian) spectrometer. The deuterated solvent was used as internal standard, for CHCl₃ (δ_{H} = 7.26; δ_{C} 77.23), CD₂HOD (δ_{H} = 3.31; δ_{C} 49.15), HDO (δ_{H} = 4.80) and DMSO-*d*₅ (δ_{H} = 2.50; δ_{C} 39.51) were used as references.

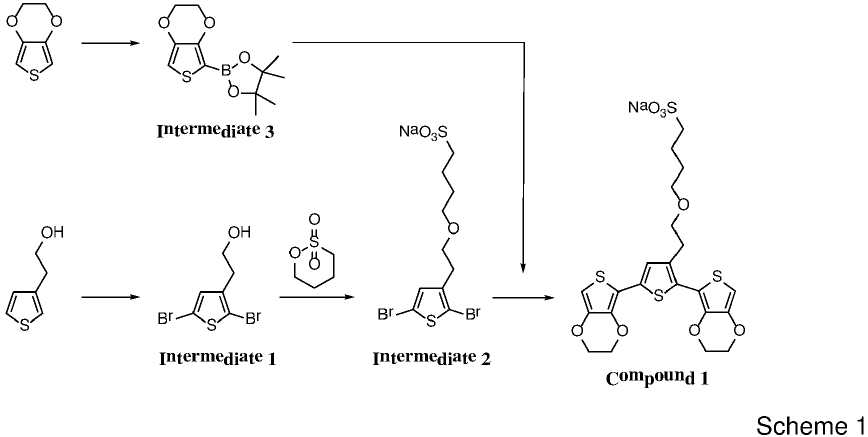

### Synthesis of compound 1 (Scheme 1)

### Intermediate 1

To a stirred solution of hydroxyethyl thiophene (2g, 15.60 mmol) in dry DMF (80 ml, 0°C) was added NBS (5.83g, 32.76 mmol) in small portions over 15 min. The resulting mixture was allowed to reach RT and stand for 3h. The reaction was quenched with distilled water (50 ml) and extracted with EtOAc (3×50 ml). The separated organic layer was then washed with BRINE (50 ml), distilled water (50 ml) and dried with MgSO₄. Evaporation of EtOAc resulted in an oil that was purified with flash chromatography (EtOAc) to give (Intermediate 1) 3.01g (70%) as an oil. ¹H NMR (CD₃OD) δ: 2.23(s, 1H), 2.76(t, 2H), 3.76(t, 2H), 6.85(s, 1H). ¹³C NMR (CD₃OD) δ: 33.02(1 C), 61.93(1C), 109.66(1C), 111.07(1C), 131.55(1C), 139.43(1C).

### Intermediate 2

To a stirred solution of dibromo-hydroxyethyl thiophene (3.44g, 12.03 mmol) in dry Toluene (35 ml) was added NaH (0.45g, 18.53 mmol) in portions over 30 minutes. The mixture was then cooled to 0°C and 1,4-butansulton was added. The resulting mixture was allowed to reach RT before the temperature was raised to reflux. After 15 hours the resulting solution was cooled to RT. The precipitate formed was filtered and washed with acetone repeatedly. The resulting yellow/white powder was dried in a vacuum oven to be used without further purification to give (Intermediate 2) 3.2g (60%). ¹H NMR (D₂O) δ: 1.71(m, 2H), 1.84(m, 2H), 2.78(q, 4H), 3.48(t, 2H), 3.56(t, 2H), 6.98(s, 1H). ¹³C NMR (D₂O) δ: 21.77(1 C), 28.61(1C), 29.77(1C), 51.22(1C), 69.33(1C), 70.37(1C), 108.64(1C), 110.15(1C), 131.87(1C), 140.35(1C).

Intermediate 3 To a stirred solution of EDOT (2.25g, 15.83 mmol) in dry THF (32 ml, -78°C) was added BuLi (7 ml, 17.41 mmol). The resulting mixture was allowed to reach 0 °C and then cooled again to -78°C. 2-isopropoxy-4,4,5,5-tetramethyl-1,3,2-dioxaborolane (3.6 ml, 17.41 mmol) was added. The reaction was allowed to reach room temperature before quenched with a mixture of ice and ammonium chloride. The mixture was diluted with Et₂O and extracted with water (3x50 ml). The separated organic layer was then extracted with BRINE (50 ml), distilled water (50 ml) and dried with MgSO₄. Evaporation of Et₂O resulted in a crude oil/solid that was further purified by precipitation in Hexane. The solid powder was filtered and vacuum dried to give monoborolated EDOT (Intermediate 3) 2.6g (60%). ¹H NMR (CD₃OD) δ: 1.33(s, 12H), 4.17(m, 2H), 4.29(m, 2H), 6.62(s, 1H). ¹³C NMR (CD₃OD) δ: 24.86(6C), 64.41 (1C), 65.18(1C), 83.93(1C), 107.61 (1C), 142.48(1C) 149.19(1C).

### Compound 1

To a stirred solution of dibromo-hydroxyethyl thiophene (0.2g, 0.450 mmol) and monoborolated EDOT (0.25g, 0.946 mmol) in H₂O/DMF (1:2, 6ml) was added PEPPSI® (0.0092g, 0.014 mmol) and KF (0.16g, 2.7 mmol). The reaction was heated to 80°C. After three hours the resulting mixture was cooled to RT and quenched with acetone. The resulting solid/mixture was filtered through Celite and evaporated. The crude solid was dissolved in degassed Millipore water and precipitated with acetone. The procedure was repeated three times. The resulting powder was filtered and vacuum dried to give compound 1 (0.18g, 0.315 mmol) as a yellow/orange powder. ¹H NMR (CD₃OD) δ: 1.72(m, 2H), 1.86(m, 2H), 2.83(t, 2H), 2.91(t, 2H), 3.48(t, 2H), 3.63(t, 2H), 4.3(m, broad, 8H), 6.28(s, 1H), 6.45(s, 1H), 6.99(s, 1H). ¹³C NMR (CD₃OD) δ: 21.82(6C), 28.66(1C), 29.65(1C), 51.26(1C), 64.58(1C), 64.69(1C) 64.97(1C), 65.17(1C), 70.30(1C), 70.53(1C), 96.58(1C), 98.91(1C), 109.46(1C), 111.62(1C), 124.92(1C), 126.86(1C), 134.25(1C), 136.77(1C), 137.97(1C), 138.55(1C), 142.03(1C), 142.31(1C).

### Electrochemical characterization

In order to define the on-set potential of the polymerization of the trimer we used a three electrode setup. The working electrode is an ITO coated glass, the counter electrode is a Platinum sheet and the reference electrode is a Ag/AgCl saturated electrode. The electrolyte was 1 mg/ml of compound 1 in 0.01 M NaCl. The active area of the ITO was 21 mm x 8 mm. We performed cyclic voltammetry in order to define the onset potential. The potential was applied between the working and the reference electrode while the current was measured between the working and the counter electrode. The potential was scanned from +0.5 V to -0.5 V, starting from 0 V with a scan rate of 50 mV/s. During the first scan we observed that the current increases at around +0.3 V defining the onset potential of the polymerisation. The polymer is deposited on the active area of the ITO coated glass.
On-set potential 0.3 V
EDOT = 1.1 V
The results are shown in figure 30.

### The reaction of compound 1 with different parts of the plant

An aqueous solution of compound 1 was prepared (1 mg/mL).
The plant tissues used were xylem, pith, phloem and leaves, shown from left to right in figure 31A. 2 mL of the prepared solution was added to the xylem whereas 0.5 mL was added to the pith, the phloem and the leaves (Fig B. xylem, pith, phloem and leaves, shown from left to right). Figure C show the tissues after 2 hours (xylem, pith, phloem and leaves, shown from left to right). Figures D show the xylem, the pith and the phloem after 5 hours, whereas figure E shows the leaves after 48 hours.

### Compound 1 solution oxygen sensitivity

Fig 32 A: After mixing compound 1 powder with de-ionized water.
Vial X was left in room temperature under an air atmosphere.
Vial Y was sealed and left in fridge (4°C).
Vial Y': was sealed and left in fridge (4°C), but with leaves immersed in the mixture.
Vial Z was purged with N2, and then sealed in fridge.
Fig 32 B: After 18h
Fig 32 C: After 24h

### Comparison of polymerization with and without plant

Two solutions of compound 1 in water were prepared, one of which had a plant part immersed in it. UV-Vis spectra were recorded for the two solutions at t = 0 and after 45 hours. The resulting UV-Vis spectra (figure 33) clearly show that more polymerization had occurred in the solution in which the plant was immersed. Plant material thus speeds up the polymerization process.

### References

1 Raven, P. H., Evert, R. F. & Eichhorn, S. E. Biology of Plants. (W.H.Freeman and Company Publishers, 2005).
2 Grebe, M. Plant biology: Unveiling the Casparian strip. Nature 473, 294-295 (2011).
3 Tybrandt, K., Forchheimer, R. & Berggren, M. Logic gates based on ion transistors. Nature communications 3, 871 (2012).
4 Khodagholy, D. et al. In vivo recordings of brain activity using organic transistors. Nature Communications 4 (2013).
5 Ouyang, L., Shaw, C. L., Kuo, C. C., Griffin, A. L. & Martin, D. C. In vivo polymerization of poly(3,4-ethylenedioxythiophene) in the living rat hippocampus does not cause a significant loss of performance in a delayed alternation task. Journal of Neural Engineering 11 (2014).
6 Liew, S. Y., Walsh, D. A. & Thielemans, W. High total-electrode and mass-specific capacitance cellulose nanocrystal-polypyrrole nanocomposites for supercapacitors. RSC Advances 3, 9158-9162 (2013).
7 Björström Svanström, C. M. et al. Device performance of APFO-3/PCBM solar cells with controlled morphology. Advanced Materials 21, 4398-4403 (2009).
8 Hendricks, J. L., Chikar, J. A., Crumling, M. A., Raphael, Y. & Martin, D. C. Localized cell and drug delivery for auditory prostheses. Hearing Research 242, 117-131 (2008).
9 Bolin, M. H. et al. Nano-fiber scaffold electrodes based on PEDOT for cell stimulation. Sensors and Actuators B-Chemical 142, 451-456, doi:10.1016/j.snb.2009.04.062 (2009).
10 Masarovicova, E. & Kralova, K. Metal nanoparticles and plants. Ecological Chemistry and Engineering S 20, 9-22, doi:DOI: 10.2478/eces-2013-0001. (2013).
11 Giraldo, J. P. et al. Plant nanobionics approach to augment photosynthesis and biochemical sensing. Nature Materials 13, 400-408, doi:.DOI: 10.1038/NMAT3890. (2014).
12 Djikanovic', D. et al. Interaction of the CdSe quantum dots with plant cell walls. Colloids and Surfaces B: Biointerfaces 91, 41-47, doi:DOI: 10.1016/j.colsurfb.2011.10.032. (2012).
13 Kanhed, P. et al. In vitro antifungal efficacy of copper nanoparticles against selected crop pathogenic fungi. Materials Letters 115, 13-17, doi:DOI: 10.1016/j.matlet.2013.10.011. (2014).
14 Reynolds, J. R. & Morvant, M. C. In situ conductivity studies of poly(3,4-ethylenedioxythiophene). Synthetic Metals 92, 57-61 (1998).
15 Young Jo, K., Sang-Fun, C., Whitacre, J. & Bettinger, C. J. Self-deployable materials. Journal of Materials Chemistry B 1, 3781-3788, doi:.DOI: 10.1039/c3tb20183j. (2013).
16 Irimia-Vladu, M. et al. Indigo-A Natural Pigment for High Performance Ambipolar Organic Field Effect Transistors and Circuits. Advanced Materials 24, 375-380, doi:DOI: 10.1002/adma.201102619. (2012).
17 Leger, J., Berggren, M. & Carter, S. Iontronics: Ionic Carriers in Organic Electronic Materials and Devices. (CRC Press, 2010).
18 Kergoat, L. et al. Detection of Glutamate and Acetylcholine with Organic Electrochemical Transistors Based on Conducting Polymer/Platinum Nanoparticle Composites. Advanced Materials 26, 5658-+, doi: 10.1002/adma.201401608 (2014).
19 Tybrandt, K., Larsson, K. C., Richter-Dahlfors, A. & Berggren, M. Ion bipolar junction transistors. Proceedings of the National Academy of Sciences of the United States of America 107, 9929-9932, doi:10.1073/pnas.0913911107 (2010).
20 Bernards, D. A. et al. Enzymatic sensing with organic electrochemical transistors. Journal of Materials Chemistry 18, 116-120 (2008).
21. Yamada M. et al. Prussian blue nanoparticles protected by the water-soluble π-conjugated polymer PEDOT-S: synthesis and multiple-color pH-sensing with a redox reaxtion. Chem. Communications 2009, 7203-7205*.*
22. Zou, J. et al. Transparent carbon nanotube/Poly(3,4-ethylenedioxythiophene) compoisite electrical conductors electronically conducting organic part. Soft Materials 7, 355-365 (2009)
23. Yun, D.-J. et al. Multiwall Carbon Nanotube and Poly(3,4-ethylenedioxythiophene):Polystyrene Sulfonate (PEDOT:PSS) Composite Films for Transistor and Inverter Devices. Applie Materials & Interfaces 3, 43-49 (2011).
101. P. H. Raven, R. F. Evert, S. E. Eichhorn, in Biology of plants (W. H. Freeman, 2005).
102. B. Buchanan, W. Gruissem, R. Jones, Biochemistry & Molecular Biology of Plants (Wiley, ed. 2, 2015).
103. J. M. Leger, Organic Electronics: The Ions Have It. Adv. Mater. 20, 837-841 (2008).
104. X. Wang, B. Shapiro, E. Smela, Visualizing Ion Currents in Conjugated Polymers. Adv. Mater. 16, 1605-1609 (2004).
105. A. Jonsson et al., Therapy using implanted organic bioelectronics. Sci. Adv. 1, e1500039 (2015).
106. J. Rivnay et al., High-performance transistors for bioelectronics through tuning of channel thickness. Sci. Adv. 1, e1400251 (2015).
107. L. Ouyang, C. L. Shaw, C. C. Kuo, A. L. Griffin, D. C. Martin, In vivo polymerization of poly(3,4-ethylenedioxythiophene) in the living rat hippocampus does not cause a significant loss of performance in a delayed alternation task. Journal of Neural Engineering. 11, 026005 (2014).
108. M. Hamedi, A. Elfwing, R. Gabrielsson, O. Inganäs, Electronic Polymers and DNA Self-Assembled in Nanowire Transistors. Small. 9, 363-368 (2013).
109. L. Groenendaal, F. Jonas, D. Freitag, H. Pielartzik, J. R. Reynolds, Poly(3,4-ethylenedioxythiophene) and Its Derivatives: Past, Present, and Future. Adv. Mater. 12, 481-494 (2000).
[1001] Karlsson et al. Iron-Catalyzed Polymerization of Alkoxysulfonate Functionalized 3,4-Ethylenedioxythiophene Gives Water-Soluble Poly(3,4 ethylenedioxythiophene) of High Conductivity, Chem. Mat., 2009, 21 (9), 1815-1821
[1002] Persson et al. Electronic Control of Cell Detachment Using a Self-Doped Conducting Polymer, Adv. Mater., 23 (38), 2011, 4403-4408
[1003] R. Gabrielsson, Electroactive Conjugated Polyelectrolytes Based on EDOT, From Synthesis to Organic Electronics, PhD thesis, 2012, ISBN: 9789175198125
[1004] Conjugated Polymers: Theory, Synthesis, Properties, and Characterization (Handbook of Conducting Polymers, Third Edition), CRC-Press, ISBN: 1420043587.
[1005] H.E. Gottlieb, V. Kotlyar and A. Nudelman, NMR chemical shifts of common laboratory solvents as trace impurities, J Org Chem, 62 (1997) 7512-7515.
[1006] Hamedi et al., Electronic Polymers and DNA Self-Assembled in Nanowire Transistors, Small, 2013, 9 (3), 363-368
1010. R. H. Karlsson et al., Iron-Catalyzed Polymerization of Alkoxysulfonate-Functionalized 3,4-Ethylenedioxythiophene Gives Water-Soluble Poly(3,4-ethylenedioxythiophene) of High Conductivity. Chemistry of Materials. 21, 1815-1821 (2009).
1011. E. Stavrinidou et al., Adv. Mater., in press, doi:10.1002/adma.201301240.
1012. D. Nilsson et al., Bi-stable and dynamic current modulation in electrochemical organic transistors. Adv. Mater. 14, 51-54 (2002).
1013. J. Bobacka, Potential Stability of All-Solid-State Ion-Selective Electrodes Using Conducting Polymers as Ion-to-Electron Transducers. Anal. Chem. 71, 4932-4937 (1999).
1014. F. Louwet, The Organic Alternative to ITO. SID Digest of Tech. Papers. 39, 663-664 (2008).
1015. S. Ghosh, O. Inganäs, Networks of electron-conducting polymer in matrices of ion-conducting polymers. Applications to fast electrodes. Electrochemical and Solid-State Letters. 3, 213-215 (2000).
1016. B. Winther-Jensen, O. Winther-Jensen, M. Forsyth, D. R. MacFarlane, High rates of oxygen reduction over a vapor phase-polymerized PEDOT electrode. Science. 321, 671-674 (2008).
1017. P. Andersson et al., Active matrix displays based on all-organic electrochemical smart pixels printed on paper. Adv. Mater. 14, 1460-1464 (2002).
1018. D. Nilsson, N. Robinson, M. Berggren, R. Forchheimer, Electrochemical Logic Circuits. Adv. Mater. 17, 353-358 (2005).
1019. E. Masarovicova, K. Kralova, Metal nanoparticles and plants. Ecological Chemistry and Engineering S. 20, 9-22 (2013).
1020. J. P. Giraldo et al., Plant nanobionics approach to augment photosynthesis and biochemical sensing. Nat. Mater. 13, 400-408 (2014).
1021. D. Djikanovic et al., Interaction of the CdSe quantum dots with plant cell walls. Colloids and Surfaces B: Biointerfaces. 91, 41-47 (2012).
1022. H. Hussain, Z. Yi, J. Rookes, L. Kong, D. Cahill, Mesoporous silica nanoparticles as a biomolecule delivery vehicle in plants. J Nanopart Res. 15, 1676 (2013).
1023. P. Kanhed et al., In vitro antifungal efficacy of copper nanoparticles against selected crop pathogenic fungi. Materials Letters. 115, 13-17 (2014).
1024. D. M. Miller, Studies of Root Function in Zea mays: III. Xylem Sap Composition at Maximum Root Pressure Provides Evidence of Active Transport into the Xylem and a Measurement of the Reflection Coefficient of the Root. Plant Physiology. 77, 162-167 (1985).
1025. L. Bernstein, Method for Determining Solutes in the Cell Walls of Leaves. Plant Physiology. 47, 361-365 (1971).
1026. B. M. O'Leary, A. Rico, S. McCraw, H. N. Fones, G. M. Preston, The Infiltration-centrifugation Technique for Extraction of Apoplastic Fluid from Plant Leaves Using Phaseolus vulgaris as an Example. J. Vis. Exp. 94, e52113 (2014).
1027. G. Lohaus, K. Pennewiss, B. Sattelmacher, M. Hussmann, K. Hermann Muehling, Is the infiltration-centrifugation technique appropriate for the isolation of apoplastic fluid? A critical evaluation with different plant species. Physiologia Plantarum. 111, 457-465 (2001).
1028. J. Kawahara et al., Reconfigurable sticker label electronics manufactured from nanofibrillated cellulose-based self-adhesive organic electronic materials. Org. Electron. 14, 3061-3069 (2013).
1029. S. Ramakrishnan, C. Shannon, Display of Solid-State Materials Using Bipolar Electrochemistry. Langmuir. 26, 4602-4606 (2010).
1030. E. Said, N. D. Robinson, D. Nilsson, P. O. Svensson, M. Berggren, Visualizing the electric field in electrolytes using electrochromism from a conjugated polymer. Electrochemical and Solid-State Letters. 8, H12-H16 (2005).

## Claims

1. An electronic component comprising:
- an organic plant part e.g. a petal, a petiole, a stem portion, a petal portion, a petiole portion or a leaf portion;
- electronic conducting material provided within the organic plant part; and
wherein the electronic conducting material forms a continuous electronic conductor and/or a circuitry; and
wherein said electronic conducting material is an organic polymer semiconductor material.

2. An electronic component according to claim 1, wherein said electronic material is provided in one or more of the transport channel(s); and/or
wherein said electronic material is provided to surround the cells within the spongy mesophyll of the leaf; and/or
wherein said electronic material is provided on at least the external surface of the root.

3. An electronic component according to any one of the preceding claims, wherein the length of said continuous electronic conductor is at least 10 µm, or at least 100 µm or at least 1 mm, or wherein the length of said circuit is at least 100 µm or at least 1 mm.

4. An electronic component according to any one of the preceding claims, wherein the continuous electronic conductor extends along the inner wall of the transport channel in the circumferential direction; and wherein the continuous electronic conductor preferably extends along at least 10 %, or preferably at least 20 %, or preferably at least 40 %, or preferably at least 60 %, or preferably at least 80 %, or preferably at least 90 % of the circumference of the inner wall.

5. An electronic component according to any one of the preceding claims, wherein the continuous electronic conductor is formed as a layer or wire.

6. An electronic component according to any one of the preceding claims, wherein the electrical conductivity of said continuous electronic conductor is at least 10⁻³ S/m, or at least 10⁻¹ S/m, or at least 10 S/m.

7. An electronic component according to any one of the preceding claims, wherein the electric component is at least one of a conductor, a transistor, a capacitor or a super-capacitor and an electrochromic device.

8. An electronic component according to any one of the preceding claims, wherein the continuous electronic conductor comprises an electronic conducting material selected from a group comprising: electrochemically active material and semiconducting organic polymers.

9. An electronic component according to any one of the preceding claims, wherein electronic conducting material comprises a conductive polymer comprising the repeating unit of formula II 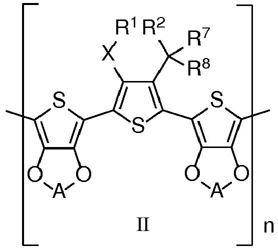 wherein
A represents a C₁-C₄-alkylene diradical optionally substituted with one to four R⁹;
X is hydrogen or CR⁵R⁶;
when X is hydrogen, R¹ is absent;
when X is CR⁵R⁶, R¹ is selected from the group C₃-C₆-cycloalkyl, aryl and C₁-C₈-alkyl in which one -CH₂- is optionally replaced by -O- or -S-, said C₃-C₆-cycloalkyl, C₁-C₈-alkyl or aryl optionally carrying one to three R³, said C₁-C₈-alkyl may be selected such that R¹ and R² form a 5-10 membered ring;
R² is selected from the group C₃-C₆-cycloalkyl, aryl and Ci-Cs-alkyl in which one -CH₂- is optionally replaced by -O- or -S-, said C₃-C₆-cycloalkyl, C₁-C₈-alkyl or aryl optionally carrying one to three R³, said C₁-C₈-alkyl may be selected such that R¹ and R² form a 5-10 membered ring;
R¹ and/or R² carries one or two R⁴;
each R³ and R⁹ is independently selected from hydrogen, hydroxy, halogen, C₁-C₃-alkyl, C₁-C₃-haloalkyl and C₁-C₃-alkoxy;
each R⁴ is independently selected from hydrogen, sulfonate, carboxylate, sulfinate, phosphonate and phosphinate or the corresponding acid or the corresponding sodium, potassium or lithium salt thereof; and
R⁵, R⁶, R⁷ and R⁸ are each independently selected from hydrogen, halogen, C₁-C₃-alkyl, C₁-C₃-haloalkyl and C₁-C₃-alkoxy.

10. An electronic component according to any one of the preceding claims, wherein the electronic component further comprises external contacts and/or connecting wires only partially inserted into said electronic component.

11. A system comprising at least one electronic component according to any one of said claims 1-10, wherein the electronic conducting material is electrically connected to a device for measuring and/or controlling properties related to the organic plant part.

12. A method of operating a component according to claim 1, wherein said electronic conducting material comprises electrochemically active material, said method comprising the steps of:
- supplying first potential to a first portion of said electronic conducting material, a second potential to a second portion of said electronic conducting material which is different from said first potential; and
- electrochemically reacting the electronic conducting material being an electrochemically active material.

13. A method of manufacturing a device according to any one of claims 1-10 comprising the steps of providing organic polymer electronic conducting material inside an organic plant part, where the polymer electronic material is polymerised inside the organic plant part or in the transport channel or transport channels of said organic plant part.

## Patentansprüche

1. Eine elektronische Komponente, umfassend:
- ein organisches Pflanzenteil, z.B. ein Blütenblatt, ein Blattstiel, ein Stängelteil, ein Blütenblattteil, ein Blattstielteil oder ein Blattteil;
- elektronisches leitendes Material, das im organischen Pflanzenteil enthalten ist; und wobei das elektronisch leitende Material einen kontinuierlichen elektronischen Leiter und / oder einen Schaltkreis bildet; und
wobei das elektronisch leitende Material ein organisches Polymerhalbleitermaterial ist.

2. Eine elektronische Komponente nach Anspruch 1,
wobei das elektronische Material in einem oder mehreren der Transportkanäle bereitgestellt wird; und / oder
wobei das elektronische Material um die Zellen innerhalb des schwammigen Mesophylls des Blattes bereitgestellt wird; und / oder
wobei das elektronische Material mindestens auf der Außenfläche der Wurzel bereitgestellt wird.

3. Elektrische Komponente nach einem der vorhergehenden Ansprüche, wobei die Länge des kontinuierlichen elektronischen Leiters mindestens 10 µm oder mindestens 100 µm oder mindestens 1 mm beträgt oder wobei die Länge des Schaltkreises mindestens 100 µm beträgt oder mindestens 1 mm.

4. Elektrische Komponente nach einem der vorhergehenden Ansprüche, wobei sich der durchgehende elektronische Leiter entlang der Innenwand des Transportkanals in Umfangsrichtung erstreckt; und wobei sich der kontinuierliche elektronische Leiter vorzugsweise entlang mindestens 10% oder vorzugsweise mindestens 20% oder vorzugsweise mindestens 40% oder vorzugsweise mindestens 60% oder vorzugsweise mindestens 80% oder vorzugsweise mindestens 90% des Umfang der Innenwand erstreckt.

5. Elektrische Komponente nach einem der vorhergehenden Ansprüche, wobei der kontinuierliche elektronische Leiter als Schicht oder Draht ausgebildet ist.

6. Elektrische Komponente nach einem der vorhergehenden Ansprüche, wobei die elektrische Leitfähigkeit des kontinuierlichen elektronischen Leiters mindestens 10⁻³ S/m oder mindestens 10⁻¹ S/m oder mindestens 10 S/m beträgt.

7. Elektronische Komponente nach einem der vorhergehenden Ansprüche, wobei die elektrische Komponente mindestens aus einem Leiter, einem Transistor, einem Kondensator oder einem Superkondensator und einer elektrochromen Vorrichtung besteht.

8. Elektronische Komponente nach einem der vorhergehenden Ansprüche, wobei der kontinuierliche elektronische Leiter ein elektronisch leitendes Material umfasst, ausgewählt aus einer Gruppe umfassend: elektrochemisch aktives Material und halbleitende organische Polymere.

9. Elektronische Komponente nach einem der vorhergehenden Ansprüche, wobei elektronisches leitendes Material ein leitfähiges Polymer umfasst, das die Wiederholungseinheit der Formel II umfasst 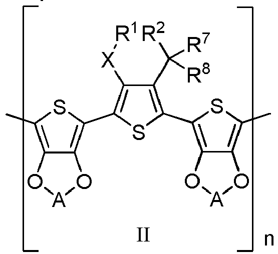 wobei
A ein C₁-C₄ -alkylen-Diradikal darstellt, gegebenenfalls substituiert mit ein bis vier R⁹;
X ist Wasserstoff oder CR⁵R⁶;
wenn X Wasserstoff ist, fehlt R¹;
wenn X CR⁵R⁶ ist, wird R¹ aus der Gruppe C₃-C₆-cycloalkyl, Aryl und C₁-C₈-alkyl ausgewählt, wobei ein -CH₂ - gegebenenfalls durch -O- oder -S- ersetzt wird , wobei das C₃-C₆-cycloalkyl, C₁-C₈-alkyl oder Aryl, gegebenenfalls ein bis drei R³ trägt, das C₁ -C₈-alkyl kann so ausgewählt werden, dass R¹ und R² einen 5- 10-gliedrigen Ring bilden; R² ist aus der Gruppe C₃-C₆-cycloalkyl, Aryl und C₁-C₈-alkyl ausgewählt, in der ein -CH₂ - gegebenenfalls durch -O- oder -S-ersetzt wird, das C₃-C₆-cycloalkyl, und C₁-C₈-alkyl oder Aryl, das gegebenenfalls ein bis drei R³ trägt, wobei das C₁-C₈-alkyl so ausgewählt werden kann, dass R¹ und R² einen 5-10-gliedrigen Ring bilden;
R¹ und / oder R² tragen ein oder zwei R⁴;
jedes R³ und R⁹ ist unabhängig ausgewählt aus Wasserstoff, Hydroxyd, Halogen, C₁-C₃-alkyl, C₁-C₃-halogenalkyl und C₁-C₃-alkoxy; jedes R⁴ ist unabhängig ausgewählt aus Wasserstoff, Sulfonat, Carboxylat, Sulfinat, Phosphonat und Phosphinat oder der entsprechenden Säure oder dem entsprechenden Natrium-, Kalium- oder Lithiumsalz davon; und
R⁵, R⁶, R⁷ und R⁸ sind jeweils unabhängig voneinander ausgewählt aus Wasserstoff, Halogen, C₁-C₃-alkyl, C₁-C₃-halogenalkyl und C₁-C₃-alkoxy.

10. Elektronische Komponente nach einem der vorhergehenden Ansprüche, wobei die elektronische Komponente ferner externe Kontakte und / oder Verbindungsdrähten umfasst, die nur teilweise in die elektronische Komponente eingeführt werden.

11. System, mindestens eine elektronische Komponente nach einem der Ansprüche 1-10 umfassend, wobei das elektronisch leitende Material elektrisch mit einer Vorrichtung zum Messen und / oder Steuern von Eigenschaften verbunden ist, die sich auf den organischen Pflanzenteil beziehen.

12. Verfahren zum Betreiben einer Komponente nach Anspruch 1, wobei das elektronisch leitende Material elektrochemisch aktives Material umfasst, wobei das Verfahren die folgenden Schritte umfasst:
- Zuführen des ersten Potentials zu einem ersten Teil des elektronisch leitenden Materials, eines zweiten Potentials zu einem zweiten Teil des elektronisch leitenden Materials, das sich von dem ersten Potential unterscheidet; und
- elektrochemische Reaktion des elektronisch leitenden Materials als elektrochemisch aktives Material.

13. Verfahren zum Bereitstellen einer Anordnung nach Anspruch 1-10, wobei das Verfahren die folgenden Schritte umfasst:
Bereitstellen eines elektronisch leitenden Materials aus organischem Polymer innerhalb eines organischen Pflanzenteils, wobei das elektronische Polymermaterial innerhalb des organischen Pflanzenteils oder in dem Transportkanal oder den Transportkanälen des organischen Pflanzenteils polymerisiert wird.

## Revendications

1. Composant électronique comprenant :
- une partie de plante biologique, par exemple une pétale, un pétiole, une partie de souche, une partie de pétale, une partie de pétiole ou une partie de feuille ;
- un matériau électroniquement conducteur monté à l'intérieur de la partie de pente biologique ; et
dans lequel le matériel électroniquement conducteur forme un conducteur électronique continu et/ou un circuit ; et
dans lequel ledit matériau électroniquement conducteur est un matériau semiconducteur polymère organique.

2. Composant électronique selon la revendication 1, dans lequel ledit matériau électronique est monté dans au moins l'un des canaux de transport ; et/ou
dans lequel ledit matériau électronique est monté afin d'entourer les cellules situées à l'intérieur du mésophile spongieux de la feuille ; et/ou
dans lequel ledit matériau électronique est monté sur au moins la surface externe de la racine.

3. Composant électronique selon l'une quelconque des revendications précédentes, dans lequel la longueur dudit conducteur électronique continu vaut au moins 10 µm, ou au moins 100 µm, ou au moins 1 mm, ou dans lequel la longueur du circuit vaut au moins 100 µm ou au moins 1 mm.

4. Composant électronique selon l'une quelconque des revendications précédentes, dans lequel le conducteur électronique continu s'étend le long de la paroi interne du canal de transport dans la direction de circonférence ; et dans lequel le conducteur électronique continu s'étend de préférence le long d'au moins 10 %, ou de préférence d'au moins 20 %, ou de préférence d'au moins 40 %, ou de préférence d'au moins 60 %, ou de préférence d'au moins 80 %, ou de préférence d'au moins 90 % de la circonférence de la paroi interne.

5. Composant électronique selon l'une quelconque des revendications précédentes, dans lequel le conducteur électronique continu est formé en tant que couche ou que câble.

6. N Composant électronique selon l'une quelconque des revendications précédentes, dans lequel la conductivité électrique dudit conducteur électronique continu vaut au moins 10⁻³ S/m, ou au moins 10⁻¹ S/m, ou au moins 10 S/m.

7. Composant électronique selon l'une quelconque des revendications précédentes, dans lequel le composant électrique est au moins l'un parmi un conducteur, un transistor, un condensateur ou un supercondensateur et un dispositif électrochromique.

8. Composant électronique selon l'une quelconque des revendications précédentes, dans lequel le conducteur électronique continu comprend un matériau électroniquement conducteur choisi dans un groupe comprenant : un matériau actif sur le plan électrochimique et des polymères organiques semi-conducteurs.

9. Composant électronique selon l'une quelconque des revendications précédentes, dans lequel le matériau électroniquement conducteur comprend en polymère conducteur comprenant l'unité de répétition de formule II 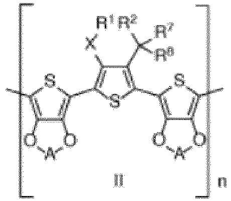 dans laquelle
A représente un diradical alkylène en C₁-C₄ éventuellement substitué par un à quatre R⁹ ;
X représente l'hydrogène ou un groupe CR⁵R⁶ ;
lorsque X représente l'hydrogène, R¹ est absent ;
lorsque X représente CR⁵R⁶, R¹ est choisi dans le groupe constitué par un groupe cycloalkyle en C₃-C₆, un groupe aryle et un groupe alkyle en C₁-C₈ dans lequel un groupe -CH₂- est éventuellement remplacé par -O- ou -S-, ledit groupe cycloalkyle en C₃-C₆, alkyle en C₁-C₈ ou aryle portant éventuellement de un à trois R³, ledit groupe alkyle en C₁-C₈ peut être choisi de telle sorte que R¹ et R² forment un cycle comportant de 5 à 10 chaînons ;
R² est choisi dans le groupe constitué par un groupe cycloalkyle en C₃-C₆, un groupe aryle et un groupe alkyle en C₁-C₈ dans lequel un groupe -CH₂- est éventuellement remplacé par -O- ou -S-, ledit groupe cycloalkyle en C₃-C₆, alkyle en C₁-C₈ ou aryle portant éventuellement de un à trois R³, ledit groupe alkyle en C₁-C₈ peut être choisi de telle sorte que R¹ et R² forment un cycle comportant de 5 à 10 chaînons ;
R¹ et/ou R² porte un ou deux R⁴ ;
chaque R³ et R⁹ est indépendamment choisi parmi l'hydrogène, l'hydroxy, un halogène, un groupe alkyle en C₁-C₃, un groupe halogénoalkyle en C₁-C₃ et un groupe alcoxy en C₁-C₃ ;
chaque R⁴ est indépendamment choisi parmi l'hydrogène, le sulfonate, le carboxylate, le sulfinate, le phosphonate et le phosphinate ou l'acide correspondant ou le sel de sodium, de potassium ou de lithium correspondant de celui-ci ; et
R⁵, R⁶, R⁷ et R⁸ sont chacun indépendamment choisis parmi l'hydrogène, un halogène, un groupe alkyle en C₁-C₃, un groupe halogénoalkyle en C₁-C₃ et un groupe alcoxy en C₁-C₃.

10. Composant électronique selon l'une quelconque des revendications précédentes, dans lequel le composant électronique comprend en outre des contacts externes et/ou des câbles de connexion seulement partiellement insérés dans ledit composant électronique.

11. Système comprenant au moins un composant électronique selon l'une quelconque des revendications 1 à 10, dans lequel le matériau électroniquement conducteur est relié électriquement à un dispositif pour la mesure et/ou le contrôle des propriétés liées à la partie de plante biologique.

12. Procédé de fonctionnement d'un pour composant selon la revendication 1, dans lequel ledit matériau électroniquement conducteur comprend un matériau actif sur le plan électrochimique, ledit procédé comprenant les étapes de :
- apport d'un premier potentiel sur une première partie dudit matériau électroniquement conducteur, d'un second potentiel sur une seconde partie dudit matériau électroniquement conducteur qui est différent dudit premier potentiel ; et
- réaction sur le plan électrochimique du matériau électroniquement conducteur étant un matériau actif sur le plan électrochimique.

13. Procédé de fabrication d'un dispositif selon l'une quelconque des revendications 1 à 10 comprenant les étapes d'apport d'un matériau électroniquement conducteur polymère organique à l'intérieur d'une partie de plante biologique, dans lequel le matériau électronique polymère est polymérisé à l'intérieur de la partie de plante biologique ou dans le canal de transport ou dans les canaux de transport de ladite partie de plante biologique.
